# EUROPEAN PATENT SPECIFICATION

(11) **EP 3 582 754 B1**
(45) Date of publication and mention of the grant of the patent: **10.01.2024**
(21) Application number: 18754750.0
(22) Date of filing: 16.02.2018
(51) Int. Cl.: A61K 9/06, A61K 38/10, A61K 38/17, C07K 14/46, A61K 47/02, A61P 17/02, A61P 39/00, C07K 14/705, A61K 9/00

(54) **COMPOSITION AND METHODS FOR PREVENTING RADIATION INJURY AND PROMOTING TISSUE REGENERATION**
ZUSAMMENSETZUNG UND VERFAHREN ZUR VERHINDERUNG EINER STRAHLUNGSVERLETZUNG UND ZUR FÖRDERUNG DER GEWEBEREGENERATION
COMPOSITION ET MÉTHODES POUR PRÉVENIR UNE RADIOLÉSION ET FAVORISER LA RÉGÉNÉRATION TISSULAIRE

(30) Priority: 16.02.2017 US 201762459924 P
(43) Date of publication of application: 25.12.2019
(73) Proprietor: Xequel Bio, Inc., Mount Pleasant, SC 29464 (US)
(72) Inventor: GHATNEKAR, Gautam, Mount Pleasant, SC 29464 (US)
(74) Representative: Hoffmann Eitle
(86) International application number: PCT/US2018/000035
(87) International publication number: WO 2018/151823

(56) References cited:
- EP-A2- 2 377 546
- WO-A1-2015/123383
- US-A1- 2005 014 785
- US-A1- 2013 177 628
- US-A1- 2016 120 171
- US-A1- 2017 128 523
- GREK CHRISTINA L ET AL: "Cardiac to cancer: Connecting connexins to clinical opportunity", FEBS LETTERS, ELSEVIER, AMSTERDAM, NL, vol. 588, no. 8, 4 March 2014 (2014-03-04) , pages 1349-1364, XP028868006, ISSN: 0014-5793, DOI: 10.1016/J.FEBSLET.2014.02.047
- ARTESI, M et al.: "Connexin 30 expression inhibits growth of human malignant gliomas but protects them against radiation therapy", Neuro-Oncology, vol. 17, no. 3, March 2015 (2015-03), pages 392-406, XP055537082,
- CITRIN, D et al.: "Radioprotectors and Mitigators of Radiation-Induced Normal Tissue Injury", The Oncologist, vol. 15, no. 4, 22 April 2010 (2010-04-22) , pages 380-371, XP055263813,
- anonymous: "Cutaneous Radiation Injury (CRI): A Fact Sheet for Physicians", CENTERS FOR DISEASE CONTROL AND PREVENTION (CDC), 29 June 2015 (2015-06-29), pages 1-13, XP055537091, Retrieved from the Internet: URL:https://emergency.cdc.gov/radiation/pd f/criphysicianfactsheet.pdf [retrieved on 2018-05-09]
- GREK, CL et al.: "A Multicenter Randomized Controlled Trial Evaluating a Cx43-Mimetic Peptide in Cutaneous Scarring", Journal of Investigative Dermatology, vol. 137, no. 3, March 2017 (2017-03), pages 620-630, XP055537094,

## Description

### BACKGROUND OF THE INVENTION

Current events have highlighted various terrorist actions that may eventually be intended to set up a nuclear explosion or disseminate radioactive materials using a radiological dispersal device (e.g., a dirty bomb). In such an event, population exposure to large doses of external and/or internal ionizing radiation is likely, in addition to traumatic injuries. In the event of a nuclear explosion, exposure to large external doses of acute ionizing radiation can result in symptoms of acute radiation syndrome (ARS) in addition to or independent of injury to the skin and underlying tissues, termed cutaneous radiation injury (CRI).

CRI is defined as dermal injury resulting from direct or indirect exposure to low-penetrating radiation. In fallout events, the deposition of hot particles on the skin can result in high doses of acute localized radiation exposure. Exposure to radiation causes damage to the basal cell layer of the skin and results in inflammation, erythema, desquamation, intense reddening, blistering, debilitating fibrosis, infection, ulceration, and necrosis of the exposed tissue.

ARS describes one or a combination of clinical syndromes that occur after exposure to ionizing radiation. ARS syndromes include hematopoietic syndrome, gastrointestinal syndrome, neurovascular syndrome, cardiac syndrome, and others, and each of these syndromes can occur alone or in combination with others. In nuclear detonation scenarios, radiation exposure frequently occurs in combination with other traumatic injury, such as burns, blunt trauma, and open wounds, all of which can be complicated by microbial infection. Combined radiation injury describes conditions where radiation injury is coupled with other insults such as burns, wounds, infection, or blunt trauma.

Radiation dermatitis is a radiation injury that results from radiation therapy, such as radiotherapy or chemoradiotherapy applied to a subject as a treatment, for example a cancer treatment. Radiation dermatitis is a common side effect or toxicity of such therapies. Radiation dermatitis can be acute, arising within about 90 days from the radiation therapy, and/or chronic. Manifestations of radiation dermatitis include erythema, desquamation, necrosis, ulceration, atrophy, telangiectasias, fibrosis, and/or other skin changes. Severe radiation dermatitis can require the reduction, interruption, or cessation of a radiation therapy schedule, which can negatively impact the efficacy of the treatment of the cancer or other disease.

While human tissues damaged by mechanical wounding, disease processes and other causes are capable of healing, complex tissue structure and function is rarely, if ever wholly restored. Instead, recovery of nearly all tissues from injury in humans and other higher vertebrates is dominated by the formation of scar tissue. The most familiar example of this is the discolored and fibrotic scars that linger following the healing of a skin cut or graze. Less well appreciated is that formation of glial scar tissue following injury to the brain or spinal cord is one of the main obstacles to restoration of neural function following damage to the central nervous system (Silver and Miller JH, 2004). There is currently no means of treating or preventing such scarring and promoting the regeneration of complex tissue structure and function following injury.

There is a need in the art for suitable therapeutic agents that can treat, prevent, and/or mitigate the progression of radiation injury, including radiation dermatitis, CRT, ARS, and combinations thereof. The present disclosure addresses this and other needs.

EP 2 377 546 A2 concerns compositions and methods for promoting wound healing and tissue regeneration.

Grek Christina et al., FEBS LETTERS, ELSEVIER, Amsterdam, NL, vol. 588, no. 8, 4 March 2014, pages 1349-1364, concerns cardiac to cancer: connecting connexins to clinical opportunity.

### BRIEF SUMMARY OF THE INVENTION

The present invention is defined by the claims.

Provided is an isolated polypeptide comprising a carboxy-terminal amino acid sequence of an alpha Connexin (also referred to herein as an alpha Connexin carboxy-Terminal (ACT) polypeptide), or a conservative variant thereof. Provided herein is a method of preventing mitigating, and treating radiation injury in a subject at risk of such injury, comprising administering to the subject one or more of the herein provided compositions (e.g., polypeptides, nucleic acids, or vectors). For example, provided herein are methods for preventing, mitigating the progression of, and treating cutaneous radiation injury (CRI), acute radiation syndrome (ARS), combined radiation injury, or any combination thereof. In some embodiments, the present disclosure provides methods for preventing, mitigating the progression of, and treating CRI, ARS, and/or combined radiation injury following exposure to ionizing radiation, for example resulting from radiological dispersal devices, improvised nuclear devices, nuclear weapons, or nuclear power plant explosions. In some embodiments, provided herein are methods for preventing, mitigating the progression of, and treating radiation dermatitis.

In some embodiments, provided herein are methods for preventing, mitigating the progression of, and treating radiation injury that results from the sun, x-ray and other diagnostic machines, tanning beds, and other sources of ionizing radiation. For example, in some embodiments, the compositions provided herein are used in a method of preventing or mitigating the progression of such injuries before exposure to the source of ionizing radiation, or after exposure but before or early after the onset of symptoms of the injury.

In some embodiments, the present disclosure provides methods for preventing, mitigating the severity of, and treating radiation injury in a subject at risk of such injury, comprising administering to the subject a composition comprising an isolated polypeptide comprising an alpha Connexin polypeptide. In some embodiments, the composition is administered prior to exposure of the subject to radiation. In other embodiments, the composition is administered to the subject after exposure to radiation. For example, in some embodiments, the composition is administered to the subject at least one day following exposure to radiation. In some embodiments, the method prevents the progression of radiation injury. In some embodiments, the composition is administered topically to the subject. In some embodiments, the composition is administered topically to the subject at a dose of from about 10 µM to about 2000 µM. In further embodiments, the composition is administered to the subject at a dose of from about 50 µM, about 100 µM, about 150 µM, about 200 µM, about 300 µM, about 400 µM, about 500 µM, about 600 µM, about 700 µM, about 800 µM, about 900 µM, or about 1,000 µM. In some embodiments, the composition is administered in a daily dosing regimen. In some embodiments, the composition is administered parenterally to the subject. In further embodiments, the composition is administered to the subject by an intravenous, subcutaneous, intraperitoneal, or intramuscular route. For example, in some embodiments, the composition is administered to the subject by intravenous injection, subcutaneous injection, intraperitoneal injection, or intramuscular injection. In some embodiments, the composition is administered parenterally to the subject at a dose of from about 1 mg/kg to about 50 mg/kg. In further embodiments, the composition is administered to the subject at a dose of about 1 mg/kg, about 5 mg/kg, about 10 mg/kg, about 15 mg/kg, about 20 mg/kg, about 25 mg/kg, about 30 mg/kg, about 35 mg/kg, about 40 mg/kg, about 45 mg/kg, or about 50 mg/kg. In some embodiments, the composition is administered in a daily dosing regimen. In some embodiments, parenteral administration of the composition treats, prevents, and/or inhibits the progression of internal radiation damage.

In some embodiments, the radiation injury is selected from the group consisting of CR1, ARS, radiation burns, radiation dermatitis, radiation injury to the nervous system or brain, radiation pneumonitis, radiation-induced enteritis, and internal radiation. In certain embodiments, the radiation injury is CRI. In certain embodiments, the radiation injury is ARS. In certain embodiments, the radiation injury is combined radiation injury. Thus, in some embodiments, the radiation injury comprises radiation injury in combination with burns, wounds, infection, or blunt trauma. In some embodiments, the radiation injury results from exposure to a source of radiation selected from the group consisting of weapons of mass destruction (WMD), nuclear explosions, and dirty bombs. In some embodiments, the radiation injury comprises radiation dermatitis, which si a specific type of radiation injury resulting from radiotherapy regimens or other interventional medical procedures. In some embodiments, the radiation injury results from exposure to the sun, x-ray and other diagnostic machines, or ionizing radiation-emitting sources such as tanning beds. In some embodiments, the peptides provided herein are provided in a sunscreen, sunblock, or suntan lotion composition.

In some embodiments, the radiation injury occurs in a tissue selected from the group consisting of skin, heart, bone, brain, spinal cord, cornea, retina, hematopoietic system, gastrointestinal system, and peripheral nerve. In particular embodiments, the radiation injury occurs on the skin. In some embodiments, the average skin score is decreased by at least about 30% in presence of the polypeptide as compared to a control. In some embodiments, the polypeptide prevents scar formation. In some embodiments the polypeptide promotes tissue regeneration. In some embodiments the polypeptide prevents tissue damage.

In some embodiments, the alpha connexin polypeptide comprises the carboxy terminal-most 4 to 30 contiguous amino acids of an alpha Connexin. In some embodiments, the polypeptide consists of the carboxy terminal-most 5 to 19 contiguous amino acids of the alpha Connexin. In some embodiments, the alpha Connexin is Connexin 37, Connexin 40, Connexin 43, or Connexin 45. In further embodiments, the polypeptide comprises the amino acid sequence selected from the group consisting of SEQ ID NO: 1, SEQ ID NO: 2, SEQ ID NO: 3, SEQ ID NO: 4, and SEQ ID NO: 5. In some embodiments, the polypeptide further comprises a cellular internalization sequence. In further embodiments, the cellular internalization sequence comprises an amino acid sequence of a protein selected from a group consisting of Antennapedia, TAT, HIV-Tat, Penetratin, Antp-3A (Antp mutant), Buforin 11, Transportan, MAP (model amphipathic peptide), K-FGF, Ku70, Prion, pVEC, Pep-1, SynB 1, Pep-7, HN-1, BGSC (Bis-Guanidinium-Spermidine-Cholesterol) and BGTC (Bis-Guanidinium-Tren-Cholesterol). In some embodiments, the cellular internalization sequence is Antennapedia, and wherein the sequence comprises the amino acid sequence of SEQ ID NO:7. In some embodiments, the polypeptide comprises the amino acid sequence selected from the group consisting of SEQ ID NO:8, SEQ ID NO:9, SEQ ID NO:10, SEQ ID NO:11, and SEQ ID NO:12. In some embodiments, the alpha connexin polypeptide comprises a biotin at the N-terminus and/or the C-terminus of the polypeptide. In some embodiments, the composition comprises SEQ ID NO: 91.

In some embodiments, the composition is administered topically. In some embodiments, the composition further comprises hydroxyethylcellulose gel. In further embodiments, the hydroxyethylcellulose gel is present at a concentration of about 0.25% (w/w), about 0.5% (w/w), about 0.75% (w/w), about 1.00% (w/w), about 1.25% (w/w), about 1.5% (w/w) or about 2.0% (w/w). In some embodiments, the composition is administered systemically. For example, in some embodiments, the composition is administered parenterally. In some embodiments, the composition for systemic administration further comprises one or more pharmaceutically acceptable excipient suitable for systemic administration to a subject. In some embodiments, the composition may be administered both topically and systemically to a subject in need thereof. For example, in some embodiments, a subject exposed to radiation injury is administered a composition provided herein both topically and systemically in order to treat, prevent, and/or mitigate the progression of both CRT and ARS. For example, in some embodiments, a subject is administered a composition comprising a polypeptide provided herein topically, and is administered a composition comprising a polypeptide herein systemically. In some embodiments, the compositions for topical and systemic administration comprise the same alpha connexin polypeptide. In some embodiments, a subject exposed to radiation injury is administered a composition provided herein both topically and systemically in order to treat, prevent, and/or mitigate the progression of combined radiation injury involving CRI and/or ARS in combination with burns, wounds, infection, or blunt trauma.

Additional advantages of the disclosed method and compositions will be set forth in part in the description which follows, and in part will be understood from the description, or may be learned by practice of the disclosed method and compositions. The advantages of the disclosed method and compositions will be realized and attained by means of the elements and combinations particularly pointed out in the appended claims. It is to be understood that both the foregoing general description and the following detailed description are exemplary and explanatory only and are not restrictive of the invention as claimed.

### BRIEF DESCRIPTION OF THE DRAWINGS

The accompanying drawings, which are incorporated in and constitute a part of this specification, illustrate several embodiments of the disclosed method and compositions and together with the description, serve to explain the principles of the disclosed method and compositions.

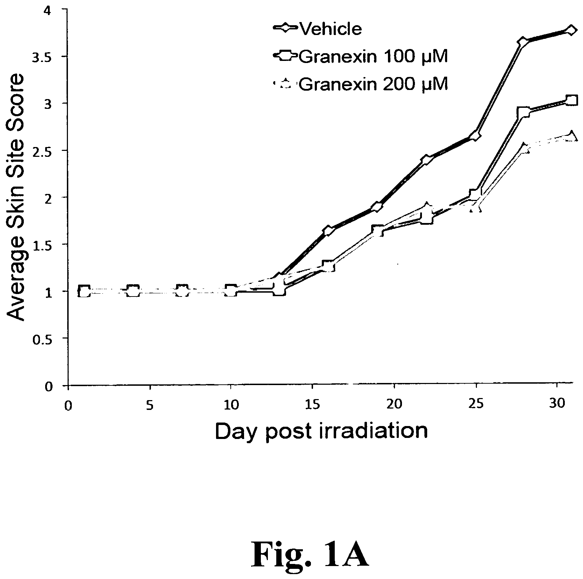

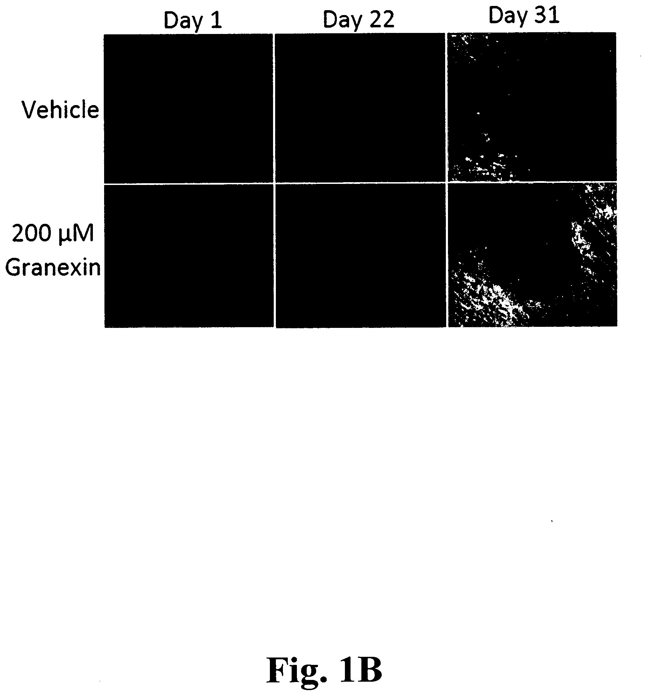

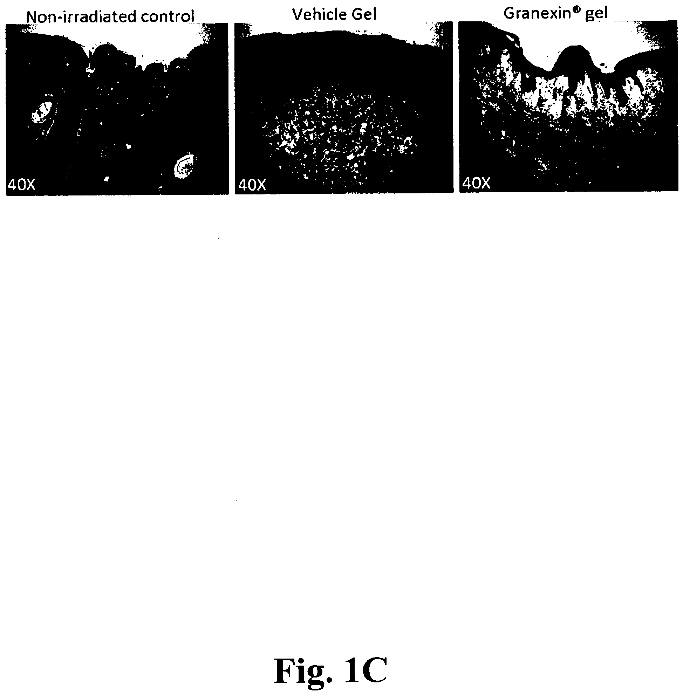

Figures 1A, 1B, and 1C show that topical administration of ACT peptide is effective in the prevention, mitigation, and treatment of cutaneous radiation injury (CRI). Yorkshire pigs were anesthetized and exposed to single fractions of low energy electrons (6 MeV). Beginning on Day 1 and every 3 days thereafter, skin sites were scored for erythema and desquamation using a Kumar Scale. Topical treatment with Granexin^{®} (100 µM or 200 µM) or vehicle was initiated upon observation of erythema (Kumar score > 1.0). and was applied once daily to exposure sites. Figure 1A shows average skin score comparisons with Granexin^{®} gel (200 µM) treatment and vehicle treatment. Figure 1B shows the effect of application of ACT peptide upon injury presentation. Granexin^{®} gel (200 µM) treatment (bottom row) was compared to vehicle treatment (top row) on Days 1, 22, and 31. Figure 1C shows H&E staining of skin biopsies from irradiation sites and non-irradiated control sites taken off the same animal on Day 43 (End-of-Study).

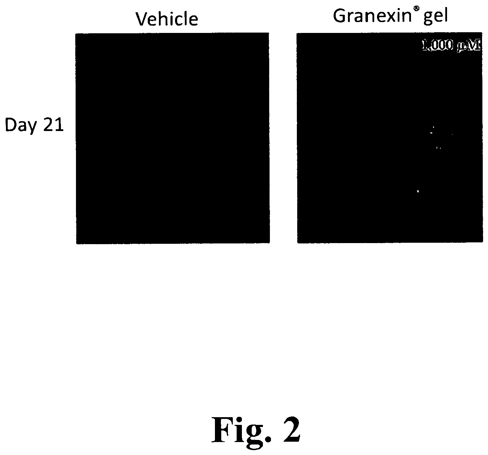

Figure 2 shows the prophylactic effect of ACT peptide on the prevention and mitigation of CRI post-exposure. Irradiated sites were prophylactically (prior to the presentation of CRI symptoms) treated with vehicle or Granexin^{®} gel 1,000 µM (beginning day 1; i.e. the day after radiation exposure) on the same daily treatment regimen. Day 21 photographic comparisons show that prophylactic treatment with Granexin^{®} gel at 1,000 µM prevents injury progression and reduces injury severity as compared to vehicle control.

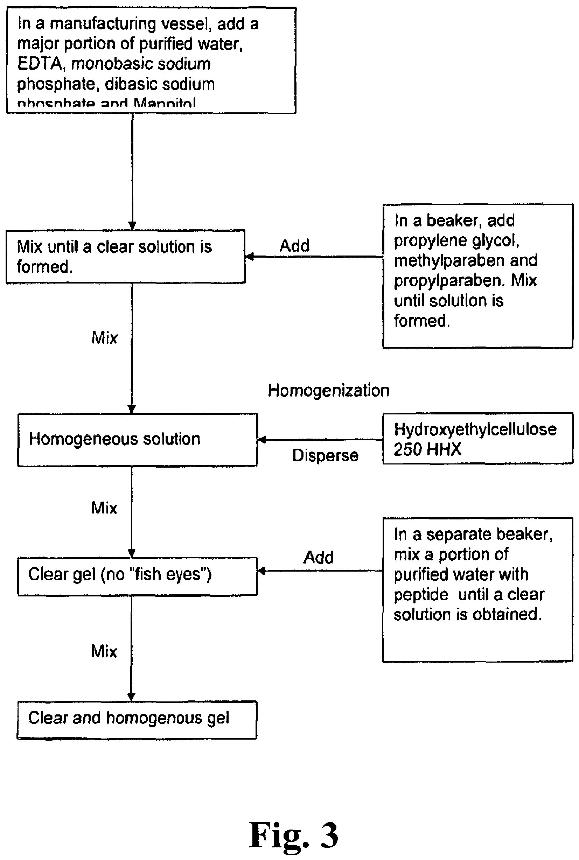

Figure 3 illustrates the manufacturing process flow chart of an ACT peptide of the invention.

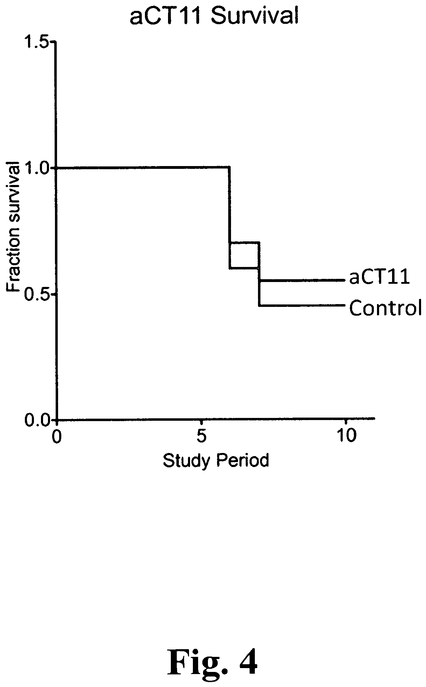

Figure 4 shows that ACT1 polypeptide reduces early mortality in a GI-toxicity model of irradiation. C57BL/6 mice treated daily with systemically delivered peptide (10mg/kg) showed increased survival compared to those treated with control saline.

### DETAILED DESCRIPTION OF THE INVENTION

The disclosed method and compositions may be understood more readily by reference to the following detailed description of particular embodiments and the Examples included therein and to the Figures and their previous and following description.

Provided is an isolated polypeptide comprising a carboxy-terminal amino acid sequence of an alpha Connexin (also referred to herein as an alpha Connexin carboxy-Terminal (ACT) polypeptide), or a conservative variant thereof. In some embodiments, the compositions and methods provided herein are related to preventing, treating, and/or mitigating the progression of radiation injury. In some embodiments, the compositions and methods provided herein are related to preventing, treating, and/or mitigating the progression of cutaneous radiation injury (CRI). In some embodiments, the compositions and methods herein are related to preventing, treating, and/or mitigating the progression of acute radiation syndrome (ARS). In some embodiments, the compositions and methods herein are related to preventing, treating, and/or mitigating the progression of combined radiation injury.

Surprisingly, the present inventors found that the compositions and methods provided herein are capable of prophylactic action in radiation injury. For example, the compositions and methods provided herein prevent CRI, ARS, or combined radiation injury, and/or mitigate the progression of injury following radiation exposure but prior to symptom manifestation. Thus, the provided compositions and methods may be used to prevent, treat, or mitigate the progression of the particular types of radiation injury caused by radiological dispersal devices, improvised nuclear devices, nuclear weapons, nuclear power plant explosions, and the like, in populations of subject that are at high risk of exposure to such devices, weapons, and explosions, and/or in subjects who have recently experienced such an exposure.

ARS, CRI, and combined radiation injury are particular types of radiation injury that result, for example, from a dirty bomb attack or a nuclear power plant explosion. In some embodiments, the compositions and method herein are related to preventing, treating, and/or mitigating the progression of internal radiation injury as well as radiation injury to the skin and underlying tissues. In some embodiments, the compositions and methods herein are particularly useful for preventing and/or mitigating the progression of CR1 and/or ARS and/or combined radiation injury in populations of subjects who are at high risk of experiencing CR1 and/or ARS and/or combined radiation injury.

In some embodiments, the compositions provided herein are applied topically to a subject to treat, prevent, and/or mitigate the progression of an injury to the skin and underlying tissues as a result of exposure to radiation. In some embodiments, the compositions provided herein are administered systemically to a subject to treat, prevent, and/or mitigate the progression of systemic injury, such as injury to internal organs, that results from exposure to radiation. In some embodiments, the same subject is administered the one or more of the compositions provided herein both topically to treat, prevent, and/or mitigate the progression of injury to the skin and underlying tissues and systemically to treat, prevent, and/or mitigate the progression of internal damage (e.g., damage to systemic organs).

In addition, the surprising prophylactic action of the provided methods and compositions is useful for preventing, treating, and mitigating the progression of radiation injury that results from the sun, diagnostic machines, and tanning beds. Diagnostic machines that use ionizing radiation include, for example, x ray machines and computed tomography (CT) scanners. In some embodiments, the methods and compositions may be used to prevent, treat, or mitigate the progression of a radiation injury resulting from the sun, a diagnostic machine, or a tanning bed, in populations of subjects who are at high risk of exposure to levels of ionizing radiation from the sun, diagnostic machine, or tanning bed, or who have recently experienced such exposure. Thus, in some embodiments, the compositions and methods herein are applied to a subject prior to the subject's exposure to the sun, diagnostic machine, or tanning bed, and/or are applied to the subject after the subject's exposure to the sun, diagnostic machine, or tanning bed.

In some embodiments, the compositions provided herein are in the form of a sunscreen or sunblock, or a product that is available at a facility that provides tanning products and/or tanning bed services (e.g., a suntan lotion, tanning accelerator, or tanning oil). In some embodiments, the present disclosure provides methods and compositions for administration to a subject prior to the subject's use of a tanning bed or prior to the subject's exposure to the sun, in order to prevent, treat, or mitigate the progression of the radiation injury that may be caused by the use or exposure. In some embodiments, the present disclosure provides methods and compositions for administration to a subject after the subject's use of a tanning bed or after the subject's exposure to the sun, in order to prevent, treat, or mitigate the progression of the radiation injury caused by the use or exposure.

In some embodiments, the present disclosure provides methods and compositions for administration to a subject prior to exposure to a diagnostic machine or procedure that emits ionizing radiation, such as an x-ray or CT scan, wherein the methods and compositions prevent, treat, or mitigate the progression of the radiation injury that is caused by that exposure. In some embodiments, the present disclosure provides methods and compositions for administration to a subject after exposure to a diagnostic machine or procedure that emits ionizing radiation, wherein the methods and compositions prevent, treat, or mitigate the progression of the radiation injury that is caused by that exposure.

In addition, the surprising prophylactic action of the provided methods and compositions is useful for preventing, treating, and mitigating the progression of radiation dermatitis. Radiation dermatitis results from radiation therapy such as a therapy for treatment of diseases such as cancer. Thus, in some embodiments, the present disclosure provides methods and compositions for administration to a subject after the administration of a radiation therapy, in order to prevent, treat, or mitigate the progression of the radiation injury caused by the radiation therapy. In some embodiments, the present disclosure provides methods and compositions for administration to a subject after exposure to a radiation therapy, wherein the methods and compositions prevent, treat, or mitigate the progression of the radiation injury that is caused by the radiation therapy. In some embodiments, the methods and compositions provided herein are administered to the subject throughout the course of treatment with the radiation therapy, wherein the methods and compositions prevent, treat, or mitigate the progression of the radiation injury that is caused by the radiation therapy. In some embodiments, the methods and compositions provided herein are administered to the subject before, during, and/or after the course of the radiation therapy, wherein the methods and compositions prevent, treat, or mitigate the progression of the radiation injury that is caused by the radiation therapy.

It is to be understood that the disclosed compositions and methods are not limited to specific synthetic methods, specific analytical techniques, or to particular reagents unless otherwise specified, and, as such, may vary. It is also to be understood that the terminology used herein is for the purpose of describing particular embodiments only and is not intended to be limiting. Disclosed are materials, compositions, and components that can be used for, can be used in conjunction with, can be used in preparation for, or are products of the disclosed methods and compositions. These and other materials are disclosed herein, and it is understood that when combinations, subsets, interactions, groups, etc. of these materials are disclosed that while specific reference of each various individual and collective combinations and permutation of these compounds may not be explicitly disclosed, each is specifically contemplated and described herein. For example, if a vector is disclosed and discussed and a number of vector components including the promoters are discussed, each and every combination and permutation of promoters and other vector components and the modifications that are possible are specifically contemplated unless specifically indicated to the contrary. Thus, if a class of molecules A, B, and C are disclosed as well as a class of molecules D, E, and F and an example of a combination molecule, A-D is disclosed, then even if each is not individually recited, each is individually and collectively contemplated. Thus, is this example, each of the combinations A-E, A-F, B-D, B-E, B-F, C-D, C-E, and C-F are specifically contemplated and should be considered disclosed from disclosure of A, B, and C; D, E, and F; and the example combination A-D. Likewise, any subset or combination of these is also specifically contemplated and disclosed. Thus, for example, the sub-group of A-E, B-F, and C-E are specifically contemplated and should be considered disclosed from disclosure of A, B, and C; D, E, and F; and the example combination A-D. This concept applies to all aspects of this application including, but not limited to, steps in methods of making and using the disclosed compositions. Thus, if there are a variety of additional steps that can be performed it is understood that each of these additional steps can be performed with any specific embodiment or combination of embodiments of the disclosed methods, and that each such combination is specifically contemplated and should be considered disclosed.

A variety of sequences are provided herein and these and others can be found in Genbank at www.pubmed.gov. Those of skill in the art understand how to resolve sequence discrepancies and differences and to adjust the compositions and methods relating to a particular sequence to other related sequences. Primers and/or probes can be designed for any sequence given the information disclosed herein and known in the art.

The herein provided polypeptide can be any polypeptide comprising the carboxy-terminal most amino acids of an alpha Connexin, wherein the polypeptide does not comprise the full-length alpha Connexin protein. Thus, in one aspect, the provided polypeptide does not comprise the cytoplasmic N-terminal domain of the alpha Connexin. In another aspect, the provided polypeptide does not comprise the two extracellular domains of the alpha Connexin. In another aspect, the provided polypeptide does not comprise the four transmembrane domains of the alpha Connexin. In another aspect, the provided polypeptide does not comprise the cytoplasmic loop domain of the alpha Connexin. In another aspect, the provided polypeptide does not comprise that part of the sequence of the cytoplasmic carboxyl terminal domain of the alpha Connexin proximal to the fourth transmembrane domain. There is a conserved proline or glycine residue in alpha Connexins consistently positioned some 17 to 30 amino acids from the carboxyl terminal-most amino acid (Table 2). For example, for human Cx43 a proline residue at amino acid 363 is positioned 19 amino acids back from the carboxyl terminal most isoleucine. In another example, for chick Cx43 a proline residue at amino acid 362 is positioned 18 amino acids back from the carboxyl terminal-most isoleucine. In another example, for human Cx45 a glycine residue at amino acid 377 is positioned 19 amino acids back from the carboxyl terminal most isoleucine. In another example for rat Cx33, a proline residue at amino acid 258 is positioned 28 amino acids back from the carboxyl terminal most methionine. Thus, in another aspect, the provided polypeptide does not comprise amino acids proximal to said conserved proline or glycine residue of the alpha Connexin. Thus, the provided polypeptide can comprise the c-terminal-most 4 to 30 amino acids of the alpha Connexin, including the c-terminal most 4, 5, 6, 7, 8, 9, 10, 11, 12, 13, 14, 15, 16, 17, 18, 19, 20, 21, 22, 23, 24, 25, 26, 27, 28, 29, 30 amino acids of the alpha Connexin.

The carboxy-terminal most amino acids of an alpha Connexin in the provided peptides can be flanked by non-alpha Connexin or non-ACT peptide Connexin amino acids. Examples of the flanking non-alpha Connexin and non-ACT Connexin amino acids are provided herein. An example of non-ACT Connexin amino acids are the carboxy-terminal 20 to 120 amino acids of human Cx43 (SEQ ID NO: 72). Another example would be the carboxy-terminal 20 to 120 amino acids of chick Cx43 (SEQ ID NO: 73). Another example would be the carboxy-terminal 20 to 120 amino acids of human Cx45 (SEQ ID NO: 74). Another example would be the carboxy-terminal 20 to 120 amino acids of chick Cx45 (SEQ ID NO: 75). Another example would be the carboxy-terminal 20 to 120 amino of human Cx37 (SEQ ID NO: 76). Another example would be the carboxy-terminal 20 to 120 amino acids of rat Cx33 (SEQ ID NO: 77).

An example of a non-alpha Connexin is the 239 amino acid sequence of enhanced green fluorescent protein. In another aspect, given that ACT1 is shown to be functional when fused to the carboxy terminus of the 239 amino acid sequence of GFP, ACT peptides are expected to retain function when flanked with non-Connexin polypeptides of up to at least 239 amino acids. Indeed, as long as the ACT sequence is maintained as the free carboxy terminus of a given polypeptide, and the ACT peptide is able to access its targets. Thus, polypeptides exceeding 239 amino acids in addition to the ACT peptide can function in reducing inflammation, promoting healing, increasing tensile strength, reducing scarring and promoting tissue regeneration following injury.

Connexins are the sub-unit protein of the gap junction channel, which is responsible for intercellular communication (Goodenough and Paul, 2003). Based on patterns of conservation of nucleotide sequence, the genes encoding Connexin proteins are divided into two families termed the alpha and beta Connexin genes. The carboxy-terminal-most amino acid sequences of alpha Connexins are characterized by multiple distinctive and conserved features (see Table 2). This conservation of organization is consistent with the ability of ACT peptides to form distinctive 3D structures, interact with multiple partnering proteins, mediate interactions with lipids and membranes, interact with nucleic acids including DNA, transit and/or block membrane channels and provide consensus motifs for proteolytic cleavage, protein cross-linking, ADP-ribosylation, glycosylation and phosphorylation. Thus, the provided polypeptide interacts with a domain of a protein that normally mediates the binding of said protein to the carboxy-terminus of an alpha Connexin. For example, nephroblastoma overexpressed protein (NOV) interacts with a Cx43 c-terminal domain (Fu et al., J Biol. Chem. 2004 279(35):36943-50). It is considered that this and other proteins interact with the carboxy-terminus of alpha Connexins and further interact with other proteins forming a macromolecular complex. Thus, the provided polypeptide can inhibit the operation of a molecular machine, such as, for example, one involved in regulating the aggregation of Cx43 gap junction channels.

As used herein, "inhibit," "inhibiting," and "inhibition" mean to decrease an activity, response, condition, disease, or other biological parameter. This can include, but is not limited to, the complete loss of activity, response, condition, or disease. This can also include, for example, a 10% reduction in the activity, response, condition, or disease as compared to the native or control level. Thus, the reduction can be a 10, 20, 30, 40, 50, 60, 70, 80, 90, 100%, or any amount of reduction in between as compared to native or control levels.

The ACT sequence of the provided polypeptide can be from any alpha Connexin. Thus, the alpha Connexin component of the provided polypeptide can be from a human, murine, bovine, monotrene, marsupial, primate, rodent, cetacean, mammalian, avian, reptilian, amphibian, piscine, chordate, protochordate or other alpha Connexin.

Thus, the provided polypeptide can comprise an ACT of a Connexin selected from the group consisting of mouse Connexin 47, human Connexin 47, Human Connexin 46.6, Cow Connexin 46.6, Mouse Connexin 30.2, Rat Connexin 30.2, Human Connexin 31.9, Dog Connexin 31.9, Sheep Connexin 44, Cow Connexin 44, Rat Connexin 33, Mouse Connexin 33, Human Connexin 36, mouse Connexin 36, rat Connexin 36, dog Connexin 36, chick Connexin 36, zebrafish Connexin 36, morone Connexin 35, morone Connexin 35, Cynops Connexin 35, Tetraodon Connexin 36, human Connexin 37, chimp Connexin 37, dog Connexin 37, Cricetulus Connexin 37, Mouse Connexin 37, Mesocricetus Connexin 37, Rat Connexin 37, mouse Connexin 39, rat Connexin 39, human Connexin 40.1, Xenopus Connexin 38, Zebrafish Connexin 39.9, Human Connexin 40, Chimp Connexin 40, dog Connexin 40, cow Connexin 40, mouse Connexin 40, rat Connexin 40, Cricetulus Connexin 40, Chick Connexin 40, human Connexin 43, Cercopithecus Connexin 43, Oryctolagus Connexin 43, Spermophilus Connexin 43, Cricetulus Connexin 43, Phodopus Connexin 43, Rat Connexin 43, Sus Connexin 43, Mesocricetus Connexin 43, Mouse Connexin 43, Cavia Connexin 43, Cow Connexin 43, Erinaceus Connexin 43, Chick Connexin 43, Xenopus Connexin 43, Oryctolagus Connexin 43, Cyprinus Connexin 43, Zebrafish Connexin 43, Danio aequipinnatus Connexin 43, Zebrafish Connexin 43.4, Zebrafish Connexin 44.2, Zebrafish Connexin 44.1, human Connexin 45, chimp Connexin 45, dog Connexin 45, mouse Connexin 45, cow Connexin 45, rat Connexin 45, chick Connexin 45, Tetraodon Connexin 45, chick Connexin 45, human Connexin 46, chimp Connexin 46, mouse Connexin 46, dog Connexin 46, rat Connexin 46, Mesocricetus Connexin 46, Cricetulus Connexin 46, Chick Connexin 56, Zebrafish Connexin 39.9 cow Connexin 49, human Connexin 50, chimp Connexin 50, rat Connexin 50, mouse Connexin 50, dog Connexin 50, sheep Connexin 49, Mesocricetus Connexin 50, Cricetulus Connexin 50, Chick Connexin 50, human Connexin 59, or other alpha Connexin. Amino acid sequences for alpha connexins are known in the art and include those identified in Table 1 by accession number.

**Table 1: Alpha Connexins**

| **Protein** | **Accession No.** | **Protein** | **Accession No.** |
|---|---|---|---|
| mouse Connexin 47 | NP_536702 | Phodopus Connexin 43 | AAR33085 |
| human Connexin 47 | AAH89439 | Rat Connexin 43 | AAH81842 |
| Human Connexin46.6 | AAB94511 | Sus Connexin 43 | AAR33087 |
| Cow Connexin 46.6 | XP_582393 | Mesocricetus Connexin 43 | AAO61857 |
| Mouse Connexin 30.2 | NP _ 84871 1 | Mouse Connexin 43 | AAH55375 |
| Rat Connexin 30.2 | XP_343966 | Cavia Connexin 43 | AAU06305 |
| Human Connexin 31.9 | AAM18801 | Cow Connexin 43 | NP_776493 |
| Dog Connexin 31.9 | XP_548134 | Erinaceus Connexin 43 | AAR33083 |
| Sheep Connexin 44 | AAD56220 | Chick Connexin 43 | AAA53027 |
| Cow Connexin 44 | 146053 | Xenopus Connexin 43 | NP_988856 |
| Rat Connexin 33 | P28233 | Oryctolagus Connexin 43 | AAS89649 |
| Mouse Connexin 33 | AAR28037 | Cyprinus Connexin 43 | AAG17938 |
| Human Connexin 36 | Q9UKL4 | Zebrafish Connexin 43 | CAH69066 |
| mouse Connexin 36 | NP_034420 | Danio aequipinnatus Connexin 43 | AAC19098 |
| rat Connexin 36 | NP_062154 | Zebrafish Connexin 43.4 | NP_571144 |
| dog Connexin 36 | XP_544602 | Zebrafish Connexin 44.2 | AAH45279 |
| chick Connexin 36 | NP_989913 | Zebrafish Connexin 44.1 | NP_571884 |
| zebrafish Connexin 36 | NP_919401 | human Connexin45 | 138430 |
| morone Connexin 35 | AAC31884 | chimp Connexin45 | XP_511557 |
| morone Connexin 35 | AAC31885 | dog Connexin 45 | XP_548059 |
| Cynops Connexin 35 | BAC22077 | mouse Connexin 45 | AAH71230 |
| Tetraodon Connexin 36 | CAG06428 | cow Connexin 45 | XP_588395 |
| human Connexin 37 | 155593 | rat Connexin 45 | AAN17802 |
| chimp Connexin 37 | XP_524658 | chick Connexin45 | NP_990834 |
| dog Connexin 37 | XP_539602 | Tetraodon Connexin 45 | CAF93782 |
| Cricetulus Connexin 37 | AAR98615 | chick Connexin 45.6 | 150219 |
| Mouse Connexin 37 | AAH56613 | human Connexin 46 | NP_068773 |
| Mesocricetus Connexin37 | AAS83433 | chimp Connexin 46 | XP _522616 |
| Rat Connexin37 | AAH86576 | mouse Connexin 46 | NP_058671 |
| mouse Connexin 39 | NP_694726 | dog Connexin 46 | XP_543178 |
| rat Connexin 39 | AAN17801 | rat Connexin 46 | NP_077352 |
| human Connexin 40.1 | NP_699199 | Mesocricetus Connexin 46 | AAS83437 |
| Xenopus Connexin38 | AAH73347 | Cricetulus Connexin 46 | AAS77618 |
| Zebrafish Connexin 39.9 | NP _ 997991 | Chick Connexin 56 | A45338 |
| Human Connexin 40 | NP_859054 | Zebrafish Connexin 39.9 | NP_997991 |
| Chimp Connexin 40 | XP_513754 | cow Connexin 49 | XP_602360 |
| dog Connexin 40 | XP_540273 | human Connexin 50 | P48165 |
| cow Connexin 40 | XP_587676 | chimp Connexin 50 | XP_524857 |
| mouse Connexin 40 | AAH53054 | rat Connexin 50 | NP_703195 |
| rat Connexin 40 | AAH70935 | mouse Connexin 50 | AAG59880 |
| Cricetulus Connexin 40 | AAP37454 | dog Connexin 50 | XP_540274 |
| Chick Connexin 40 | NP_990835 | sheep Connexin 49 | AAF01367 |
| human Connexin 43 | P17302 | Mesocricetus Connexin 50 | AAS83438 |
| Cercopithecus Connexin 43 | AAR33082 | Cricetulus Connexin 50 | AAR98618 |
| Oryctolagus Connexin 43 | AAR33084 | Chick Connexin 50 | BAA05381 |
| Spermophilus Connexin 43 | AAR33086 | human Connexin 59 | AAG09406 |
| Cricetulus Connexin 43 | AAO61858 | | |

Thus, the provided polypeptide can comprise the amino acid sequence SEQ ID NO:1, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:43, SEQ ID NO:89, SEQ ID NO: 90, or SEQ. ID NO:91 or conservative variants or fragments thereof.

The 20-30 carboxy-terminal-most amino acid sequence of alpha Connexins are characterized by a distinctive and conserved organization. This distinctive and conserved organization would include a type II PDZ binding motif (Φ-x-Φ; wherein x = any amino acid and Φ =a Hydrophobic amino acid; e.g., Table 2, **BOLD**) and proximal to this motif, Proline (P) and/or Glycine (G) hinge residues; a high frequency phospho-Serine (S) and/or phospho-Threonine (T) residues; and a high frequency of positively charged Arginine (R), Lysine (K) and negatively charged Aspartic acid (D) or Glutamic acid (E) amino acids. For many alpha Connexins, the P and G residues occur in clustered motifs (e.g., Table 2, *italicized*) proximal to the carboxy-terminal type 11 PDZ binding motif. The S and T phosphor-amino acids of most alpha Connexins also are typically organized in clustered, repeat-like motifs (e.g., Table 2, underlined). This organization is particularly the case for Cx43, where 90% of 20 carboxyl terminal-most amino acids are comprised of the latter seven amino acids. In a further example of the high conservation of the sequence, ACT peptide organization of Cx43 is highly conserved from humans to fish (e.g., compare Cx43 ACT sequences for humans and zebrafish in Table 2). In another example, the ACT peptide organization of Cx45 is highly conserved from humans to birds (e.g., compare Cx45 ACT sequences for humans and chick in Table 2).). In another example, the ACT peptide organization of Cx36 is highly conserved from primates to fish (e.g., compare Cx36 ACT sequences for chimp and zebrafish in Table 2).

**Table 2. Alpha Connexin Carboxy-Terminal (ACT) Amino Acid Sequences**

| **Gene** | **Sequence** | **SEQ ID NO** |
|---|---|---|
| Human alpha Cx43 | P SSRA SSR *PRP* D **DLEI** | (SEQ ID NO:1) |
| Chick alpha Cx43 | P S RA SSRA SSR *PRP* D **DLEI** | (SEQ ID NO:29) |
| Zebrafish alpha Cx43 | P CSRA SSRM SSRA R P D **DLDV** | (SEQ ID NO:90) |
| Human alpha Cx45 | G SNKS TA SSKS *GDG* KN **SVWI** | (SEQ ID NO:30) |
| Chick alpha Cx45 | G SNKSS A SSKS *GDG* KN **SVWI** | (SEQ ID NO:31) |
| Human alpha Cx46 | **G** RA SKAS RASS *GRARP* E **DLAI** | (SEQ ID NO:32) |
| Human alpha Cx46.6 | **G** SASS RD GK **TVWI** | (SEQ ID NO:33) |
| Chimp alpha Cx36 | P RVSV *PNFG* R TQ SSD **SAYV** | (SEQ ID NO:34) |
| Chick alpha Cx36 | P RMSM *PNFG* R TQ SSD **SAYV** | (SEQ ID NO:35) |
| Zebrafish alpha Cx36 | P RMSM *PNFG* R TQ SSD **S AYV** | (SEQ ID NO:90) |
| Human alpha Cx47 | P RAGSEK G SASS R DG KT **TVWI** | (SEQ ID NO:36) |
| Human alpha Cx40 | **G** HRL ***PHG*** YHSDKRRL SKASS KARSD **DLSV** | (SEQ ID NO:37) |
| Human alpha Cx50 | P ELTTDDAR P LSRL SKASS RARSD **DLTV** | (SEQ ID NO:38) |
| Human alpha Cx59 | P NHVV SLTN NLI *GRRPP* T **DLQI** | (SEQ ID NO:39) |
| Rat alpha Cx33 | P S CV SSS A VLTTIC SS DQW *PVG* L SS **FYM** | (SEQ ID NO:40) |
| Sheep alpha Cx44 | G R SSKA SKSS *GG* RARAA **DLAI** | (SEQ ID NO:41) |
| Human beta Cx26 | LC YLLIR YCSGK SKKPV | (SEQ ID NO:42) |

Thus, in one aspect, the provided polypeptide comprises one, two, three or all of the amino acid motifs selected from the group consisting of 1) a type II PDZ binding motif, 2) Proline (P) and/or Glycine (G) hinge residues; 3) clusters of phospho-Serine (S) and/or phospho-Threonine (T) residues; and 4) a high frequency of positively charged Arginine (R) and Lysine (K) and negatively charged Aspartic acid (D) and/or Glutamic acid (E) amino acids). In another aspect, the provided polypeptide comprises a type 11 PDZ binding motif at the carboxy-terminus, Proline (P) and/or Glycine (G) hinge residues proximal to the PDZ binding motif, and positively charged residues (K, R, D, E) proximal to the hinge residues.

PDZ domains were originally identified as conserved sequence elements within the postsynaptic density protein PSD95/SAP90, the *Drosophila* tumor suppressor dlg-A, and the tight junction protein ZO-1. Although originally referred to as GLGF or DHR motifs, they are now known by an acronym representing these first three PDZ-containing proteins (PSD95/DLG/ZO-1). These 80-90 amino acid sequences have now been identified in well over 75 proteins and are characteristically expressed in multiple copies within a single protein. Thus, in one aspect, the provided polypeptide can inhibit the binding of an alpha Connexin to a protein comprising a PDZ domain. The PDZ domain is a specific type of protein-interaction module that has a structurally well-defined interaction 'pocket' that can be filled by a PDZ-binding motif, referred to herein as a "PDZ motif". PDZ motifs are consensus sequences that are normally, but not always, located at the extreme intracellular carboxyl terminus. Four types of PDZ motifs have been classified: type I (S/T-x-Φ), type II (Φ-x-Φ), type III (Ψ-x-Φ) and type IV (D-x-V), where x is any amino acid, Φ is a hydrophobic residue (V, I, L, A, G, W, C, M, F) and Ψ is a basic, hydrophilic residue (H, R, K). (Songyang, Z., et al. 1997. Science 275, 73-77). Thus, in one aspect, the provided polypeptide comprises a type II PDZ binding motif.

It is noted that the 18 carboxy-terminal-most amino acid sequence of alpha Cx37 represents an exceptional variation on the ACT peptide theme. The Cx37 ACT-like sequence is GQKPPSRPSSSASKKQ*YV (SEQ ID NO: 43). Thus the carboxy terminal 4 amino acids of Cx37 conform only in part to a type II PDZ binding domain. Instead of a classical type II PDZ binding domain, Cx37 has a neutral Q* at position 2 where a hydrophobic amino acid would be expected. As such Cx37 comprises what might be termed a type II PDZ binding domain-like sequence. Nonetheless, Cx37 strictly maintains all other aspects of ACT peptide organization including clustered serine residues, frequent R and K residues and a P-rich sequence proximal to the PDZ binding domain-like sequence. Given this overall level of conservation of ACT-like organization in common with the other >70 alpha Connexins listed above, it is understood that the Cx37 ACT-like carboxy terminus functions in the provided capacity.

For comparison, the beta Connexin Cx26 is shown in Table 2. Cx26 has no carboxyl terminal type II PDZ binding motif; less than 30% of the carboxyl terminal most amino acids comprise S, T, R, D or E residues; it has no evidence of motifs proximal to a type II PDZ binding motif or PDZ binding like motif containing clusters of P and G hinge residues; and no evidence of clustered, repeat-like motifs of serine and threonine phospho-amino acids. Cx26 does have three Lysine (K) residues, clustered one after the other near the carboxy terminus of the sequence. However, no alpha Connexin surveyed in the >70 alpha Connexins listed above was found to display this feature of three repeated K residues domain at carboxy terminus (Cx26 is a beta connexin, thus by definition does not have an ACT domain).

As provided herein, the unique functional characteristics of this relatively short stretch of amino acids encompass unexpected roles in reducing inflammation, promoting healing, reducing scarring, increasing tensile strength, and promoting regeneration of complex tissue structure and function following injury in tissues as diverse as skin and brain. Thus, in one aspect, the provided polypeptide comprises a type II PDZ binding motif (Φ-x-Φ; wherein x = any amino acid and Φ = a Hydrophobic amino acid). In another aspect, greater than 50%, 60%, 70%, 80%, 90% of the amino acids of the provided ACT polypeptide is comprised one or more of Proline (P), Glycine (G), phospho-Serine (S), phospho-Threonine (T), Arginine (R), Lysine (K), Aspartic acid (D), or Glutamic acid (E) amino acid residues.

The amino acids Proline (P), Glycine (G), Arginine (R), Lysine (K), Aspartic acid (D), and Glutamic acid (E) are necessary determinants of protein structure and function. Proline and Glycine residues provide for tight turns in the 3D structure of proteins, enabling the generation of folded conformations of the polypeptide required for function. Charged amino acid sequences are often located at the surface of folded proteins and are necessary for chemical interactions mediated by the polypeptide including protein-protein interactions, protein-lipid interactions, enzyme-substrate interactions and protein-nucleic acid interactions. Thus, in another aspect Proline (P) and Glycine (G) Lysine (K), Aspartic acid (D), and Glutamic acid (E) rich regions proximal to the type II PDZ binding motif provide for properties necessary to the provided actions of ACT peptides. In another aspect, the provided polypeptide comprises Proline (P) and Glycine (G) Lysine (K), Aspartic acid (D), and/or Glutamic acid (E) rich regions proximal to the type II PDZ binding motif.

Phosphorylation is the most common post-translational modification of proteins and is crucial tor modulating or modifying protein structure and function. Aspects of protein structure and function modified by phosphorylation include protein conformation, protein-protein interactions, protein-lipid interactions, protein-nucleic acid interactions, channel gating, protein trafficking and protein turnover. Thus, in one aspect the phospho-Serine (S) and/or phospho-Threonine (T) rich sequences are necessary for modifying the function of ACT peptides, increasing or decreasing efficacy of the polypeptides in their provided actions. In another aspect, the provided polypeptide comprise Serine (S) and/or phospho-Threonine (T) rich sequences or motifs.

In another example, respecting definition of an ACT peptide, it is highly auspicious, in light of the high degree of tissue/organ regeneration potential in lower animals such as fish, that a methionine occurs near the amino terminus of the ACT sequence of zebrafish Cx43 (Table 2). In addition to encoding methionine, the methionine base pair triplet is an alternate translation start site. If translation initiated from this methionine, the sequence SSRARPDDLDV (SEQ ID NO:89), would be produced. This translation product maintains all the conserved and distinctive features of a canonical ACT peptide. Specifically this peptide comprises a carboxy terminal type II PDZ binding domain and has a domain enriched in P, R and D residues proximal to the PDZ binding domain. In addition, the sequence comprises a clustered S motif, with potential to modulate ACT peptide function at its amino terminal. This raises the interesting prospect that animals with high tissue/organ regeneration potential such as fish may translate ACT peptides sequences directly.

Thus, the provided polypeptide can comprise the c-terminal sequence of human Cx43. Thus, the provided polypeptide can comprise the amino acid sequence SEQ ID NO:1 or SEQ ID NO:2. The polypeptide can comprise 9 amino acids of the carboxy terminus of human Cx40. Thus, the polypeptide can comprise the amino acid sequence SEQ ID NO:5.

When specific proteins are referred to herein, variants, derivatives, and fragments are contemplated. Protein variants and derivatives are well understood to those of skill in the art and in can involve amino acid sequence modifications. For example, amino acid sequence modifications typically fall into one or more of three classes: substitutional, insertional or deletional variants. Insertions include amino and/or carboxyl terminal fusions as well as intrasequence insertions of single or multiple amino acid residues. Insertions ordinarily will be smaller insertions than those of amino or carboxyl terminal fusions, for example, on the order of one to four residues. Deletions are characterized by the removal of one or more amino acid residues from the protein sequence. These variants ordinarily are prepared by site specific mutagenesis of nucleotides in the DNA encoding the protein, thereby producing DNA encoding the variant, and thereafter expressing the DNA in recombinant cell culture. Techniques for making substitution mutations at predetermined sites in DNA having a known sequence are well known and include, for example, M13 primer mutagenesis and PCR mutagenesis. Amino acid substitutions are typically of single residues, but can occur at a number of different locations at once; insertions usually will be on the order of about from 1 to 10 amino acid residues. Deletions or insertions preferably are made in adjacent pairs, i.e., a deletion of 2 residues or insertion of 2 residues. Substitutions, deletions, insertions or any combination thereof may be combined to arrive at a final construct. The mutations must not place the sequence out of reading frame and preferably will not create complementary regions that could produce secondary mRNA structure unless such a change in secondary structure of the mRNA is desired. Substitutional variants are those in which at least one residue has been removed and a different residue inserted in its place. Such substitutions generally are made in accordance with the following Table 3 and are referred to as conservative substitutions.

**TABLE 3: Amino Acid Substitutions**

| **Original Residue** | **Exemplary Substitutions** |
|---|---|
| Ala | Ser |
| Arg | Lys |
| Asn | Gln |
| Asp | Glu |
| Cys | Ser |
| Gln | Asn |
| Glu | Asp |
| Gly | Pro |
| His | Gln |
| Ile | Leu; Val |
| Leu | Ile; Val |
| Lys | Arg; Gln |
| Met | Leu; Ile |
| Phe | Met; Leu; Tyr |
| Pro | Gly |
| Ser | Thr |
| Thr | Ser |
| Trp | Tyr |
| Tyr | Trp; Phe |
| Val | Ile; Leu |

For example, the replacement of one amino acid residue with another that is biologically and/or chemically similar is known to those skilled in the art as a conservative substitution. For example, a conservative substitution would be replacing one hydrophobic residue for another, or one polar residue for another. The substitutions include combinations shown in Table 3. Conservatively substituted variations of each explicitly disclosed sequence are included within the polypeptides provided herein.

Typically, conservative substitutions have little to no impact on the biological activity of a resulting polypeptide. In a particular example, a conservative substitution is an amino acid substitution in a peptide that does not substantially affect the biological function of the peptide. A peptide can include one or more amino acid substitutions, for example 2-10 conservative substitutions, 2-5 conservative substitutions, 4-9 conservative substitutions, such as 2, 5 or 10 conservative substitutions.

A polypeptide can be produced to contain one or more conservative substitutions by manipulating the nucleotide sequence that encodes that polypeptide using, for example, standard procedures such as site-directed mutagenesis or PCR. Alternatively, a polypeptide can be produced to contain one or more conservative substitutions by using standard peptide synthesis methods. An alanine scan can be used to identify which amino acid residues in a protein can tolerate an amino acid substitution. In one example, the biological activity of the protein is not decreased by more than 25%, for example not more than 20%, for example not more than 10%, when an alanine, or other conservative amino acid (such as those listed below), is substituted for one or more native amino acids.

Further information about conservative substitutions can be found in, among other locations, in Ben-Bassat et al., (J. Bacteriol. 169:751-7, 1987), O'Regan et al., (Gene 77:237 51, 1989), Sahin Toth et al., (Protein Sci. 3:240-7, 1994), Hochuli ct al., (Bio/Technology 6:1321-5, 1988) and in standard textbooks of genetics and molecular biology.

Substitutional or deletional mutagenesis can be employed to insert sites for N-glycosylation (Asn-X-Thr/Ser) or O-glycosylation (Ser or Thr). Deletions of cysteine or other labile residues also may be desirable. Deletions or substitutions of potential proteolysis sites, e.g. Arg, is accomplished for example by deleting one of the basic residues or substituting one by glutaminyl or histidyl residues.

Certain post-translational derivatizations are the result of the action of recombinant host cells on the expressed polypeptide. Glutaminyl and asparaginyl residues are frequently post-translationally deamidated to the corresponding glutamyl and asparyl residues. Alternatively, these residues are deamidated under mildly acidic conditions. Other post-translational modifications include hydroxylation of proline and lysine, phosphorylation of hydroxyl groups of seryl or threonyl residues, methylation of the o-amino groups of lysine, arginine, and histidine side chains (T. E. Creighton, Proteins: Structure and Molecular Properties, W. H. Freeman & Co., San Francisco pp 79-86 [1983]), acetylation of the N-terminal amine and, in some instances, amidation of the C-terminal carboxyl.

It is understood that there are numerous amino acid and peptide analogs which can be incorporated into the disclosed compositions. For example, there are numerous D amino acids or amino acids which have a different functional substituent than the amino acids shown in Table 3. The opposite stereoisomers of naturally occurring peptides are disclosed, as well as the stereoisomers of peptide analogs. These amino acids can readily be incorporated into polypeptide chains by charging tRNA molecules with the amino acid of choice and engineering genetic constructs that utilize, for example, amber codons, to insert the analog amino acid into a peptide chain in a site specific way (Thorson et al., Methods in Molec. Biol. 77:43-73 (1991), Zoller, Current Opinion in Biotechnology, 3:348-354 (1992); Ibba, Biotechnology & Genetic Engineering Reviews 13:197-216 (1995), Cahill et al., TIBS, 14(10):400-403 (1989); Benner, TIB Tech, 12:158-163 (1994); Ibba and Hennecke, Bio/technology, 12:678-682 (1994).

Molecules can be produced that resemble polypeptides, but which are not connected via a natural peptide linkage. For example, linkages for amino acids or amino acid analogs can include CH₂NH--, --CH₂S--, --CH₂----, --CH=CH-- (cis and trans), --COCH₂--,-CH(OH)OH₂--, and --CHH₂SO-- (These and others can be found in Spatola, A. F. in Chemistry and Biochemistry of Amino Acids, Peptides, and Proteins, B. Weinstein, eds., Marcel Dekker, New York, p. 267 (1983); Spatola, A. F., Vega Data (March 1983), Vol. 1, Issue 3, Peptide Backbone Modifications (general review); Morley, Trends Pharm Sci (1980) pp. 463-468; Hudson, D. et al., Int J Pept Prot Res 14:177-185 (1979) (--CH₂NH--, CH₂CH₂--); Spatola et al. Life Sci 38:1243-1249 (1986) (--CH H₂--S); Hann J. Chem. Soc Perkin Trans. I 307-314 (1982) (--CH----, cis and trans); Almquist et al. J. Med. Chem. 23:1392-1398 (1980) (--COCH₂--); Jennings-White et al. Tetrahedron Lett 23:2533 (1982) (--COCH₂--); Szelke et al. European Appln, EP 45665 CA (1982): 97:39405 (1982) (-CH(OH)CH₂-); Holladay et al. Tetrahedron. Lett 24:4401-4404 (1983) (--C(OH)CH₂--); and Hruby Life Sci 31:189-199 (1982) (--CH₂--S--). It is understood that peptide analogs can have more than one atom between the bond atoms, such as b-alanine, g-aminobutyric acid, and the like.

Amino acid analogs and peptide analogs often have enhanced or desirable properties, such as, more economical production, greater chemical stability, enhanced pharmacological properties (half-life, absorption, potency, efficacy, etc.), altered specificity (e.g., a broad-spectrum of biological activities), reduced antigenicity, greater ability to cross biological barriers (e.g., gut, blood vessels, blood-brain-barrier), and others.

D-amino acids can be used to generate more stable peptides, because D amino acids are not recognized by peptidases and such. Systematic substitution of one or more amino acids of a consensus sequence with a D-amino acid of the same type (e.g., D-lysine in place of L-lysine) can be used to generate more stable peptides. Cysteine residues can be used to cyclize or attach two or more peptides together. This can be beneficial to constrain peptides into particular conformations. (Rizo and Gierasch Ann. Rev. Biochem. 61:387 (1992)).

Thus, the provided polypeptide can comprise a conservative variant of the c-terminus of an alpha Connexin (ACT). As shown in Table 4, an example of a single conservative substitution within the sequence SEQ ID NO:2 is given in the sequence SEQ ID NO:3. An example of three conservative substitutions within the sequence SEQ ID NO:2 is given in the sequence SEQ ID NO:4. Thus, the provided polypeptide can comprise the amino acid SEQ ID NO:3 or SEQ ID NO:4.

**Table 4. ACT Polypeptide Variants Sequence**

| **Sequence** | **SEQ ID NO** |
|---|---|
| RPRPDDLEI | SEQ ID NO:2 |
| RPRPDDLEV | SEQ ID NO:3 |
| RPRPDDVPV | SEQ ID NO:4 |
| SSRASSRASSRPRPDDLEV | SEQ ID NO:44 |
| RPKPDDLE1 | SEQ ID NO:45 |
| SSRASSRASSRPKPDDLEI | SEQ ID NO:46 |
| RPKPDDLDI | SEQ ID NO:47 |
| SSRASSRASSRPRPDDLDI | SEQ ID NO:48 |
| SSRASTRASSRPRPDDLEI | SEQ ID NO:49 |
| RPRPEDLEI | SEQ ID NO:50 |
| SSRASSRASSRPRPEDLEI | SEQ ID NO:51 |
| GDGKNSVWV | SEQ ID NO:52 |
| SKAGSNKSTASSKSGDGKNSVWV | SEQ ID NO:53 |
| GQKPPSRPSSSASKKLYV | SEQ ID NO: 54 |

It is understood that one way to define any variants, modifications, or derivatives of the disclosed genes and proteins herein is through defining the variants, modification, and derivatives in terms of sequence identity (also referred to herein as homology) to specific known sequences. Specifically disclosed are variants of the nucleic acids and polypeptides herein disclosed which have at least 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 percent sequence identity to the stated or known sequence. Those of skill in the art readily understand how to determine the sequence identity of two proteins or nucleic acids. For example, the sequence identity can be calculated after aligning the two sequences so that the sequence identity is at its highest level.

Another way of calculating sequence identity can be performed by published algorithms. Optimal alignment of sequences for comparison may be conducted by the local sequence identity algorithm of Smith and Waterman Adv. Appl. Math. 2: 482 (1981), by the sequence identity alignment algorithm of Needleman and Wunsch, J. Mol. Biol. 48: 443 (1970), by the search for similarity method of Pearson and Lipman, Proc. Natl. Acad. Sci. U.S.A. 85: 2444 (1988), by computerized implementations of these algorithms (GAP, BESTFIT, FASTA, and TFASTA in the Wisconsin Genetics Software Package, Genetics Computer Group, 575 referred to Science Dr., Madison, Wis.), or by inspection. These references are in their entirety for the methods of calculating sequence identity.

The same types of sequence identity can be obtained for nucleic acids by, for example, the algorithms disclosed in Zuker, M. Science 244:48-52, 1989, Jaeger et al. Proc. Natl. Acad. Sci. USA 86:7706-7710, 1989, Jaeger et al. Methods Enzymol. 183:281-306, 1989.

Thus, the provided polypeptide can comprise an amino acid sequence with at least 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 percent sequence identity to the c-terminus of an alpha Connexin (ACT). Thus, in one aspect, the provided polypeptide comprises an amino acid sequence with at least 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 percent sequence identity to SEQ ID NO:1, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO: 89 or SEQ ID NO:90. As an example, provided is a polypeptide (SEQ ID NO:4) having 66% sequence identity to the same stretch of 9 amino acids occurring on the carboxy-terminus of human Cx43 (SEQ ID NO:2).

The herein provided polypeptides can be added directly to a tissue injury in a subject. However, efficiency of cytoplasmic localization of the provided polypeptide is enhanced by cellular internalization transporter chemically linked in *cis* or *trans* with the polypeptide. Efficiency of cell internalization transporters are enhanced further by light or co-transduction of cells with Tat-HA peptide.

Thus, the provided polypeptide can comprise a cellular internalization transporter or sequence. The cellular internalization sequence can be any internalization sequence known or newly discovered in the art, or conservative variants thereof. Non-limiting examples of cellular internalization transporters and sequences include Antennapedia sequences, TAT, HIV-Tat, Penetratin, Antp-3A (Antp mutant), Buforin 11, Transportan, MAP (model amphipathic peptide), K-FGF, Ku70, Prion, pVEC, Pep-1, SynB1, Pep-7, HN-1, BGSC (Bis-Guanidinium-Spermidine-Cholesterol, and BGTC (Bis-Guanidinium-Tren-Cholesterol) (see Table 5).

**Table 5: Cell Internalization Transporters**

| **Name** | **Sequence** | **SEQ ID NO** |
|---|---|---|
| Antp | RQPKIWFPNRRKPWKK | (SEQ ID NO: 7) |
| HIV-Tat | GRKKRRQRPPQ | (SEQ ID NO:1 4) |
| Penetratin | RQIKIWFQNRRMKWKK | (SEQ ID NO: 1 5) |
| Antp-3A | RQIAIWFQNRRMKWAA | (SEQ ID NO: 1 6) |
| Tat | RKKRRQRRR | (SEQ ID NO: 1 7) |
| Buforin II | TRSSRAGLQFPVGRVHRLLRK | (SEQ ID NO: 18) |
| Transportan | GWTLNSAGYLLGKINKALAALA | (SEQ ID NO:1 9) |
| | KKIL | |
| model amphipathic peptide (MAP) | KLALKLALKALKAALKLA | (SEQ ID NO: 20) |
| K-FGF | AAVALLPAVLLALLAP | (SEQ ID NO: 21) |
| Ku70 | VPMLK-PMLKE | (SEQ ID NO: 22) |
| Prion | MANLGYWLLALFVTMWTDVGL | (SEQ ID NO: 23) |
| | CKKRPKP | |
| pVEC | LLIILRRRIRKQAHAHSK | (SEQ ID NO: 24) |
| Pep-1 | KETWWETWWTEWSQPKKKRKV | (SEQ ID NO: 25) |
| SynB1 | RGGRLSYSRRRFSTSTGR | (SEQ ID NO: 26) |
| Pep-7 | SDLWEMMMVSLACQY | (SEQ ID NO: 27) |
| HN-1 | TSPLNIHNGQKL | (SEQ ID NO: 28) |
| BGSC (Bis- Guanidinium-Spermidine- Cholesterol) | 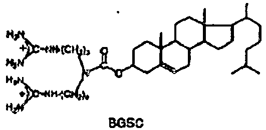 | |
| BGTC (Bis- Guanidinium-Tren- Cholesterol) | 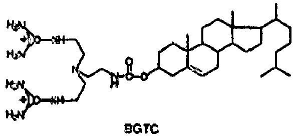 | |

Thus, the provided polypeptide can further comprise the amino acid sequence SEQ ID NO:7, SEQ ID NO: 14 (Bucci, M. et al. 2000. Nat. Med. 6, 1362-1367), SEQ ID NO:1 5 (Derossi, D., et al. 1994. Biol. Chem. 269, 10444-10450), SEQ ID NO:16 Fischer P. M. et al 2000. J. Pept. Res. 55, 163-172), SEQ ID NO:17 (Frankel, A. D. & Pabo, C. O. 1988. Cell 55, 1189-1193; Green, M. & Loewenstein, P. M. 1988. Cell 55, 1179-1188), SEQ ID NO:18 (Park, C. B., et al. 2000. Proc. Natl. Acad. Sci. USA 97, 8245-8250), SEQ ID NO:19 (Pooga, M., et al. 1998. FASEB J. 12, 67-77), SEQ ID NO:20 (Oehlke, J. et al. 1998. Biochim. Biophys. Acta. 1414, 127-139), SEQ ID NO:21 (Lin, Y. Z., et al. 1995. J. Biol. Chem. 270, 14255-14258), SEQ ID NO:22 (Sawada, M., et al. 2003. Nature Cell Biol. 5, 352-357), SEQ ID NO:23 (Lundberg, P. et al. 2002. Biochem. Biophys. Res. Commun. 299, 85-90), SEQ ID NO:24 (Elmquist, A., et al. 2001. Exp. Cell Res. 269, 237-244), SEQ ID NO:25 (Morris, M. C., et al. 2001. Nature Biotechnol. 19, 1173-1176), SEQ ID NO:26 (Rousselle, C. et al. 2000. Mol. Pharmacol. 57, 679-686), SEQ ID NO:27 (Gao, C. et al. 2002. Bioorg. Med. Chem. 10, 4057-4065), or SEQ ID NO:28 (Hong, F. D. & Clayman, G. L. 2000. Cancer Res. 60, 6551-6556). The provided polypeptide can further comprise BGSC (Bis-Guanidinium-Spermidine-Cholesterol) or BGTC (Bis-Guanidinium-Tren-Cholesterol) (Vigneron, J. P. et al. 1998. Proc. Natl. Acad. Sci. USA. 93, 9682-9686). The preceding references are hereby referred to in their entirety for the teachings of cellular internalization vectors and sequences. Any other internalization sequences now known or later identified can be combined with a peptide of the invention.

The provided polypeptide can comprise any ACT sequence (e.g, any of the ACT peptides disclosed herein) in combination with any of the herein provided cell internalization sequences. Examples of said combinations are given in Table 6. Thus, the provided polypeptide can comprise an Antennapedia sequence comprising amino acid sequence SEQ ID NO:7. Thus, the provided polypeptide can comprise the amino acid sequence SEQ ID NO:8, SEQ ID NO:9, SEQ ID NO:10, SEQ ID NO:11, or SEQ ID NO: 12.

**Table 6: ACT Polypeptides with Cell Internalization Sequences (CIS)**

| **CIS/ACT** | **Sequence** | **SEQ ID NO** |
|---|---|---|
| Antp/ ACT 2 | RQPKIWFPNRRKPWKK PSSRASSRASSRPRPDDLEI | SEQ ID NO:8 |
| Antp/ ACT 1 | RQPKIWFPNRRKPWKK RPRPDDLEI | SEQ ID NO:9 |
| Antp/ ACT 3 | RQPKIWFPNRRKPWKK RPRPDDLEV | SEQ ID NO:10 |
| Antp/ ACT 4 | RQPKIWFPNRRKPWKK RPRPDDVPV | SEQ ID NO:11 |
| Antp/ ACT 5 | RQPKIWFPNRRKPWKK KARSDDLSV | SEQ ID NO:12 |
| HIV-Tat/ ACT 1 | GRKKRRQRPPQ RPRPDDLEI | SEQ ID NO:56 |
| Penetratin/ ACT 1 | RQIKIWFQNRRMKWKK RPRPDDLEI | SEQ ID NO:57 |
| Antp-3 A/ ACT 1 | RQIAIWFQNRRMKWAA RPRPDDLEI | SEQ ID NO:58 |
| Tat/ ACT1 | RKKRRQRRR RPRPDDLEI | SEQ ID NO:59 |
| Buforin II/ ACT 1 | TRSSRAGLQFPVGRVHRLLRK RPRPDDLEI | SEQ ID NO:60 |
| Transportan/ ACT 1 | GWTLNSAGYLLGKINKALAALAKKIL RPRPDDLEI | SEQ ID NO:61 |
| MAP/ ACT 1 | KLALKLALKALKAALKLA RPRPDDLEI | SEQ ID NO:62 |
| K-FGF/ ACT 1 | AAVALLPAVLLALLAP RPRPDDLEI | SEQ ID NO:63 |
| Ku70/ ACT 1 | VPMLKPMLKE RPRPDDLEI | SEQ ID NO:64 |
| Prion/ ACT 1 | MANLGYWLLALFVTMWTDVGLCKKRPKP RPRPDDLEI | SEQ ID NO:65 |
| pVEC/ ACT 1 | LLIILRRRIRKQAHAHSK RPRPDDLEI | SEQ ID NO:66 |
| Pep-1/ ACT 1 | KETWWETWWTEWSQPKKKRKV RPRPDDLEI | SEQ ID NO:67 |
| SynB1/ ACT 1 | RGGRLSYSRRRFSTSTGR RPRPDDLEI | SEQ D NO:68 |
| Pep-7/ ACT 1 | SDLWEMMMVSLACQY RPRPDDLEI | SEQ ID NO:69 |
| HN-1/ ACT I | TSPLNIHNGQKL RPRPDDLEI | SEQ ID NO:70 |

Also provided are isolated nucleic acids encoding the polypeptides provided herein. The disclosed nucleic acids are made up of for example, nucleotides, nucleotide analogs, or nucleotide substitutes. Non-limiting examples of these and other molecules are discussed herein. It is understood that for example, when a vector is expressed in a cell, the expressed mRNA will typically be made up of A, C, G, and U.

By "isolated nucleic acid" or "purified nucleic acid" is meant DNA that is free of the genes that, in the naturally-occurring genome of the organism from which the DNA of the description description is derived, flank the gene. The term therefore includes, for example, a recombinant DNA which is incorporated into a vector, such as an autonomously replicating plasmid or virus; or incorporated into the genomic DNA of a prokaryote or eukaryote (e.g., a transgene); or which exists as a separate molecule (e.g., a cDNA or a genomic or cDNA fragment produced by PCR, restriction endonuclease digestion, or chemical or *in vitro* synthesis). It also includes a recombinant DNA, which is part of a hybrid gene encoding additional polypeptide sequence. The term "isolated nucleic acid" also refers to RNA, e.g., an mRNA molecule that is encoded by an isolated DNA molecule, or that is chemically synthesized, or that is separated or substantially free from at least some cellular components, e.g., other types of RNA molecules or polypeptide molecules.

Thus, provided is an isolated nucleic acid encoding a polypeptide comprising the amino acid sequence SEQ ID NO:1, SEQ ID NO:2, SEQ ID NO:3, SEQ ID NO:4, SEQ ID NO:5, SEQ ID NO:7, SEQ ID NO:8, SEQ ID NO:9, SEQ ID NO:10, SEQ ID NO:1 1, or SEQ ID NO:12.

Thus, the provided nucleic acid can comprise the nucleic acid sequence SEQ ID NO:79, SEQ ID NO:80, SEQ ID NO:81, SEQ ID NO:82, SEQ ID NO:83, SEQ ID NO:84, SEQ ID NO:85, SEQ ID NO:86, SEQ ID NO:87 SEQ ID NO:88, or SEQ ID NO:89.

The herein provided nucleic acid can be operably linked to an expression control sequence. Also provided is a vector comprising one or more of the herein provided nucleic acids, wherein the nucleic acid is operably linked to an expression control sequence. There are a number of compositions and methods which can be used to deliver nucleic acids to cells, either *in vitro* or *in vivo.* These methods and compositions can largely be broken down into two classes: viral based delivery systems and non-viral based delivery systems. For example, the nucleic acids can be delivered through a number of direct delivery systems such as, electroporation, lipofection, calcium phosphate precipitation, plasmids, viral vectors, viral nucleic acids, phage nucleic acids, phages, cosmids, or via transfer of genetic material in cells or carriers such as cationic liposomes. Appropriate means for transfection, including viral vectors, chemical transfectants, or physico-mechanical methods such as electroporation and direct diffusion of DNA, are described by, for example, Wolff, J. A., et al., Science, 247, 1465-1468, (1990); and Wolff, J. A. Nature, 352, 815-818, (1991). Such methods are well known in the art and readily adaptable for use with the compositions and methods described herein. In certain cases, the methods will be modified to specifically function with large DNA molecules. Further, these methods can be used to target certain diseases and cell populations by using the targeting characteristics of the carrier.

Transfer vectors can be any nucleotide construction used to deliver genes into cells (e.g., a plasmid), or as part of a general strategy to deliver genes, e.g., as part of recombinant retrovirus or adenovirus (Ram et al. Cancer Res. 53:83-88, (1993)).

As used herein, plasmid or viral vectors are agents that transport the disclosed nucleic acids, such as SEQ ID NO:6, into the cell without degradation and include a promoter yielding expression of the gene in the cells into which it is delivered. In some embodiments the promoters are derived from either a virus or a retrovirus. Viral vectors are, for example, Adenovirus, Adeno-associated virus, Herpes virus, Vaccinia virus, Polio virus, AIDS virus, neuronal trophic virus, Sindbis and other RNA viruses, including these viruses with the HIV backbone. Also disclosed are any viral families which share the properties of these viruses which make them suitable for use as vectors. Retroviruses include Murine Maloney Leukemia virus, MMLV, and retroviruses that express the desirable properties of MMLV as a vector. Retroviral vectors are able to carry a larger genetic payload, i.e., a transgene or marker gene, than other viral vectors, and for this reason are a commonly used vector. However, they are not as useful in non-proliferating cells. Adenovirus vectors are relatively stable and easy to work with, have high titers, and can be delivered in aerosol formulation, and can transfect non-dividing cells. Pox viral vectors are large and have several sites for inserting genes, they are thermostable and can be stored at room temperature. Also disclosed is a viral vector which has been engineered so as to suppress the immune response of the host organism, elicited by the viral antigens. Vectors of this type can carry coding regions for Interleukin 8 or 10.

Viral vectors can have higher transaction (ability to introduce genes) abilities than chemical or physical methods to introduce genes into cells. Typically, viral vectors contain, nonstructural early genes, structural late genes, an RNA polymerase III transcript, inverted terminal repeats necessary for replication and encapsidation, and promoters to control the transcription and replication of the viral genome. When engineered as vectors, viruses typically have one or more of the early genes removed and a gene or gene/promotor cassette is inserted into the viral genome in place of the removed viral DNA. Constructs of this type can carry up to about 8 kb of foreign genetic material. The necessary functions of the removed early genes are typically supplied by cell lines, which have been engineered to express the gene products of the early genes in trans.

A retrovirus is an animal virus belonging to the virus family of Retroviridae, including any types, subfamilies, genus, or tropisms. Retroviral vectors, in general, are described by Verma, T. M., Retroviral vectors for gene transfer. In Microbiology-1985, American Society for Microbiology, pp. 229-232, Washington, (1985) Examples of methods for using retroviral vectors for gene therapy are described in U.S. Pat. Nos. 4,868,116 and 4,980,286; PCT applications WO 90/02806 and WO 89/07136; and Mulligan, (Science 260:926-932 (1993)).

A retrovirus is essentially a package which has packed into it nucleic acid cargo. The nucleic acid cargo carries with it a packaging signal, which ensures that the replicated daughter molecules will be efficiently packaged within the package coat. In addition to the package signal, there are a number of molecules which are needed in cis, for the replication, and packaging of the replicated virus. Typically a retroviral genome, contains the gag, pol, and env genes which are involved in the making of the protein coat. It is the gag, pol, and env genes which are typically replaced by the foreign DNA that it is to be transferred to the target cell. Retrovirus vectors typically contain a packaging signal for incorporation into the package coat, a sequence which signals the start of the gag transcription unit, elements necessary for reverse transcription, including a primer binding site to bind the tRNA primer of reverse transcription, terminal repeat sequences that guide the switch of RNA strands during DNA synthesis, a purine rich sequence 5' to the 3' LTR that serve as the priming site for the synthesis of the second strand of DNA synthesis, and specific sequences near the ends of the LTRs that enable the insertion of the DNA state of the retrovirus to insert into the host genome. The removal of the gag, pol, and env genes allows for about 8 kb of foreign sequence to be inserted into the viral genome, become reverse transcribed, and upon replication be packaged into a new retroviral particle. This amount of nucleic acid is sufficient for the delivery of a one to many genes depending on the size of each transcript.

Since the replication machinery and packaging proteins in most retroviral vectors have been removed (gag, pol, and env), the vectors are typically generated by placing them into a packaging cell line. A packaging cell line is a cell line which has been transfected or transformed with a retrovirus that contains the replication and packaging machinery, but lacks any packaging signal. When the vector carrying the DNA of choice is transfected into these cell lines, the vector containing the gene of interest is replicated and packaged into new retroviral particles, by the machinery provided in cis by the helper cell. The genomes for the machinery are not packaged because they lack the necessary signals.

The construction of replication-defective adenoviruses has been described (Berkner et al., J. Virology 61:1213-1220 (1987); Massie et al., Mol. Cell. Biol. 6:2872-2883 (1986); Haj-Ahmad et al., J. Virology 57:267-274 (1986); Davidson et al., J. Virology 61:1226-1239 (1987); Zhang "Generation and identification of recombinant adenovirus by liposome-mediated transfection and PCR analysis" BioTechniques 15:868-872 (1993)). The benefit of the use of these viruses as vectors is that they are limited in the extent to which they can spread to other cell types, since they can replicate within an initial infected cell, but are unable to form new infectious viral particles. Recombinant adenoviruses have been shown to achieve high efficiency gene transfer after direct, *in vivo* delivery to airway epithelium, hepatocytes, vascular endothelium, CNS parenchyma and a number of other tissue sites (Morsy, J. Clin. Invest. 92:1580-1586 (1993); Kirshenbaum, J. Clin. Invest. 92:381-387 (1993); Roessler, J. Clin. Invest. 92:1085-1092 (1993); Moullier, Nature Genetics 4:154-159 (1993); La Salle, Science 259:988-990 (1993); Gomez-Foix, J. Biol. Chem. 267:25129-25134 (1992); Rich, Human Gene Therapy 4:461-476 (1993); Zabner, Nature Genetics 6:75-83 (1994); Guzman, Circulation Research 73:1201-1207 (1993); Bout, Human Gene Therapy 5:3-10 (1994); Zabner, Cell 75:207-216 (1993); Caillaud, Eur. J. Neuroscience 5:1287-1291 (1993); and Ragot, J. Gen. Virology 74:501-507 (1993)). Recombinant adenoviruses achieve gene transduction by binding to specific cell surface receptors, after which the virus is internalized by receptor-mediated endocytosis, in the same manner as wild type or replication-defective adenovirus (Chardonnet and Dales, Virology 40:462-477 (1970); Brown and Burlingham, J. Virology 12:386-396 (1973); Svensson and Persson, J. Virology 55:442-449 (1985); Seth, et al., J. Virol. 51:650-655 (1984); Seth, et al., Mol. Cell. Biol. 4:1528-1533 (1984); Varga et al., J. Virology 65:6061-6070 (1991); Wickham et al., Cell 73:309-319(1993)).

A viral vector can be one based on an adenovirus which has had the E1 gene removed, and these virons are generated in a cell line such as the human 293 cell line. In one aspect, both the E1 and E3 genes are removed from the adenovirus genome.

Another type of viral vector is based on an adeno-associated virus (AAV). This defective parvovirus can infect many cell types and is nonpathogenic to humans. AAV type vectors can transport about 4 to 5 kb and wild type AAV is known to stably insert into chromosome 19. As an example, this vector can be the P4.1 C vector produced by Avigen, San Francisco, Calif., which can contain the herpes simplex virus thymidine kinase gene, HSV-tk, and/or a marker gene, such as the gene encoding the green fluorescent protein, GFP.

In another type of AAV virus, the AAV contains a pair of inverted terminal repeats (ITRs) which flank at least one cassette containing a promoter which directs cell-specific expression operably linked to a heterologous gene. Heterologous in this context refers to any nucleotide sequence or gene which is not native to the AAV or B19 parvovirus.

Typically the AAV and B19 coding regions have been deleted, resulting in a safe, noncytotoxic vector. The AAV ITRs, or modifications thereof, confer infectivity and site-specific integration, but not cytotoxicity, and the promoter directs cell-specific expression. U.S. Pat. No. 6,261,834 is herein referred to for material related to the AAV vector.

The disclosed vectors thus provide DNA molecules which are capable of integration into a mammalian chromosome without substantial toxicity.

The inserted genes in viral and retroviral usually contain promoters, and/or enhancers to help control the expression of the desired gene product. A promoter is generally a sequence or sequences of DNA that function when in a relatively fixed location in regard to the transcription start site. A promoter contains core elements required for basic interaction of RNA polymerase and transcription factors, and may contain upstream elements and response elements.

Molecular genetic experiments with large human herpes viruses have provided a means whereby large heterologous DNA fragments can be cloned, propagated and established in cells permissive for infection with herpes viruses (Sun et al., Nature genetics 8: 33-41, 1994; Cotter and Robertson, Curr Opin Mol Ther 5: 633-644, 1999). These large DNA viruses (herpes simplex virus (HSV) and Epstein-Barr virus (EBV), have the potential to deliver fragments of human heterologous DNA >150 kb to specific cells. EBV recombinants can maintain large pieces of DNA in the infected B-cells as episomal DNA. Individual clones carried human genomic inserts up to 330 kb appeared genetically stable. The maintenance of these episomes requires a specific EBV nuclear protein, EBNA1, constitutively expressed during infection with EBV. Additionally, these vectors can be used for transfection, where large amounts of protein can be generated transiently *in vitro.* Herpesvirus amplicon systems are also being used to package pieces of DNA>220 kb and to infect cells that can stably maintain DNA as episomes.

Other useful systems include, for example, replicating and host-restricted non-replicating vaccinia virus vectors.

The disclosed compositions can be delivered to the target cells in a variety of ways. For example, the compositions can be delivered through electroporation, or through lipofection, or through calcium phosphate precipitation. The delivery mechanism chosen will depend in part on the type of cell targeted and whether the delivery is occurring for example *in vivo* or *in vitro.*

Thus, the compositions can comprise, in addition to the disclosed polypeptides, nucleic acids or vectors, for example, lipids such as liposomes, such as cationic liposomes (e.g., DOTMA, DOPE, DC-cholesterol) or anionic liposomes. Liposomes can further comprise proteins to facilitate targeting a particular cell, if desired. Administration of a composition comprising a compound and a cationic liposome can be administered to the blood afferent to a target organ or inhaled into the respiratory tract to target cells of the respiratory tract. Regarding liposomes, see, e.g., Brigham et al. Am. J. Resp. Cell. Mol. Biol. 1 :95-100 (1989); Felgner et al. Proc. Natl. Acad. Sci. USA 84:7413-7417 (1987); U.S. Pat. No. 4,897,355. Furthermore, the compound can be administered as a component of a microcapsule that can be targeted to specific cell types, such as macrophages, or where the diffusion of the compound or delivery of the compound from the microcapsule is designed for a specific rate or dosage.

In the methods described above which include the administration and uptake of exogenous DNA into the cells of a subject (i.e., gene transduction or transfection), delivery of the compositions to cells can be via a variety of mechanisms. As one example, delivery can be via a liposome, using commercially available liposome preparations such as LIPOFECTIN, LIPOFECTAMINE (GIBCO-BRL, Inc., Gaithersburg, Md.), SUPERFECT (Qiagen, Inc. Hilden, Germany) and TRANSFECTAM (Promega Biotec, Inc., Madison, Wis.), as well as other liposomes developed according to procedures standard in the art. In addition, the disclosed nucleic acid or vector can be delivered *in vivo* by electroporation, the technology for which is available from Genetronics, Inc. (San Diego, Calif.) as well as by means of a SONOPORATION machine (ImaRx Pharmaceutical Corp., Tucson, Ariz.).

Nucleic acids that are delivered to cells which are to be integrated into the host cell genome, typically contain integration sequences. These sequences are often viral related sequences, particularly when viral based systems are used. These viral integration systems can also be incorporated into nucleic acids which are to be delivered using a non-nucleic acid based system of deliver, such as a liposome, so that the nucleic acid contained in the delivery system can become integrated into the host genome.

Other general techniques for integration into the host genome include, for example, systems designed to promote homologous recombination with the host genome. These systems typically rely on sequence flanking the nucleic acid to be expressed that has enough homology with a target sequence within the host cell genome that recombination between the vector nucleic acid and the target nucleic acid takes place, causing the delivered nucleic acid to be integrated into the host genome. These systems and the methods necessary to promote homologous recombination are known to those of skill in the art.

The compositions can be delivered to the subject's cells *in vivo* and/or *ex vivo* by a variety of mechanisms well known in the art (e.g., uptake of naked DNA, liposome fusion, intramuscular injection of DNA via a gene gun, endocytosis and the like).

If *ex vivo* methods are employed, cells or tissues can be removed and maintained outside the body according to standard protocols well known in the art. The compositions can be introduced into the cells via any gene transfer mechanism, such as, for example, calcium phosphate mediated gene delivery, electroporation, microinjection or proteoliposomes. The transduced cells can then be infused (e.g., in a pharmaceutically acceptable carrier) or homotopically transplanted back into the subject per standard methods for the cell or tissue type. Standard methods are known for transplantation or infusion of various cells into a subject.

The nucleic acids that are delivered to cells typically contain expression controlling systems. For example, the inserted genes in viral and retroviral systems usually contain promoters, and/or enhancers to help control the expression of the desired gene product. A promoter is generally a sequence or sequences of DNA that function when in a relatively fixed location in regard to the transcription start site. A promoter contains core elements required for basic interaction of RNA polymerase and transcription factors, and may contain upstream elements and response elements.

Promoters controlling transcription from vectors in mammalian host cells may be obtained from various sources, for example, the genomes of viruses such as: polyoma, Simian Virus 40 (SV40), adenovirus, retroviruses, hepatitis-B virus, cytomegalovirus, or from heterologous mammalian promoters, e.g. beta actin promoter. The early and late promoters of the SV40 virus are conveniently obtained as an SV40 restriction fragment which also contains the SV40 viral origin of replication (Fiers et al., Nature, 273: 113 (1978)). The immediate early promoter of the human cytomegalovirus is conveniently obtained as a HindIII E restriction fragment (Greenway, P. J. et al., Gene 18: 355-360 (1982)). Of course, promoters from the host cell or related species also are useful herein.

Enhancer generally refers to a sequence of DNA that functions at no fixed distance from the transcription start site and can be either 5' (Laimins, L. et al., Proc. Natl. Acad. Sci. 78: 993 (1981)) or 3' (Lusky, M. L., et al., Mol. Cell. Bio. 3: 1108 (1983)) to the transcription unit. Furthermore, enhancers can be within an intron (Banerji, J. L. et al., Cell 33: 729 (1983)) as well as within the coding sequence itself (Osborne, T. F., et al., Mol. Cell. Bio. 4: 1293 (1984)). They are usually between 10 and 300 bp in length, and they function in cis. Enhancers function to increase transcription from nearby promoters. Enhancers also often contain response elements that mediate the regulation of transcription. Promoters can also contain response elements that mediate the regulation of transcription. Enhancers often determine the regulation of expression of a gene. While many enhancer sequences are now known from mammalian genes (globin, elastase, albumin, α-fetoprotein and insulin), typically one will use an enhancer from a eukaryotic cell virus for general expression. Examples are the SV40 enhancer on the late side of the replication origin (bp 100-270), the cytomegalovirus early promoter enhancer, the polyoma enhancer on the late side of the replication origin, and adenovirus enhancers.

The promoter and/or enhancer may be specifically activated either by light or specific chemical events which trigger their function. Systems can be regulated by reagents such as tetracycline and dexamethasone. There are also ways to enhance viral vector gene expression by exposure to irradiation, such as gamma irradiation, or alkylating chemotherapy drugs.

In certain embodiments the promoter and/or enhancer region can act as a constitutive promoter and/or enhancer to maximize expression of the region of the transcription unit to be transcribed. In certain constructs the promoter and/or enhancer region be active in all eukaryotic cell types, even if it is only expressed in a particular type of cell at a particular time. A promoter of this type is the CMV promoter (650 bases). Other such promoters are SV40 promoters, cytomegalovirus (full length promoter), and retroviral vector LTR.

It has been shown that all specific regulatory elements can be cloned and used to construct expression vectors that are selectively expressed in specific cell types such as melanoma cells. The glial fibrillary acetic protein (GFAP) promoter has been used to selectively express genes in cells of glial origin.

Expression vectors used in eukaryotic host cells (yeast, fungi, insect, plant, animal, human or nucleated cells) may also contain sequences necessary for the termination of transcription which may affect mRNA expression. These regions are transcribed as polyadenylated segments in the untranslated portion of the mRNA encoding tissue factor protein. The 3' untranslated regions also include transcription termination sites. The transcription unit can also contain a polyadenylation region. One benefit of this region is that it increases the likelihood that the transcribed unit will be processed and transported like mRNA. The identification and use of polyadenylation signals in expression constructs is well established. Homologous polyadenylation signals can be used in the transgene constructs. In certain transcription units, the polyadenylation region is derived from the SV40 early polyadenylation signal and consists of about 400 bases. Transcribed units an contain other standard sequences alone or in combination with the above sequences improve expression from, or stability of, the construct.

The viral vectors can include nucleic acid sequence encoding a marker product. This marker product is used to determine if the gene has been delivered to the cell and once delivered is being expressed. Example marker genes are the *E. Coli* lacZ gene, which encodes β-galactosidase, and green fluorescent protein.

In some embodiments the marker may be a selectable marker. Examples of suitable selectable markers for mammalian cells are dihydrofolate reductase (DHFR), thymidine kinase, neomycin, neomycin analog G418, hydromycin, and puromycin. When such selectable markers are successfully transferred into a mammalian host cell, the transformed mammalian host cell can survive if placed under selective pressure. There are two widely used distinct categories of selective regimes. The first category is based on a cell's metabolism and the use of a mutant cell line which lacks the ability to grow independent of a supplemented media. Two examples are: Chinese hamster ovary (CHO) DHFR-cells and mouse LTK-cells. These cells lack the ability to grow without the addition of such nutrients as thymidine or hypoxanthine. Because these cells lack certain genes necessary for a complete nucleotide synthesis pathway, they cannot survive unless the missing nucleotides are provided in a supplemented media. An alternative to supplementing the media is to introduce an intact DHFR or TK gene into cells lacking the respective genes, thus altering their growth requirements. Individual cells which were not transformed with the DHFR or TK gene will not be capable of survival in non-supplemented media.

The second category is dominant selection which refers to a selection scheme used in any cell type and does not require the use of a mutant cell line. These schemes typically use a drug to arrest growth of a host cell. Those cells which have a novel gene would express a protein conveying drug resistance and would survive the selection. Examples of such dominant selection use the drugs neomycin, (Southern P. and Berg, P., J. Molec. Appl. Genet. 1:327 (1982)), mycophenolic acid, (Mulligan, R. C. and Berg, P. Science 209: 1422 (1980)) or hygromycin, (Sugden, B. et al., Mol. Cell. Biol. 5: 410-413 (1985)). The three examples employ bacterial genes under eukaryotic control to convey resistance to the appropriate drug G418 or neomycin (geneticin), xgpt (mycophenolic acid) or hygromycin, respectively. Others include the neomycin analog G418 and puramycin.

Also provided is a cell comprising one or more of the herein provided vectors. As used herein, "cell", "cell line", and "cell culture" may be used interchangeably and all such designations include progeny. The disclosed cell can be any cell used to clone or propagate the vectors provided herein. Thus, the cell can be from any primary cell culture or established cell line. The method may be applied to any cell, including prokaryotic or eukaryotic, such as bacterial, plant, animal, and the like. The cell type can be selected by one skilled in the art based on the choice of vector and desired use.

Disclosed are animals produced by the process of transfecting a cell within the animal with any of the nucleic acid molecules or vectors disclosed herein. Disclosed are animals produced by the process of transfecting a cell within the animal any of the nucleic acid molecules or vectors disclosed herein, wherein the animal is a mammal. Also disclosed are animals produced by the process of transfecting a cell within the animal any of the nucleic acid molecules or vectors disclosed herein, wherein the mammal is mouse, rat, rabbit, cow, sheep, pig, or primate.

Provided is a composition comprising one or more of the herein provided polypeptides, nucleic acids, or vectors in a pharmaceutically acceptable carrier. Thus, provided is a composition comprising a combination of two or more of any of the herein provided ACT polypeptides in a pharmaceutically acceptable carrier. For example, provided is a composition comprising SEQ ID NO:1 and SEQ ID NO:5 in a pharmaceutically acceptable carrier.

By "pharmaceutically acceptable" is meant a material that is not biologically or otherwise undesirable, i.e., the material may be administered to a subject, along with the nucleic acid or vector, without causing any undesirable biological effects or interacting in a deleterious manner with any of the other components of the pharmaceutical composition in which it is contained. The carrier would naturally be selected to minimize any degradation of the active ingredient and to minimize any adverse side effects in the subject, as would be well known to one of skill in the art.

The herein provide composition can further comprise any known or newly discovered substance that can be administered to a wound, tissue injury, site of inflammation or cancer. For example, the provided composition can further comprise one or more of classes of **antibiotics** (e.g. Aminoglycosides, Cephalosporins, Chloramphenicol, Clindamycin, Erythromycins, Fluoroquinolones, Macrolides, Azolides, Metronidazole, Penicillin's, Tetracycline's, Trimethoprim-sulfamethoxazole, Vancomycin), **steroids** (e.g. Andranes (e.g. Testosterone), Cholestanes (e.g. Cholesterol), Cholic acids (e.g. Cholic acid), Corticosteroids (e.g. Dexamethasone), Estraenes (e.g. Estradiol), Pregnanes (e.g. Progesterone), **narcotic and non-narcotic analgesics** (e.g. Morphine, Codeine, Heroin, Hydromorphone, Levorphanol, Meperidine, Methadone, Oxydone, Propoxyphene, Fentanyl, Methadone, Naloxone, Buprenorphine, Butorphanol, Nalbuphine, Pentazocine), **chemotherapy** (e.g. **anti-cancer drugs** such as but not limited to Altretamine, Asparaginase, Bleomycin, Busulfan, Carboplatin, Carrnustine, Chlorambucil, Cisplatin, Cladribine, Cyclophosphamide, Cytarabine, Dacarbazine, Diethylstilbesterol, Ethinyl estradiol, Etoposide, Floxuridine, Fludarabine, Fluorouracil, Flutamide, Goserelin, Hydroxyurea, Idarubicin, Ifosfamide, Leuprolide, Levamisole, Lomustine, Mechlorethamine, Medroxyprogesterone, Megestrol, Melphalan, Mercaptopurine, Methotrexate, Mitomycin, Mitotane, Mitoxantrone, Paclitaxel, pentastatin, Pipobroman, Plicamycin, Prednisone, Procarbazine, Streptozocin, Tamoxifen, Teniposide, Vinblastine, Vincristine), **anti-inflammatory agents** (e.g. Alclofenac; Alclometasone Dipropionate; Algestone Acetonide; alpha Amylase; Amcinafal; Amcinafide; Amfenac Sodium; Amiprilose Hydrochloride; Anakinra; Anirolac; Anitrazafen; Apazone; Balsalazide Disodium; Bendazac; Benoxaprofen; Benzydamine Hydrochloride; Bromelains; Broperamole; Budesonide; Carprofen; Cicloprofen; Cintazone; Cliprofen; Clobetasol Propionate; Clobetasone Butyrate; Clopirac; Cloticasone Propionate; Cormethasone Acetate; Cortodoxone; Decanoate; Deflazacort; Delatestryl; Depo-Testosterone; Desonide; Desoximetasone; Dexamethasone Dipropionate; Diclofenac Potassium; Diclofenac Sodium; Diflorasone Diacetate; Diflumidone Sodium; Diflunisal; Difluprednate; Diftalone; Dimethyl Sulfoxide; Drocinonide; Endrysone; Enlimomab; Enolicam Sodium; Epirizole; Etodolac; Etofenamate; Felbinac; Fenamole; Fenbufen; Fenclofenac; Fenclorac; Fendosal; Fenpipalone; Fentiazac; Flazalone; Fluazacort; Flufenamic Acid; Flumizole; Flunisolide Acetate; Flunixin; Flunixin Meglumine; Fluocortin Butyl; Fluorometholone Acetate; Fluquazone; Flurbiprofen; Fluretofen; Fluticasone Propionate; Furaprofen; Furobufen; Halcinonide; Halobetasol Propionate; Halopredone Acetate; Ibufenac; Ibuprofen; Ibuprofen Aluminum; Ibuprofen Piconol; Ilonidap; Indomethacin; Indomethacin Sodium; Indoprofen; Indoxole; Intrazole; Isoflupredone Acetate; Isoxepac; Isoxicam; Ketoprofen; Lofemizole Hydrochloride; Lomoxicam; Loteprednol Etabonate; Meclofenamate Sodium; Meclofenamic Acid; Meclorisone Dibutyrate; Mefenamic Acid; Mesalamine; Meseclazone; Mesterolone; Methandrostenolone; Methenolone; Methenolone Acetate; Methylprednisolone Suleptanate; Momiflumate; Nabumetone; Nandrolone; Naproxen; Naproxen Sodium; Naproxol; Nimazone; Olsalazine Sodium; Orgotein; Orpanoxin; Oxandrolane; Oxaprozin; Oxyphenbutazone; Oxymetholone; Paranyline Hydrochloride; Pentosan Polysulfate Sodium; Phenbutazone Sodium Glycerate; Pirfenidone; Piroxicam; Piroxicam Cinnamate; Piroxicam Olamine; Pirprofen; Prednazate; Prifelone; Prodolic Acid; Proquazone; Proxazole; Proxazole Citrate; Rimexolone; Romazarit; Salcolex; Salnacedin; Salsalate; Sanguinarium Chloride; Seclazone; Sermetacin; Stanozolol; Sudoxicam; Sulindac; Suprofen; Talmetacin; Talniflumate; Talosalate; Tebufelone; Tenidap; Tenidap Sodium; Tenoxicam; Tesicam; Tesimide; Testosterone; Testosterone Blends; Tetrydamine; Tiopinac; Tixocortol Pivalate; Tolmetin; Tolmetin Sodium; Triclonide; Triflumidate; Zidometacin; Zomepirac Sodium), or **anti-histaminic agents** (e.g. Ethanolamines (like diphenhydrmine carbinoxamine), Ethylenediamine (like tripelennamine pyrilamine), Alkylamine (like chlorpheniramine, dexchlorpheniramine, brompheniramine, triprolidine), other anti-histamines like astemizole, loratadine, fexofenadine, Bropheniramine, Clemastine, Acetaminophen, Pseudoephedrine, Triprolidine).

The compositions may be administered topically, orally, or parenterally. For example, the compositions can be administered extracorporeally, intracranially, intravaginally, intraanally, subcutaneously, intradermally, intracardiac, intragastric, intravenously, intramuscularly, by intraperitoneal injection, transdermally, intranasally, or by inhalant. As used herein, "intracranial administration" means the direct delivery of substances to the brain including, for example, intrathecal, intracisternal, intraventricular or trans-sphenoidal delivery via catheter or needle.

Parenteral administration of the composition, if used, is generally characterized by injection. Injectables can be prepared in conventional forms, either as liquid solutions or suspensions, solid forms suitable for solution of suspension in liquid prior to injection, or as emulsions. A more recently revised approach for parenteral administration involves use of a slow release or sustained release system such that a constant dosage is maintained. See, e.g., U.S. Pat. No. 3,610,795.

As used herein, "topical intranasal administration" means delivery of the compositions into the nose and nasal passages through one or both of the nares and can comprise delivery by a spraying mechanism or droplet mechanism, or through aerosolization of the nucleic acid or vector. Administration of the compositions by inhalant can be through the nose or mouth via delivery by a spraying or droplet mechanism. Delivery can also be directly to any area of the respiratory system (e.g., lungs) via intubation.

In some embodiments, the compositions provided herein may be applied topically once per day, twice per day, three times per day, four times per day, five times per day, or more. In some embodiments, the compositions provided herein may be applied topically once every two days, once every three days, once every four days, once every four days, once every five days, once every six days, or once per week. In some embodiments, the compositions provided herein may be applied topically on an as-needed basis. For example, in some embodiments, the compositions provided herein may be applied during or after the manifestation or increasing severity of symptoms of the radiation injury. In some embodiments, the compositions provided herein may be applied topically once per day for about 5, about 10, about 15, about 20, about 25, about 30, about 35, about 40, about 45, about 50, about 55, about 60, about 65, about 70, about 75, about 80, about 85, about 90, about 95, about 100, or more consecutive days. In some embodiments, the compositions provided herein comprise an ACT peptide provided herein and a gel, and may be topically applied. In further embodiments, the compositions provided herein comprise SEQ ID NO: 91. In some embodiments, the compositions provided herein comprise SEQ ID NO: 91 and hydroxyethylcellulose gel, wherein the compositions are topically applied.

In some embodiments, the compositions provided herein include a topical gel termed "Granexin^{®}". In some embodiments, Granexin^{®} comprises 1.25% hydroxyethyl cellulose gel and the ACT1 peptide. The chemical structure of the ACT1 peptide in Granexin^{®} is: Biotin-Ahx-Arg-Gln-Pro-Lys-Ile-Trp-Phe-Pro-Asn-Arg-Arg-Lys-Pro-Trp-Lys-Lys-Arg-Pro-Arg-Pro-Asp-Asp-Leu-Glu-Ile-OH (SEQ ID NO: 91), wherein Ahx is L-2-aminohexanoic acid (6-aminohexanoic acid). In some embodiments, Granexin^{®} further comprises one or more preservative, solvent, buffer agent, stabilizer, chelating agent, and/or any additional pharmaceutically acceptable excipient or carrier.

In some embodiments, the compositions provided herein are topically and/or systemically administered about 1, about 2, about 5, about 10, about 15, about 20, about 24, about 48, or more hours prior to exposure to ionizing radiation. In some embodiments, the compositions provided herein are topically and/or systemically administered about 1, about 2, about 5, about 10, about 15, about 20, about 24, about 48, or more hours post exposure to ionizing radiation. In some embodiments, compositions provided herein are topically and/or systemically administered in a window of time of about 1 to about 48 hours post exposure to ionizing radiation, or about 4 to about 24 hours post exposure, or about 10 to about 36 hours post exposure. In particular embodiments, the compositions provided herein are topically applied about 24 hours post exposure to ionizing radiation. In some embodiments, a thin layer of a composition provided herein (e.g., a composition comprising an ACT peptide provided herein and a gel) is applied over the areas exposed to the ionizing radiation. Thus, in some embodiments, the present disclosure provides methods for treatment of exposure to ionizing radiation, comprising topically applying to the area of exposure a layer of a composition comprising SEQ ID NO: 91 and hydroxyethylcellulose gel.

In some embodiments, the compositions provided herein may be administered systemically once per day, twice per day, three times per day, four times per day, five times per day, or more. In some embodiments, the compositions provided herein may be administered systemically once every two days, once every three days, once every four days, once every four days, once every five days, once every six days, or once per week. In some embodiments, the compositions provided herein may be administered systemically on an as-needed basis prior to or after exposure to ionizing radiation. For example, in some embodiments, the compositions provided herein may be administered during or after the manifestation or increasing severity of symptoms of the radiation injury. In some embodiments, the compositions provided herein may be administered systemically once per day for about 5, about 10, about 15, about 20, about 25, about 30, about 35, about 40, about 45, about 50, about 55, about 60, about 65, about 70, about 75, about 80, about 85, about 90, about 95, about 100, or more consecutive days. In some embodiments, the compositions provided herein for systemic delivery to a subject in need thereof comprise an ACT peptide provided herein. In certain embodiments, the compositions provided herein for systemic delivery to a subject in need thereof comprise ACTI polypeptide (SEQ ID NO: 2). In further embodiments, the compositions provided herein for systemic delivery to a subject in need thereof comprise one or more pharmaceutically acceptable carrier.

In some embodiments, the compositions provided herein are systemically administered about 1, about 2, about 5, about 10, about 15, about 20, about 24, about 48, or more hours post exposure to ionizing radiation. In particular embodiments, the compositions provided herein are systemically administered about 24 hours post exposure to ionizing radiation. In some embodiments, the compositions provided herein are systemically administered post exposure to ionizing radiation and prior to manifestation of symptoms of ARS. In some embodiments, the compositions provided herein are systemically administered post exposure to ionizing radiation and after manifestation of symptoms of ARS, wherein the compositions treat, inhibit, or mitigate the progression of ARS.

The exact amount of the compositions required will vary from subject to subject, depending on the species, age, weight and general condition of the subject, the severity of the allergic disorder being treated, the particular nucleic acid or vector used, its mode of administration and the like. Thus, it is not possible to specify an exact amount for every composition. However, an appropriate amount can be determined by one of ordinary skill in the art using only routine experimentation given the teachings herein.

The materials may be in solution or suspension (for example, incorporated into microparticles, liposomes, or cells). These may be targeted to a particular cell type via antibodies, receptors, or receptor ligands. The following references are examples of the use of this technology to target specific proteins to tumor tissue (Senter, et al., Bioconjugate Chem., 2:447-451, (1991); Bagshawe, K. D., Br. J. Cancer, 60:275-281, (1989); Bagshawe, et al., Br. J. Cancer, 58:700-703, (1988); Senter, et al., Bioconjugate Chem., 4:3-9, (1993); Battelli, et al., Cancer Immunol. Immunother., 35:421-425, (1992); Pietersz and McKenzie, Immunolog. Reviews, 129:57-80, (1992); and Roffler, et al., Biochem. Pharmacol, 42:2062-2065, (1991)). Vehicles such as "stealth" and other antibody conjugated liposomes (including lipid mediated drug targeting to colonic carcinoma), receptor mediated targeting of DNA through cell specific ligands, lymphocyte directed tumor targeting, and highly specific therapeutic retroviral targeting of murine glioma cells *in vivo.* The following references are examples of the use of this technology to target specific proteins to tumor tissue (Hughes et al., Cancer Research, 49:6214-6220, (1989); and Litzinger and Huang, Biochimica et Biophysica Acta, 1104:179-187, (1992)). In general, receptors are involved in pathways of endocytosis, either constitutive or ligand induced. These receptors cluster in clathrin-coated pits, enter the cell via clathrin-coated vesicles, pass through an acidified endosome in which the receptors are sorted, and then either recycle to the cell surface, become stored intracellularly, or are degraded in lysosomes. The internalization pathways serve a variety of functions, such as nutrient uptake, removal of activated proteins, clearance of macromolecules, opportunistic entry of viruses and toxins, dissociation and degradation of ligand, and receptor-level regulation. Many receptors follow more than one intracellular pathway, depending on the cell type, receptor concentration, type of ligand, ligand valency, and ligand concentration. Molecular and cellular mechanisms of receptor-mediated endocytosis has been reviewed (Brown and Greene, DNA and Cell Biology 10:6, 399-409 (1991)).

Suitable carriers and their formulations are described in Remington: The Science and Practice of Pharmacy (19th ed.) ed. A. R. Gennaro, Mack Publishing Company, Easton, Pa. 1995. Typically, an appropriate amount of a pharmaceutically-acceptable salt is used in the formulation to render the formulation isotonic. Examples of the pharmaceutically-acceptable carrier include, but are not limited to, saline, Ringer's solution and dextrose solution. The pH of the solution can be from about 5 to about 8, from about 7 to about 7.5. Further carriers include sustained release preparations such as semipermeable matrices of solid hydrophobic polymers containing the antibody, which matrices are in the form of shaped articles, e.g., films, liposomes or microparticles. It will be apparent to those persons skilled in the art that certain carriers may be more preferable depending upon, for instance, the route of administration and concentration of composition being administered.

Pharmaceutical carriers are known to those skilled in the art. These most typically would be standard carriers for administration of drugs to humans, including solutions such as sterile water, saline, and buffered solutions at physiological pH. The compositions can be administered intramuscularly or subcutaneously. Other compounds will be administered according to standard procedures used by those skilled in the art.

Pharmaceutical compositions may include carriers, thickeners, diluents, buffers, preservatives, surface active agents and the like in addition to the molecule of choice. Pharmaceutical compositions may also include one or more active ingredients such as antimicrobial agents, anti-inflammatory agents, anesthetics, and the like.

The pharmaceutical composition may be administered in a number of ways depending on whether local or systemic treatment is desired, and on the area to be treated. Administration may be topically (including ophthalmically, vaginally, rectally, intranasally), orally, by inhalation, or parenterally, for example by intravenous drip, subcutaneous, intraperitoneal or intramuscular injection. In certain embodiments, administration is by topical administration. In certain embodiments, administration is by systemic administration. Systemic administration includes, for example, enteral or parenteral administration. In some embodiments, systemic administration is by intravenous injection, subcutaneous injection, intradermal injection, intramuscular injection, or inhalation. In some embodiments, systemic administration is by aerosolized delivery.

Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like.

Formulations for topical administration may include ointments, lotions, creams, gels (e.g., poloxamer gel), drops, suppositories, sprays, liquids and powders. Conventional pharmaceutical carriers, aqueous, powder or oily bases, thickeners and the like may be necessary or desirable. The disclosed compositions can be administered, for example, in a microfiber, polymer (e.g., collagen), nanosphere, aerosol, lotion, cream, fabric, plastic, tissue engineered scaffold, matrix material, tablet, implanted container, powder, oil, resin, wound dressing, bead, microbead, slow release bead, capsule, injectables, intravenous drips, pump device, silicone implants, or any bio-engineered materials. In some embodiments, the formulation for topical administration is a sunscreen, sunblock, or similar formulation. Such compositions may absorb, filter, reflect, or block UV radiation from the sun or other source of UV radiation. Thus, in some embodiments, the present disclosure provides formulations that function both to reduce UV radiation exposure and to prevent, treat or mitigate the progression of radiation injury from UV radiation. In other embodiments, the formulation for topical administration is a suntan lotion, tanning accelerator, tanning oil, or similar formulation. In some embodiments, the sunscreen, sunblock, suntan lotion, tanning accelerator, or tanning oil includes an amount of the polypeptides provided herein that can prevent, treat, or mitigate the progression of a radiation injury caused by UV light (e.g., from the sun, a tanning bed, or other source). For example, in some embodiments, the sunscreen includes a concentration of a polypeptide provided herein (e.g., from about 0.001% (w/w) to about 5.0% (w/w), or from about 10 µM to about 2000 µM.

In one aspect the provided pharmaceutically acceptable carrier is a poloxamer. Poloxamers, referred to by the trade name Pluronics^{®}, are nonionic surfactants that form clear thermoreversible gels in water. Poloxamers are polyethylene oxide-polypropylene oxide-polyethylene oxide (PEO-PPO-PEO) tri-block copolymers. The two polyethylene oxide chains are hydrophilic but the polypropylene chain is hydrophobic. These hydrophobic and hydrophilic characteristics take charge when placed in aqueous solutions. The PEO-PPO-PEO chains take the form of small strands where the hydrophobic centers would come together to form micelles. The micelle, sequentially, tend to have gelling characteristics because they come together in groups to form solids (gels) where water is just slightly present near the hydrophilic ends. When it is chilled, it becomes liquid, but it hardens when warmed. This characteristic makes it useful in pharmaceutical compounding because it can be drawn into a syringe for accurate dose measurement when it is cold. When it warms to body temperature (when applied to skin) it thickens to a perfect consistency (especially when combined with soy lecithin/isopropyl palmitate) to facilitate proper inunction and adhesion. Pluronic^{®} F127 (F127) is widely used because it is obtained easily and thus it is used in such pharmaceutical applications. F127 has a EO:PO:EO ratio of 100:65:100, which by weight has a PEO:PPO ratio of 2:1. Pluronic gel is an aqueous solution and typically contains 20-30% F-127. Thus, the provided compositions can be administered in F127.

Excipients for use in topical gels are well-known in the art and examples may be found in the Handbook of Pharmaceutical Excipients (Rowe, R. C. et al, APhA Publications; 5th ed., 2005). Exemplary excipients may include waxes, various sugars and types of starch, polymers, gels, emollients, thickening agents, rheology modifiers, humectants, glycerol, organic basic compounds, cellulose derivatives, gelatin, vegetable oils, polyethylene glycols and solvents. Examples of rheology modifiers include Carbopol, hydroxypropyl cellulose, C₂₆₋₂₈ alkyl dimethicone, C₂₆₋₂₈ alkyl methicone, polyphenylsisquioxane, trimethylsiloxysilicate, crosspolymers of cyclopentasiloxane and dimethicone/vinyltrimethylsiloxysilicate, fumed silica (e.g. Cab-O-Sil M5P), and mixtures thereof. Examples of emollients include glycerine, pentylene glycol, sodium pyrrolidone carboxylic acid, lanolin, saccharide isomerate, stearoxy dimethicone, stearyl dimethicone, and mixtures thereof. Emollients may be useful to prevent stratum corneum dehydration occurring due to the use of anhydrous solvents in the formulation. Examples of organic bases include 2-amino-2-methyl propanol, niacinamide, methanolamines, triethanolamines, Trisamino, AMP-95, AmP-Ultra PC 2000, triisopropanolamine, diisopropanolamine, Neutrol TE, Ethomeen, and mixtures thereof. The organic base may render the pH of the medicament basic or neutral.

Other exemplary excipients include water-soluble porogens. A water-soluble porogen is an additive that may facilitate water uptake and diffusion into the gel. Any suitable porogen may be used, but in some embodiments, the porogen may include sodium chloride, potassium chloride, sucrose, glucose, lactose, sorbitol, xylitol, polyethylene glycol, polyvinylpyrrollidone, polyvinyl alcohol or mixtures thereof.

Polymers may also act as excipients in topical gels. Exemplary polymers include hydrophilic polyurethanes, hydrophilic polyacrylates, co-polymers of carboxymethylcellulose and acrylic acid, N-vinylpyrrolidone, poly(hydroxy acids), polyanhydrides, polyorthoesters, polyamides, polycarbonates, polyalkylenes (e.g., polyethylene and polypropylene), polyalkylene glycols (e.g., poly(ethylene glycol)), polyalkylene oxides (e.g., polyethylene oxide), polyalkylene terephthalates (e.g., polyethylene terephthalate), polyvinyl alcohols, polyvinyl ethers, polylvinyl esters, polyvinyl halides (e.g., poly(vinyl chloride)), polyvinylpyrrolidone, polysiloxanes, poly(vinyl acetates), polystyrenes, polyurethane copolymers, cellulose, derivatized celluloses (e.g., hydroxyethyl cellulose, hydroxypropyl cellulose, hydroxypropyl methyl cellulose, methylcellulose, ethylcellulose, carboxymethyl cellulose, or cellulose acetate), alginates, poly(acrylic acid), poly(acrylic acid) derivatives, acrylic acid copolymers, methacrylic acid, methacrylic acid derivatives, methacrylic acid copolymers, poly(butyric acid), poly(valeric acid), poly(lactide-co-caprolactone), copolymers thereof and blends thereof.

In some embodiments of the invention, the polymers may be superabsorbent polymers (SAPs). A polymer is considered superabsorbent, as defined per IUPAC, as a polymer that can absorb and retain extremely large amounts of water relative to its own mass. SAPs may absorb water up to 500 times their own weight and may swell up to 1000-times their original volume. Particular SAPs of interest include sodium polyacrylate, the polyurethane Tecophilic TG-2000, and polymers prepared by the use of polyacrylamide copolymer, ethylene maleic anhydride copolymer, cross-linked carboxy-methyl-cellulose, polyvinyl alcohol copolymers, polyvinylpyrrolindone and cross-linked polyethylene oxide.

In some embodiments of the invention, polymers that are relatively hydrophobic may be used. Any suitable hydrophobic polymer may be used. However, exemplary polymers that are relatively hydrophobic include aromatic polyurethanes, silicone rubber, polysiloxanes, polycaprolactone, polycarbonate, polyvinylchloride, polyethylene, poly-L-lactide, poly-DL-glycolide, polyetheretherketone (PEEK), polyamide, polyimide and polyvinyl acetate. In addition, a hydrophobic gel-base and/or rheology modifier may be used.

In some embodiments of the invention, the polymers may act as thickening agents in the medicaments. Specifically, the polymeric portion of the gel may act as a viscoelastic substance and may retain the gel at the site of application, along with the alpha connexin polypeptides dispersed therein.

In some other embodiments, a gel that includes a polymer may have spreadability such that it forms a thin film when applied on the skin surface. This film may enable the application of the contained alpha connexin polypeptides over a wide area, and may serve to maintain the alpha connexin polypeptides on the affected area of the skin.

Other excipients may include various ionic or non-ionic compounds to maintain stability of the formulation, thereby protecting from the de-emulsification, settling, agglomeration or degradation of the formulation constituents that may reduce its therapeutic or aesthetic value.

Examples of ionic compounds may include salts such as sodium chloride, potassium chloride; cationic, anionic or zwitterionic surfactants such as sodium dodecyl sulfate (SDS), perfluorooctanoate (PFOA), perfluorooctanesulfonate (PFOS), ammonium lauryl sulfate (ALS), sodium lauryl ether sulfate (SLES), alkyl benzene sulfonate, cetyl trimethylammonium bromide (CTAB), cetylpyridinium chloride (CPC), polyethoxylated tallow amine (POEA), benzalkonium chloride (BAC), benzethonium chloride, dodecyl betaine, cocamidopropyl betaine and cocoampho glycinate.

Examples of non-ionic compounds that may act as excipients include non-ionic surfactants such as Pluronic, Tween, AMP, and Brij family of surfactants; and surfactants derived from biological sources, e.g., natural or semi-synthetic surfactants, such as oleic acid, sorbitan trioleate, sorbitan monooleate, lecithin, cocamide MEA, cocamide DEA and cocamidopropyl betaine. Surfactants (both ionic and non-ionic) may reduce the interfacial surface energy and may facilitate spreading of the topical formulation over a wider area.

In some embodiments of the invention, solvent excipients may be used as a carrier vehicle for the alpha connexin polypeptides and other excipients. The polymer chains may interact with the solvent and undergo swelling to form a network that may impart viscoelastic properties to the topical formulation. In some embodiments of the topical formulation, the solvent may evaporate upon application, leaving a residual film of the polymer along with the entrapped alpha connexin polypeptides.

Exemplary solvent excipients that may be useful in hydrophilic formulations may include dimethyl isosorbide, propylene glycol, glycerol, isopropanol, ethanol, benzyl alcohol, ethylene glycol, polyethylene glycol, ethoxydiglycol or mixtures thereof. Exemplary solvent excipients that may be useful in hydrophobic formulations may include capric/caprylic triglycerides, isopropyl myristate, mineral oil, isododecane, isodecyl neopentanoate, butylene glycol, pentylene glycol, hexylene glycol, methoxypolyethyleneglycol, cyclopentasiloxane, cyclotetrasiloxane, dimethicone, caprylyl methicone or mixtures thereof.

In addition to the alpha connexin polypeptides and excipients, the topical formulation may also include at least one additional therapeutic agent such as antimicrobial agents, anti-acne agents, anti-inflammatory agents, analgesic agents, anesthetic agents, antihistamine agents, antiseptic agents, immunosuppressants, antihemorrhagic agents, vasodilators, wound healing agents, anti-biofilm agents and mixtures thereof.

Examples of antimicrobial agents include penicillins and related drugs, carbapenems, cephalosporins and related drugs, erythromycin, aminoglycosides, bacitracin, gramicidin, mupirocin, chloramphenicol, thiamphenicol, fusidate sodium, lincomycin, clindamycin, macrolides, novobiocin, polymyxins, rifamycins, spectinomysin, tetracyclines, vanomycin, teicoplanin, streptogramins, anti-folate agents including sulfonamides, trimethoprim and its combinations and pyrimethamine, synthetic antibacterials including nitrofurans, methenamine mandelate and methenamine hippurate, nitroimidazoles, quinolones, fluoroquinolones, isoniazid, ethambutol, pyrazinamide, para-aminosalicylic acid (PAS), cycloserine, capreomycin, ethionamide, prothionamide, thiacetazone, viomycin, eveminomycin, glycopeptide, glyclyclycline, ketolides, oxazolidinone; imipenen, amikacin, netilmicin, fosfomycin, gentamycin, ceftriaxone, Ziracin, Linezolid, Synercid, Aztreonam, and Metronidazole, Epiroprim, Sanfetrinem sodium, Biapenem, Dynemicin, Cefluprenam, Cefoselis, Sanfetrinem celexetil, Cefpirome, Mersacidin, Rifalazil, Kosan, Lenapenem, Veneprim, Sulopenem, ritipenam acoxyl, Cyclothialidine, micacocidin A, carumonam, Cefozopran and Cefetamet pivoxil.

Examples of topical anti-acne agents include adapalene, azelaic acid, benzoyl peroxide, clindamycin and clindamycin phosphate, doxycycline, erythromycin, keratolytics such as salicylic acid and retinoic acid ("Retin-A"), norgestimate, organic peroxides, retinoids such as isotretinoin and tretinoin, sulfacetamide sodium, and tazarotene. Particular anti-acne agents include adapalene, azelaic acid, benzoyl peroxide, clindamycin (e.g., clindamycin phosphate), doxycycline (e.g., doxycycline monohydrate), erythromycin, isotretinoin, norgestimate, sulfacetamide sodium, tazarotene, etretinate and acetretin.

Examples of antihistamine agents include diphenhydramine hydrochloride, diphenhydramine salicylate, diphenhydramine, chlorpheniramine hydrochloride, chlorpheniramine maleate isothipendyl hydrochloride, tripelennamine hydrochloride, promethazine hydrochloride, methdilazine hydrochloride, and the like. Examples of local anesthetic agents include dibucaine hydrochloride, dibucaine, lidocaine hydrochloride, lidocaine, benzocaine, p-buthylaminobenzoic acid 2-(die-ethylamino) ethyl ester hydrochloride, procaine hydrochloride, tetracaine, tetracaine hydrochloride, chloroprocaine hydrochloride, oxyprocaine hydrochloride, mepivacaine, cocaine hydrochloride, piperocaine hydrochloride, dyclonine and dyclonine hydrochloride.

Examples of antiseptic agents include alcohols, quaternary ammonium compounds, boric acid, chlorhexidine and chlorhexidine derivatives, iodine, phenols, terpenes, bactericides, disinfectants including thimerosal, phenol, thymol, benzalkonium chloride, benzethonium chloride, chlorhexidine, povidone iode, cetylpyridinium chloride, eugenol and trimethylammonium bromide.

Examples of anti-inflammatory agents include nonsteroidal antiinflammatory agents (NSAIDs); propionic acid derivatives such as ibuprofen and naproxen; acetic acid derivatives such as indomethacin; enolic acid derivatives such as meloxicam, acetaminophen; methyl salicylate; monoglycol salicylate; aspirin; mefenamic acid; flufenamic acid; indomethacin; diclofenac; alclofenac; diclofenac sodium; ibuprofen; ketoprofen; naproxen; pranoprofen; fenoprofen; sulindac; fenclofenac; clidanac; flurbiprofen; fentiazac; bufexamac; piroxicam; phenylbutazone; oxyphenbutazone; clofezone; pentazocine; mepirizole; tiaramide hydrochloride; steroids such as clobetasol propionate, bethamethasone dipropionate, halbetasol proprionate, diflorasone diacetate, fluocinonide, halcinonide, amcinonide, desoximetasone, triamcinolone acetonide, mometasone furoate, fluticasone proprionate, betamethasone diproprionate, triamcinolone acetonide, fluticasone propionate, desonide, fluocinolone acetonide, hydrocortisone vlaerate, prednicarbate, triamcinolone acetonide, fluocinolone acetonide, hydrocortisone and others known in the art, predonisolone, dexamethasone, fluocinolone acetonide, hydrocortisone acetate, predonisolone acetate, methylpredonisolone, dexamethasone acetate, betamethasone, betamethasone valerate, flumetasone, fluorometholone, beclomethasone diproprionate, fluocinonide, topical corticosteroids, and may be one of the lower potency corticosteroids such as hydrocortisone, hydrocortisone-21-monoesters (e.g., hydrocortisone-21-acetate, hydrocortisone-21-butyrate, hydrocortisone-21-propionate, hydrocortisone-21-valerate, etc.), hydrocortisone-17,21-diesters (e.g., hydrocortisone-1 7,21-diacetate, hydrocortisone-17-acetate-21-butyrate, hydrocortisone-17,21-dibutyrate, etc.), alclometasone, dexamethasone, flumethasone, prednisolone, or methylprednisolone, or may be a higher potency corticosteroid such as clobetasol propionate, betamethasone benzoate, betamethasone dipropionate, diflorasone diacetate, fluocinonide, mometasone furoate, triamcinolone acetonide.

Examples of analgesic agents include alfentanil, benzocaine, buprenorphine, butorphanol, butamben, capsaicin, clonidine, codeine, dibucaine, enkephalin, fentanyl, hydrocodone, hydromorphone, indomethacin, lidocaine, levorphanol, meperidine, methadone, morphine, nicomorphine, opium, oxybuprocaine, oxycodone, oxymorphone, pentazocine, pramoxine, proparacaine, propoxyphene, proxymetacaine, sufentanil, tetracaine and tramadol.

Examples of anesthetic agents include alcohols such as phenol; benzyl benzoate; calamine; chloroxylenol; dyclonine; ketamine; menthol; pramoxine; resorcinol; troclosan; procaine drugs such as benzocaine, bupivacaine, chloroprocaine; cinchocaine; cocaine; dexivacaine; diamocaine; dibucaine; etidocaine; hexylcaine; levobupivacaine; lidocaine; mepivacaine; oxethazaine; prilocalne; procaine; proparacaine; propoxycaine; pyrrocaine; risocaine; rodocaine; ropivacaine; tetracaine; and derivatives, such as pharmaceutically acceptable salts and esters including bupivacaine HCl, chloroprocaine HCl, diamocaine cyclamate, dibucaine HCl, dyclonine HCl, etidocaine HCl, levobupivacaine HCl, lidocaine HCl, mepivacaine HCl, pramoxine HCl, prilocalne HCl, procaine HCl, proparacaine HCl, propoxycaine HCl, ropivacaine HCl, and tetracaine HCl.

Examples of antihemorrhagic agents include thrombin, phytonadione, protamine sulfate, aminocaproic acid, tranexamic acid, carbazochrome, carbaxochrome sodium sulfanate, rutin and hesperidin.

Beside the bioactive polypeptide component, the instant invention may also contain other active agents such as niacinamide, phytantriol, farnesol, bisabolol and salicylic acid. It is expected that certain additional active agents will act synergistically with the bioactive peptide component, or will enhance the shelf-life of the formulation.

Examples of wound treatments that may be used together with the drug product of the present invention include fibrinolytic enzymes, such as fibrinolysin, deoxyribonuclease, streptokinase, and streptodornase, necrotomy tissue agents containing lysozyme chloride, antimicrobial agents containing gentamicin sulfate, sulfadiazine silver, bacitracin, and fradiomycin sulfate, incarnant agents containing trafermin, bucladesine sodium, tretinoin tocoferil (tocoretinate), alprostadil alfadex, solcoseryl (extract from hemolysed blood of young cattle), and alcloxa, iodine preparations containing white soft sugar, povidone iodine, and iodine, and preparations containing bendazac, dimethyl isopropylazulene (guaiazulene), and epinephrine as active ingredients.

In addition to the alpha connexin polypeptides, excipients, and other therapeutic agents, the gels may also include other compounds that improve the organoleptic properties of the topical formulation.

Examples of such compounds include perfumes, dyes and colorants; chelating agents including but not limited to edetate disodium (EDTA), EGTA, CP94, citric acid; preservatives including but not limited to quaternary ammonium compounds, such as benzalkonium chloride, benzethonium chloride, cetrimide, dequalinium chloride, and cetylpyridinium chloride; mercurial agents, such as phenylmercuric nitrate, phenylmercuric acetate, and thimerosal; alcoholic agents, for example, chlorobutanol, phenylethyl alcohol, and benzyl alcohol; antibacterial esters, for example, esters of parahydroxybenzoic acid; and other anti-microbial agents such as chlorhexidine, chlorocresol, benzoic acid and polymyxin.

In certain embodiments are provided a topical formulation comprising at least one alpha connexin polypeptide and hydroxyethylcellulose gel, wherein the hydroxyethylcellulose gel stabilizes the alpha connexin polypeptide. In certain embodiments, the hydroxyethylcellulose gel stabilizes the alpha connexin polypeptide so that after 3 months of storage at 5 °C, at least 80%, 81%, 82%, 83%, 84%, 85%, 86%, 87%, 88%, 89%, 90%, 91%, 92%, 93%, 94%, 95%, 96%, 97%, 98%, 99%, or 100% of the alpha connexin polypeptide is detectable by analytical methods. In some embodiments, the alpha connexin polypeptide is present in the formulation at a concentration of about 0.0025% (w/w), of about 0.005% (w/w), of about 0.0075% (w/w), of about 0.010% (w/w), of about 0.015% (w/w), of about 0.020% (w/w), of about 0.025% (w/w), of about 0.030% (w/w), of about 0.035% (w/w), of about 0.040% (w/w), of about 0.045% (w/w), of about 0.050% (w/w), of about 0.055% (w/w), of about 0.060% (w/w), of about 0.065% (w/w), of about 0.070% (w/w), of about 0.075% (w/w), of about 0.080% (w/w), of about 0.085% (w/w), of about 0.090% (w/w), of about 0.095% (w/w), of about 0.100% (w/w), of about 0.150% (w/w), of about 0.200% (w/w), of about 0.250% (w/w), of about 0.500% (w/w), of about 0.750% (w/w), of about 1.00% (w/w), of about 1.50% (w/w), of about 2.00% (w/w), of about 2.50% (w/w), or of about 3.00% (w/w), or of about 3.50% (w/w), or of about 4.00% (w/w), or of about 4.50% (w/w), or of about 5.00% (w/w), or more. In one embodiment, the alpha connexin polypeptide is present in the formulation at a concentration of between about 0.005% (w/w) and about 1.00% (w/w).

In other embodiments, the drug product of the invention is a clear colorless gel which contains 0.0072% (w/w) (20 µM) of the ACT peptide, 0.018% (w/w) (50 µM) of the ACT peptide, 0.036% (w/w) (100 µM) of the ACT peptide, 0.072% (w/w) (200 µM) of the ACT peptide, or 0.36% (w/w) (1000 µM) of the ACT peptide. The ACT peptide may be dissolved in a semisolid dosage form that contains >0% water, >10% water, >20% water, >30% water, >40% water, >50% water, >60% water, >70% water, >80% water, or >90% water and 0.25% gelling agent (polymer), 0.55% gelling agent (polymer), 0.75% gelling agent (polymer), 1.00% gelling agent (polymer), 1.25% gelling agent (polymer), 1.50% gelling agent (polymer), 1.75% gelling agent (polymer), 2.00% gelling agent (polymer), 2.25% gelling agent (polymer), or 2.50% gelling agent (polymer), or 3.00% gelling agent (polymer), or 3.50% gelling agent (polymer), or 4.00% gelling agent (polymer), or 4.50% gelling agent (polymer), or 5.00% gelling agent (polymer).

In certain embodiments, the ACT peptide may be well preserved and adequately buffered to about pH 5.5, about pH 6, about pH 6.5, about pH 7, about pH 7.5 or about pH8. In certain embodiments of the topical formulation, the qualitative and quantitative composition is that listed in Table 7.

**Table 7: Drug Product Gel Qualitative & Quantitative Composition**

| **Ingredients** | **Grade** | **Function** | **Concentration (% w/w)** |
|---|---|---|---|
| Peptide 328967 (ACT peptide) | -- | Active | 0.0072; 0.018 |
| | | | 0.036; 0.072 |
| Methylparaben | NF | Preservative | 0.17 |
| Propylparaben | NF | Preservative | 0.02 |
| Glycerin | USP | Solvent | 5.0 |
| Sodium Phosphate Monobasic | USP | Buffer Agent | 0.263 |
| Sodium Phosphate Dibasic | USP | Buffer Agent | 0.044 |
| Propylene Glycol | USP | Solvent | 3.0 |
| Edetate Disodium | USP | Chelating Agent | 0.05 |
| D-Mannitol | USP | Stabilizer | 0.05 |
| Hydroxyethylcellulose, 250HHX | NF | Gelling Agent | 1.25% |
| Purified Water, qsad | USP | Solvent | 100% |

The ACT1 peptide sequence is listed in Table 8 below in which Ahx refers to L-2-aminohexanoic acid, also known as 6-aminohexanoic acid:

**Table 8: Peptide 328967 (ACT1) sequence (SEQ ID NO:91)**

| |
|---|
| Biotin-Ahx-Arg-Gln-Pro-Lys-Ile-Trp-Phe-Pro-Asn-Arg-Arg-Lys-Pro-Trp-Lys-Lys-Arg-Pro-Arg-Pro-Asp-Asp-Leu-Glu-Ile-OH |

The general properties of the Peptide 328967 are listed in Table 9 below.

**Table 9: General Physical Properties of Peptide 328967**

| Physical Appearance | White to off-white powder |
|---|---|
| Molecular Weight | 3597.33 ± 2.0 amu |
| Counter-Ion | AcOH |
| Solubility | Soluble in water at room temperature |

In certain aspects, the excipients used in the drug product topical preparation are selected from the group consisting of or are one or more of the following:
Methylparaben
Propylparaben
Glycerin
Sodium Phosphate Monobasic
Sodium Phosphate Dibasic
Propylene Glycol
Edetate Disodium (EDTA)
D-Mannitol
Hydroxyethylcellulose, 250 HHX
Purified Water

In some embodiments, the drug product topical preparation comprises a peptide, D-mannitol, hydroxyethylcellulose, and purified water. Said preparation may further comprise one or more of methylparaben, propylparaben, glycerin, sodium phosphate monobasic, sodium phosphate dibasic, propylene glycol, and edetate disodium (EDTA). In further embodiments, the hydroxyethylcellulose is 250 HHX. In other embodiments, the peptide is an alpha connexin polypeptide.

In some embodiments, the drug product topical preparation comprises a peptide at a concentration between about 0.001% (w/w) and about 0.5% (w/w) (for example, at about 0.0072%, 0.018%, 0.036%, or 0.072% (w/w)); methylparaben at a concentration between about 0.10% (w/w) and about 0.25% (w/w) (for example, about 0.17% (w/w)); propylparaben at a concentration between about 0.01% (w/w) and about 0.03% (w/w) (for example, about 0.02% (w/w)); glycerin at a concentration between about 1% (w/w) and about 10% w/w) (for example, about 5% (w/w)); sodium phosphate monobasic at a concentration between about 0.1% (w/w) and about 0.5% (w/w) (for example, about 0.263% (w/w)); sodium phosphate dibasic at a concentration between about 0.02% and about 0.06% (for example, about 0.044% (w/w)); propylene glycol at a concentration between about 1% (w/w) and about 5% (w/w) (for example, about 3% (w/w)); EDTA at a concentration between about 0.01% and about 0.1% (for example, about 0.05% (w/w)); D-mannitol at a concentration between about 0.01% (w/w) and about 0.1% (w/w) (for example, about 0.05% (w/w)); hydroxyethylcellulose at a concentration between about 0.5% and about 2.5% (for example, about 1.25% (w/w)), and purified water at a concentration of about 0.1% to about 10% (for example, about 1%). In further embodiments, the peptide is an alpha connexin polypeptide.

Derived through *in vitro* and *in vivo* studies, these stabilizers and excipients are incorporated into the drug product since they are non-irritating, non-staining, and non-immunogenic. Stability studies showed that the ACT1 peptide is more stable in the gelling agent Hydroxyethylcellulose, 250 HHX (1.25%) compared to Pluronic gels. The ACT peptide within the drug product containing 1.25% hydroxyethylcellulose only dropped to 98% of the label claim (i.e., initial concentration) when stored at 5 °C. for three months and to 84% of the label claim when stored at 25 °C for the same duration. In one aspect of the description, edetate disodium (EDTA) and Mannitol are incorporated within the drug product to provide stability to the ACT1 peptide. In some embodiments of the invention, the Mannitol is present in the formulation at 0.01% (w/w) to 1.6% (w/w), 0.01% (w/w) to 1.5% (w/w), 0.01% (w/w) to 1.4% (w/w), 0.01% (w/w) to 1.3% (w/w), 0.01% (w/w) to 1.2% (w/w), 0.01% (w/w) to 1.1% (w/w), 0.01% (w/w) to 1.0% (w/w), 0.01% (w/w) to 0.9% (w/w), 0.01% (w/w) to 0.8% (w/w), 0.01% (w/w) to 0.7% (w/w), 0.01% (w/w) to 0.6% (w/w), 0.01% (w/w) to 0.5% (w/w), 0.01% (w/w) to 0.4% (w/w), 0.01% (w/w) to 0.3% (w/w), 0.01% (w/w) to 0.2% (w/w), 0.01% (w/w) to 0.1% (w/w), or 0.01% (w/w) to 0.0.05% (w/w). In specific embodiments, the Mannitol is present in the formulation at about 0.05% (w/w).

In other aspects, a buffering agent is included in the topical formulation to maintain the pH in a certain range. Suitable buffering agents can include, but are not limited to, acetate buffers, citrate buffers, phosphate buffers, lactic acid buffers, malic acid buffers, succinic acid buffers, borate buffers, sodium hydroxide, potassium hydroxide, and ammonium hydroxide. Phosphate salts such as monosodium phosphate (NaH₂PO₄; also known as monobasic sodium phosphate), disodium hydrogen phosphate (Na₂HPO₄; also known as dibasic sodium phosphate), monopotassium phosphate (KH2PO₄), dipotassium phosphate (K₂HPO₄), and mixtures thereof can also be used. In certain embodiments, phosphate buffer provides superior stability compared to citrate buffer. In certain aspects, a buffer capacity at 25 mM was found to be adequate for the drug product. Buffer is needed to control pH of the gel system and to maintain stability of the peptide drug. In certain embodiments, the pH range of the topical formulation may be pH 2 to pH 12, pH 4 to pH 10, or pH 6 to pH 8. In some embodiments, an optimal pH range is about pH 5.0 to about pH 7.0. In other embodiments, the pH of the topical formulation of the present invention is 4.0, 4.5, 5.0, 5.5, 6.0, 6.5, 7.0, 7.5, or 8.0. In other aspects, propylene glycol in the amount of 3% provides better solubilization of parabens in the aqueous system.

In some embodiments, the formulation with an ACT peptide incorporated into hydroxyethylcellulose enables large scale manufacturing of a product with the characteristics that make it practical for clinical treatments as well as meeting desired storage and stability requirements. While a formulation for ACT1 using Pluronic gel may require a relatively long incorporation time of approximately 2.5 hours and only yield 50 gram batches, a formulation with hydroxyethylcellulose provides allows for significantly faster incorporation of the ACT peptide into the gel and yields much larger batches. For example, when using Pluronic F 127 gel in the topical formulation it takes over an hour to incorporate the polymer, and the formulation needs to be placed in a water bath to help with incorporation. In contrast, hydroxyethylcellulose (e.g., HEC 250 HHX) is easily incorporated at room temperature and hydrates within 30 minutes. Thus, the use of hydroxyethylcellulose may facilitate large scale manufacturing. The manufacturing process with Pluronic gel may require a cold bath to bring its viscosity into a desired range and require more energy than the manufacturing process with hydroxyethylcellulose. In addition, the final formulation in Pluronic gel may be very thin at a storage condition of 5 °C.

It has been discovered that hydroxyethylcellulose (HEC) is a suitable gelling agent and acceptable carrier of the drug product of the present invention. In some embodiments, the gelling agent is Hydroxyethylcellulose (HEC), 250 HHX. In certain embodiments, the percent (w/w) of HEC is in the range of 1-5%. In other embodiments, the percent (w/w) of HEC is 1.25%. In the manufacture of HEC, a purified cellulose is reacted with sodium hydroxide to produce a swollen alkali cellulose. The alkali-treated cellulose is more chemically reactive than cellulose. By reacting the alkali cellulose with ethylene oxide, a series of hydroxyethylcellulose ethers is produced. In this reaction, the hydrogen atoms in the hydroxyl groups of cellulose are replaced by hydroxyethyl groups, which confer water solubility to the gel. It is contemplated in this description that a single HEC ether may be used, or a mixture of HEC ethers of difference molecular weight and structure may be used. Suitable grades of HEC for pharmaceutical purposes are well known and full described in the pharmaceutical literature. Suitable commercially available brands of HEC include but are not limited to Fuji HEC-HP; Fuji HEC-AG 15; NATRO-SOL 250HR; NATROSOL 250 MH; NATROSOL 250G; CELLOSIZE QP 30000; TYLOSE H SERIES; NATROSOL 180L; NATROSOL 300H; TYLOSE P-X; NATROSOL 250M; CELLOSIZE WP 4400; CELLOSIZE UT 40; NATROSOL 250H4R; Tylose H 20P; NATROSOL LR; TYLOSE MHB; NATROSOL 250HHP; HERCULES N 100; CELLOSIZE WP 300; TYLOSE P-Z SERIES; NATROSOL 250H; TYLOSE PS-X; Cellobond HEC 400; CELLOSIZE QP; CELLOSIZE QP 1500; NATRO-SOL 250; HYDROXYETHYL CELLULOSE ETHER; HESPAN; TYLOSE MHB-Y; NATROSOL 240JR; HYDROXYETHYL STARCH; CELLOSIZE WP; CELLOSIZE WP 300H; 2-HYDROXYETHYL CELLULOSE ETHER; BL 15; CELLOSIZE QP 4400; CELLOSIZE QP3; TYLOSE MB; CELLULOSE HYDROXY-ETHYLATE; CELLOSIZE WPO 9H17; CELLOSIZE 4400H16; CELLULOSE HYDROXYETHYL ETHER; Hydroxyethyl Cellulose; Hydroxyl Ethyl Cellulose (HEC); Hydroxyethyl Cellulose 100H (celocell 100h); TYLOSE MH-XP; NATROSOL 250HX; Natrosol; Daicel EP 500; HEC-Unicel; HEC (Hydroxyethyl cellulose); Cellosize; HEC-Al 5000; Fuji HEC-AL 15; HEC-Unicel QP 09L; Cellulose, ethers, 2-hydroxyethyl ether; Unicel QP 52000H; HEC-QP 4400; SP 250 (cellulose); Hetastarch; Cellulose, ethers, 2-hydroxyethyl ether; Glutofix 600; FL 52; Fuji HEC-AX 15F; Tylose H 300P; HEC-Unicel QP 300H; Tylose H 300; Daicel SP 550; Daicel SE 600; Unicel QP 15000; HEC-QP 100 MH; HEC-QP 9H; OETs; Daicel EP 850; H. E. Cellulose; Cellobond 25T; Unicel QP 100 MH; Tylose H 4000; SE 850K; Tylomer H 20; Daicel SE 850K; Tylose H 30000YP; Unicel QP 4400; SP 407; Tylose H 100000; Daicel SP 200; Culminal HEC 5000PR; Tylopur H 300; Daicel SP 750; Sanhec; BL 15 (cellulose derivative); Unicel QP 300H; Tylomer H 200; J 164; Tylose H 10; Tylose H 20; AH 15; Daicel SP 600; Daicel SE 900; HEC-Unicel QP 4400H; AX 15; Daicel SP 800; Fuji HEC-AW 15F; HEC-SE 850; HEC-A 5-25CF; Metolose 90SEW; AW 15 (polysaccharide); Cellobond HEC 5000; HEC-QP 100M; Cellobond HEC 15A; Tylose H 15000YP2; Walocel HT 6.000 PFV; 2-Hydroxyethyl cellulose (Natrosol Type 250HRCS); Fuji HEC-BL 20; Fuji HEC-SY 25F; Telhec; HEC-SP 200; HEC-AH 15; HEC-Unicel QP 30000H; see; HEC 10A; Daicel SP 400; Admiral 3089FS; Fuji HEC-A 5000F; HEC-SP 400; Hydroxyethyl Methyl Cellulose (HEMC); HYDROXYETHYL CELLULOSE (HEC); Hydroxyethyl Starch (CAS No:9004-62-0); Hydroxy Ethyl Cellulose; "Natrosol" [Aqualon]; HEC; 2-HYDROXYETHYL CELLULOSE; NATROSOL 150L; TYLOSE MHB-YP; HYDROXYETHYL ETHER CELLULOSE; NATROSOL 250L; CELLOSIZE WP 400H; TYLOSE P; CELLULOSE, 2-HYDROXYETHYL ETHER; TYLOSE MH-K; NATROSOL 250HHR.

In other embodiments, the drug product of the present invention is packaged in 20 mL vial (USP Type I, Borosilicate clear scintillation glass with poly-seal cone urea screw cap). A mixture of methylparaben at 0.17% (w/w) and propylparaben 0.02% (w/w) is used as a preservative.

In some embodiments, the present description includes a method of reating or preventing radiation injury in a subject at risk of such injury, comprising administering to the subject a topical formulation comprising at least one alpha connexin polypeptide and hydroxyethylcellulose gel, wherein the hydroxyethylcellulose gel stabilizes the alpha connexin polypeptide. In certain embodiments, the drug product of the present invention may be used to mitigate excessive scar formation associated with radiation injury. In these embodiments, the drug product of the present invention may be applied at the time of exposure to radiation, 1 hour after exposure to radiation, 2 hours after exposure to radiation, 3 hours after exposure to radiation, 4 hours after exposure to radiation, 5 hours after exposure to radiation, 6 hours after exposure to radiation, 7 hours after exposure to radiation, 8 hours after exposure to radiation, 9 hours after exposure to radiation, 10 hours after exposure to radiation, 11 hours after exposure to radiation, 12 hours after exposure to radiation, 13 hours after exposure to radiation, 14 hours after exposure to radiation, 15 hours after exposure to radiation, 16 hours after exposure to radiation, 17 hours after exposure to radiation, 18 hours after exposure to radiation, 19 hours after exposure to radiation, 20 hours after exposure to radiation, 21 hours after exposure to radiation, 22 hours after exposure to radiation 23 hours after exposure to radiation, 24 hours after exposure to radiation, 48 hours after exposure to radiation, 72 hours after exposure to radiation, or thereafter.

In other aspects, the drug product is manufactured with the following steps:
Step 1: In a suitable size of beaker, add propylene glycol, glycerin, methylparaben and propylparaben. Mix with a propeller until the parabens are completely dissolved.
Step 2: In a manufacturing vessel, add purified water (part 1), EDTA, monobasic sodium phosphate, dibasic sodium phosphate and D-mannitol. Mix with a propeller until a clear solution is obtained.
Step 3: Add the solution from step 1 to the manufacturing vessel. Rinse the beaker with purified water (part II, divided into approximately 3 equal portions) and add the rinse back to the vessel. Continue with propeller mixing until the solution is visually homogeneous.
Step 4: With homogenization mixing, add hydroxyethyl cellulose into the manufacturing vessel from Step 3. Mix until the polymer is fully dispersed.
Step 5: In a separate beaker, add purified water (part III) and an alpha connexin polypeptide (e.g., Peptide 328967, ACT1 peptide). Mix with a stir bar or propeller mixer until the peptide is completely dissolved and a gel is formed.
Step 6: With continuous propeller mixing, add the drug solution from step 5 to the manufacturing vessel. Rinse the beaker with purified water (part IV, divided into approximately 3 equal portions) and add the rinse back to the vessel. Mix until the gel is homogeneous.

The manufacturing process flow chart is provided in Figure 3.

Formulations of alpha connexin polypeptides that may be used in the present description are described in detail in U.S. Pat. No. 8,846,605, The entire contents of U.S. Pat. Nos. 7,786,074. 7,888,319; 8,357,668; 8,809,257; 8,859,733; 8,916,515; and 394,351; 9,408,381; 9,844,214; and 9, 9,855,313 are also refered to

Compositions for oral administration include powders or granules, suspensions or solutions in water or non-aqueous media, capsules, sachets, or tablets. Thickeners, flavorings, diluents, emulsifiers, dispersing aids or binders may be desirable.

Some of the compositions may potentially be administered as a pharmaceutically acceptable acid- or base-addition salt, formed by reaction with inorganic acids such as hydrochloric acid, hydrobromic acid, perchloric acid, nitric acid, thiocyanic acid, sulfuric acid, and phosphoric acid, and organic acids such as formic acid, acetic acid, propionic acid, glycolic acid, lactic acid, pyruvic acid, oxalic acid, malonic acid, succinic acid, maleic acid, and fumaric acid, or by reaction with an inorganic base such as sodium hydroxide, ammonium hydroxide, potassium hydroxide, and organic bases such as mono-, di-, trialkyl and aryl amines and substituted ethanolamines.

Effective dosages and schedules for administering the compositions may be determined empirically, and making such determinations is within the skill in the art. The dosage ranges for the administration of the compositions are those large enough to produce the desired effect in which the symptoms disorder are effected. The dosage should not be so large as to cause adverse side effects, such as unwanted cross-reactions, anaphylactic reactions, and the like. Generally, the dosage will vary with the age, condition, sex and extent of the disease in the patient, route of administration, or whether other drugs are included in the regimen, and can be determined by one of skill in the art. The dosage can be adjusted by the individual doctor in the event of any counterindications. Dosage can vary, and can be administered in one or more dose administrations daily, for one or several days. Guidance can be found in the literature for appropriate dosages for given classes of pharmaceutical products. The range of dosage largely depends on the application of the compositions herein, severity of condition, and its route of administration.

For example, in applications as a laboratory tool for research, the ACT peptide compositions can be used in doses as low as 0.01% w/v. The dosage can be as low as 0.0002% w/v and possibly as high as 20% w/v in topical skin wound treatments. Significantly higher concentrations of the compositions by themselves or in combination with other compounds may be used in applications like cancer/tumor therapy or as an early concentrated bolus immediately following an acute tissue injury, such as CRI. Recommended upper limits of dosage for parenteral routes of administration for example intramuscular, intracerebral, intracardicardiac and intraspinal could be up to 1% w/v or v/v depending on the severity of the injury. This upper dosage limit may vary by formulation, depending for example on how the polypeptide(s) is combined with other agents promoting its action or acting in concert with the polypeptide(s).

For continuous delivery of the provided polypeptides, for example, in combination with an intravenous drip, upper limits of 0.01 g/Kg body weight over time courses determined by the doctor based on improvement in the condition can be used. In another example, upper limits of concentration of the provided nucleic acids delivered topically, for example, in skin wounds would be 5-10 µg/cm² of wound depending for example on how the nucleic acid is combined with other agents promoting its action or acting in concert with the nucleic acids. This would be repeated at a frequency determined by the Doctor based on improvement. In another example, upper limits of concentration of the provided nucleic acids delivered internally for example, intramuscular, intracerebral, intracardiac and intraspinal would be 50-100 µg/ml of solution. Again, the frequency would be determined by the Doctor based on improvement.

Also disclosed is the pre-conditioning of an area with the provided polypeptides prior to surgery. The concentration of the polypeptides can be 10-200 µM mixed in with 10-30% pluronic gel or any such carrier that enables penetration of the peptide(s) within the site of interest for a period of at least 3-6 hours prior to surgery. This pre-procedural conditioning can improve the subsequent healing response to surgery, including reduced inflammatory response.

Also disclosed is the treatment of a subject at risk of exposure to radiation with the provided polypeptides. In certain aspects, this treatment prevents subsequent radiation injury, such as cutaneous radiation injury. In certain embodiments, the provided polypeptides are administered at a concentration of about 10 µM to about 1000 µM. In some embodiments, the provided polypeptides are administered at a concentration of about 1µM , about 5µM , about 10µM, about 15 µM , about 20µM , about 25µM , about 30µM , about 35µM , about 40µM , about 45µM , about 50µM , about 55µM , about 60µM , about 65µM , about 70µM , about 75µM , about 80µM , about 85µM , about 90µM , about 95µM, about 100µM , about 110µM , about 120µM , about 130µM , about 140µM , about 150µM , about 160µM , about 170µM , about 180µM , about 190µM , about 200µM , about 225µM , about 250µM , about 275µM , or about 300 µM, or about 400µM, or about 500µM, or about 600µM, or about 700µM, or about 800µM, or about 900µM or about 1000µM, or about 1200µM, or about 1500µM, or about 2000µM. In some embodiments, the provided polypeptides are administered at a concentration of at least about 100 µm. In other embodiments, the provided polypeptides are administered at a concentration of at least about 200 µm. In still other embodiments, the provided polypeptides are administered at a concentration of at least about 1000 µm.

In some embodiments, the composition is administered systemically to the subject. For example, in some embodiments, the composition is administered by inhalation. In other embodiments, the composition is administered parenterally to the subject. For example, in some embodiments, the composition is administered to the subject by intravenous injection, subcutaneous injection, intraperitoneal injection, or intramuscular injection. In some embodiments, the composition is administered parenterally to the subject at a dose of from about 0.01 mg/kg to about 100 mg/kg, or about 0.1 mg/kg to about 100 mg/kg, or about 0.5 mg/kg to about 50 mg/kg, or about 1 mg/kg to about 50 mg/kg, or about 2 mg/kg to about 25 mg/kg, or about 5 mg/kg to about 25 mg/kg. In some embodiments, the composition is administered to the subject at a dose of about 1 mg/kg, about 2 mg/kg, about 3 mg/kg, about 4 mg/kg, about 5 mg/kg, about 6 mg/kg, about 7 mg/kg, about 8 mg/kg, about 9 mg/kg, about 10 mg/kg, about 15 mg/kg, about 20 mg/kg, about 25 mg/kg, about 30 mg/kg, about 35 mg/kg, about 40 mg/kg, about 45 mg/kg, about 50 mg/kg, about 60 mg/kg, about 70 mg/kg, about 80 mg/kg, about 90 mg/kg, or about 100 mg/kg.

In certain embodiments, the provided polypeptides are administered to the subject in a daily dosing regimen. In other aspects, the provided polypeptides are administered to the subject in a weekly dosing regimen. In other aspects, the provided polypeptides are administered to the subject in a monthly dosing regimen.

In certain embodiments, the provided polypeptides are administered to the subject at least about 2 years, at least about 1 year, at least about 6 months, at least about 60 days, at least about 30 days, at least about 20 days, at least about 10 days, at least about 7 days, at least about 3 days, at least about 1 day, at least about 23 hours, at least about 22 hours, at least about 21 hours, at least about 20 hours, at least about 19 hours, at least about 18 hours, at least about 17 hours, at least about 16 hours, at least about 15 hours, at least about 14 hours, at least about 13 hours, at least about 12 hours, at least about 11 hours, at least about 10 hours, at least about 9 hours, at least about 8 hours, at least about 7 hours, at least about 6 hours, at least about 5 hours, at least about 4 hours, at least about 3 hours, at least about 2 hours, or at least about 1 hour prior to exposure to radiation. In other embodiments, the provided polypeptides are administered to the subject at least about 1 hour, at least about 2 hours, at least about 3 hours, at least about 4 hours, at least about 5 hours, at least about 6 hours, at least about 7 hours, at least about 8 hours, at least about 9 hours, at least about 10 hours, at least about 11 hours, at least about 12 hours, at least about 13 hours, at least about 14 hours, at least about 15 hours, at least about 16 hours, at least about 17 hours, at least about 18 hours, at least about 19 hours, at least about 20 hours, at least about 21 hours, at least about 22 hours, at least about 23 hours, at least about 1 day, at least about 3 days, at least about 7 days, at least about 10 days, at least about 20 days, at least about 30 days, at least about 60 days, at least about 6 months, least about 1 year, or least about 2 years after exposure to radiation. In certain aspects, the provided polypeptides are administered from at least about 1 day to at least about 30 days after exposure to radiation. In specific embodiments, the provided polypeptides are administered at least about 1 day after exposure to radiation.

In some embodiments, the tissue exposed to radiation includes, without limitation, skin, heart, bone, brain, spinal cord, cornea, retina, and peripheral nerve. In certain aspects, the tissue exposed to radiation is the skin. In certain aspects, the prevention of radiation injury by the provided polypeptides to a subject at risk of exposure to such injury is assessed by measuring the average skin score. In certain embodiments, the average skin score is reduced by at least about 10%, at least about 20%, at least about 30%, at least about 40%, at least about 50%, at least about 60%, at least about 70%, at least about 80%, or at least about 90% in the presence of the provided polypeptides as compared to a control. In specific embodiments, the average skin score is reduced by at least about 30% in the presence of the provided polypeptides as compared to a control.

As used herein, the term "skin score" refers to any means for assessing the extent of damage to the skin and underlying tissues. For example, in some embodiments, the skin score is obtained using a scale known as the Kumar scale, an example of which is provided herein at Table 11. Thus, the term "average skin score," as used herein, is the average of the scores obtained using any method for assessing and scoring injury to the skin and underlying tissues.

In certain embodiments, the radiation injury resulting from exposure to the radiation includes, without limitation, cutaneous radiation injury (CRT; i.e., injury to the skin and underlying tissues from acute exposure to radiation), acute radiation syndrome (ARS; serious illness that occurs following an acute high dose of penetrating whole body irradiation), radiation burns, radiation dermatitis, radiation injury to the nervous system or brain, radiation pneumonitis, and radiation-induced enteritis. In certain aspects, the radiation injury is cutaneous radiation injury. In other aspects, the radiation injury is radiation burns. In yet other aspects, the radiation injury is radiation dermatitis.

In some embodiments, ARS includes any one or more of hematopoietic syndrome or bone marrow syndrome; gastrointestinal syndrome; neurovascular syndrome; cardiovascular syndrome, or central nervous system syndrome. These terms are used interchangeably with other terms know in the art such as "hematopoietic targeting ARS," "bone marrow targeting ARS," "gastrointestinal (GI) targeting ARS," "cardiac targeting ARS," and the like. In some embodiments a subject exposed to ionizing radiation may have any one or any combination of these syndromes. In some embodiments, the syndromes are referred to as, for example,

In some embodiments, the compositions and methods provided herein are useful for treating a subject having combined radiation injury. In some embodiments, a subject having combined radiation injury has dermal trauma, radiation burns, CRI, ARS, radiation dermatitis, and/or any other injury or syndrome related to exposure to ionizing radiation. Thus, in some embodiments, the present disclosure provides methods and compositions for treatment of various complications of exposure to ionizing radiation, and may include topical as well as systemic application of the provided compositions.

In some embodiments, the radiation injury results from a dirty bomb attack. A dirty bomb is an explosive device that disperses harmful radiological substances. Exposure to high levels of radiation from a dirty bomb could result in symptoms of acute radiation syndrome or radiation burns. Exposure to radiation causes damage to the basal cell layer of the skin and results in inflammation, erythema, desquamation, intense reddening, blistering, and ulceration of the irradiated site.

In certain embodiments, radionuclide-contaminated wounds and burns promote systemic uptake of radionuclides that greatly complicate topical decontamination and triage. In other embodiments, radionuclide contamination also interferes with wound healing. Therefore, in certain aspects, the provided polypeptides prevent systemic uptake of radionuclides from a site of radiation injury. In other aspects, the provided polypeptides prevent or treat radiation injury by reducing hemorrhage, severity of thermal burn injury, and formation of scar tissue at the site of radiation injury. In yet other aspects, the provided polypeptides promote tissue regeneration at the site of radiation injury.

Also provided are materials comprising the herein provided compositions (e.g., polypeptides, nucleic acids, or vectors). For example, provided are materials used to treat wounds and/or radiation injury, wherein the materials are coated with an ACT polypeptide. Non-limiting examples of materials used to treat wounds include bandages, steri-strip, sutures, staples, or grafts (e.g., skin grafts).

For example, the material (e.g., bandage, steri-strip, suture, staple, graft) can be soaked in the provided polypeptide at a concentration ranging from 10-200 µM. The material can then be dried and sealed in a sterile container. The material can also be immersed in liquid 10-30% pluronic gel at 4 °C. containing polypeptide at 10-200 µM concentration. The material can then be brought to approximate room temperature so that the gel polymerizes, leaving a coat of polypeptide-impregnated gel surrounding the material, which can be sealed in a sterile container. The polypeptide can also be incorporated into a cross-linkable hydrogel system, such as the poly(lactic-co-glycolic acid) (PLGA) or polyurethane, which can then be fashioned into materials for treating wounds (e.g., bandage, steri-strip, suture, staple, graft). Thus, provided are composite hydrogel-peptide materials.

Also disclosed are medical implants coated with the provided polypeptide before implantation in a subject. For example, a common problem in such implant surgeries is the formation of a contraction capsule around the implant from scar tissue formation that leads to undue hardening, contraction and ultimately misshaping of the tissue of interest. The use of the present polypeptides in or on the implant can reduce or prevent this misshaping. Non-limiting examples of medical implants include: limb prostheses, breast implants, penile implants, testicular implants, artificial eyes, facial implants, artificial joints, heart valve prostheses, vascular prostheses, dental prostheses, facial prosthesis, tilted disc valve, caged ball valve, ear prosthesis, nose prosthesis, pacemakers, cochlear implants, and skin substitutes (e.g., porcine heterograft/pigskin, BIOBRANE, cultured keratinocytes).

Provided herein is a method of promoting wound healing following tissue injury in a subject, comprising administering to the subject one or more of the herein provided compositions (e.g., polypeptides, nucleic acids, or vectors) in a pharmaceutically acceptable carrier. In some embodiments, the wound is a result of a cutaneous radiation injury. Further provided is a method of treating a subject with tissue injury (e.g., CRI), comprising administering to the subject one or more of the herein provided compositions (e.g., polypeptides, nucleic acids, or vectors) in a pharmaceutically acceptable carrier.

"Promote," "promotion," and "promoting" refer to an increase in an activity, response, condition, disease, or other biological parameter. This can include but is not limited to the initiation of the activity, response, condition, or disease. This may also include, for example, a 10% increase in the activity, response, condition, or disease as compared to the native or control level. Thus, the increase can be a 10, 20, 30, 40, 50, 60, 70, 80, 90, 100%, or any amount of increase in between as compared to native or control levels.

By "treat" or "treatment" is meant a method of reducing the effects of a disease or condition. Treatment can also refer to a method of reducing the underlying cause of the disease or condition itself rather than just the symptoms. The treatment can be any reduction from native levels and can be but is not limited to the complete ablation of the disease, condition, or the symptoms of the disease or condition. For example, a disclosed method for promoting wound healing is considered to be a treatment if there is a 10% reduction in one or more symptoms of the disease in a subject with the disease when compared to native levels in the same subject or control subjects. Thus, the reduction can be a 10, 20, 30, 40, 50, 60, 70, 80, 90, 100%, or any amount of reduction in between as compared to native or control levels.

In some aspects, the present disclosure provides compositions and methods for preventing ARS, CRI, combined radiation injury, and/or radiation dermatitis; or mitigating the progression of injury as a result of ARS, CRI, combined radiation injury, and/or radiation dermatitis, in a particular patient population, wherein the patient population comprises subjects who are at risk of such an injury.

As used herein, "subject" includes, but is not limited to, animals, plants, bacteria, viruses, parasites and any other organism or entity that has nucleic acid. The subject may be a vertebrate, more specifically a mammal (e.g., a human, horse, pig, rabbit, dog, sheep, goat, non-human primate, cow, cat, guinea pig or rodent), a fish, a bird or a reptile or an amphibian. The subject can be an invertebrate, more specifically an arthropod (e.g., insects and crustaceans). The term does not denote a particular age or sex. Thus, adult and newborn subjects, as well as fetuses, whether male or female, are intended to be covered. In some embodiments, a patient refers to a subject afflicted with a disease or disorder. In some embodiments, a patient population refers to a particular, defined set of subjects having a disease or disorder or at risk of developing a particular disease or disorder. For example, in some embodiments, the present disclosure provides methods for treating, preventing, or mitigating the severity of radiation injury in a patient population comprising soldiers and/or civilians present in an area subject to war and/or terrorism. In some embodiments, the present disclosure provides methods for treating, preventing, or mitigating the severity of radiation injury in a patient population comprising cancer subjects receiving radiation therapy. The term "patient" includes human and veterinary subjects.

The provided method can reduce scar tissue formation in a subject following tissue injury. By "scar tissue" is meant the fibrous (fibrotic) connective tissue that forms at the site of injury or disease in any tissue of the body, caused by the overproduction of disorganized collagen and other connective tissue proteins, which acts to patch the break in the tissue. Scar tissue may replace injured skin and underlying muscle, damaged heart muscle, or diseased areas of internal organs such as the liver. Dense and thick, it is usually paler than the surrounding tissue because it is poorly supplied with blood, and although it structurally replaces destroyed tissue, it cannot perform the functions of the missing tissue. It is composed of collagenous fibers, which will often restrict normal elasticity in the tissue involved. Scar tissue may therefore limit the range of muscle movement or prevent proper circulation of fluids when affecting the lymphatic or circulatory system. Glial scar tissue following injury to the brain or spinal cord is one of the main obstacles to restoration of neural function following damage to the central nervous system. A reduction in scar tissue can be assessed by the population of cell types within the injured site. For example, a reduction in glial scar tissue can be estimated by an increased ratio of neuronal to astrocytic cells. A reduction in scar tissue formation can be measured by a simple measurement of scar width or area of scar tissue (Wilgus et al., 2003). In addition histological assessments can be made about the restoration of structural complexity within healed tissue in comparison to normal tissue.

In addition to reducing fibrotic tissue formation in a subject in following tissue injury, the provided compositions and methods can also be used to treat disorders associated with pathological increases in fibrotic tissue formation in a subject, such as for example, psoriasis, cutaneous and systemic mastocytosis, asthma, eczema, sinusitis, atherosclerosis, rheumatoid arthritis, inflammatory bowel disease, multiple sclerosis, pulmonary fibrosis and cystic fibrosis. A reduction in fibrotic tissue formation in a subject can be measured by clinical judgment of a doctor assessing whether a regain in normal structure and function of a given tissue and/or organ in a subject has resulted following a treatment. As an example, for psoriasis a doctor would assess the subject's skin to determine whether there has been a reduction in patches of raised red skin covered by flaky white buildup. Certain kinds of psoriasis, are characterized by a pimple-ish (pustular psoriasis) or burned (erythrodermic) appearance. In such cases, the doctor would determine whether treatment has resulted in the reduction of these symptoms. In the case of an tissue or organ in which a subject where a doctor judges that a biopsy is clinically available and/or necessary or in an animal model of the human disease, tissue fragments of bioposies would be prepared and tissue histological structure would be assessed by a clinical pathologist and/or trained histopathologist to determine if reduction in fibrosis and restoration of normal tissue structure and function has occurred. The area of fibrosis to normal tissue could also be quantitatively assessed on such histological preparations.

The provided method can restore normal tissue mechanical properties such as tensile strength following tissue injury in a subject. "Tensile strength" refers to the amount of stress or strain required to break the tissue or wound.

The tensile strength of treated wounds can be 60, 65, 70, 75, 80, 85, 90, 95, 100% that of uninjured tissue within 3 months after treatment. Thus, provided is a method of restoring tissue mechanical properties, including increasing tensile strength of a healed injury to approach or reach that of normal uninjured tissue, in a subject comprising administering to the subject one or more of the herein provided compositions (e.g., polypeptides, nucleic acids, or vectors) in a pharmaceutically acceptable carrier.

The type of wounds that would be important with respect to tensile strength/extensibility would include injuries to musculoskeletal structures/tissues, and the skin covering these structures. For example, the provided methods can improve tensile strength of articulating joints, bone, cartilage, tendons, or ligaments. The provided methods can also improve tensile strength of skin under higher degrees of stress/strain, such as the skin covering the elbow, knee, or foot. The most common problems associated with healing of joint injuries is that excessive scarring in these areas leads to contraction, and non-extensibility of the healed joint area. This has serious cosmetic and psychological consequences. The properties of the peptides will help modulate and lessen the formation of such scar tissue leading to greater mobility of the joint.

The provided method can improve tissue regeneration following tissue injury in a subject. By "regeneration" is meant the renewal, re-growth, or restoration of a body or a bodily part, tissue, or substance after injury or as a normal bodily process. In contrast to scarring, tissue regeneration involves the restoration of the tissue to its original structural, functional, and physiological condition. This is also referred to herein as tissue "complexity". The restoration can be partial or complete, meaning 10, 20, 30, 40, 50, 60, 70, 80, 90, 100% restoration, or any amount of restoration in between as compared to native or control levels. As an example, in the case of a skin injury, tissue regeneration can involve the restoration of hair follicles, glandular structures, blood vessels, muscle, or fat. In the case of a brain injury, tissue regeneration can involve maintenance or restoration of neurons. As an example in the case of skin an improvement in tissue regeneration can be assessed by measurements of the volume of fibrous scar tissue to normal regenerated skin as a ratio. As another example, counts can be made of discrete regenerating structures such as regenerating skin glands normalized to the volume of the wound area.

In one aspect, tissue regeneration involves the recruitment and differentiation of stem cells to replace the damaged cells. As used herein, a "stem cell" is an undifferentiated cell found among differentiated cells in a tissue or organ, or introduced from an external source for e.g., Embryonic stem cells, Adult Bone Marrow stem cells, that can renew itself and differentiate to yield the major specialized cell types of the tissue or organ. The primary roles of stem cells in a living organism are to maintain and repair the tissue in which they are found. By stem cell differentiation is meant the process whereby an unspecialized cell (e.g., stem cell) acquires the features of a specialized cell such as a skin, neural, heart, liver, or muscle cell. As an example, in the case of a skin injury, tissue regeneration can involve the differentiation of stem cells present in the epithelium into hair follicles (Alonso and Fuchs, 2003). In the case of a brain injury, tissue regeneration can involve the differentiation of stem cells into neurons. The provided method can enhance stem cell differentiation following tissue injury in a subject. Enhanced stem cell differentiation can be measured by providing a clinically acceptable genetic or other means of marking endogenous or engrafted stem cells and determining the frequency of differentiation and incorporation of marked stem cells into normal tissue structures. As another example, certain structures such as hair follicles are known to be regenerated from endogenous stem cells following tissue injury. As such, counts of hair follicles normalized to tissue injury area would serve as a quantitative assessment of enhanced stem cell differentiation.

The provided method can reduce inflammation in a subject. By "inflammation", "inflammatory response" or "immune response" is meant the reaction of living tissues to injury, infection or irritation characterized by redness, warmth, swelling, pain, and loss of function, produced as the result of increased blood flow and an influx of immune cells and secretions. Inflammation is the body's reaction to invading infectious microorganisms and results in an increase in blood flow to the affected area, the release of chemicals that draw white blood cells, an increased flow of plasma, and the arrival of monocytes (or astrocytes in the case of the brain) to clean up the debris. Anything that stimulates the inflammatory response is said to be inflammatory. Thus, in addition to reducing inflammation in a subject in response to tissue injury, the provided compositions and methods can also be used to treat disorders associated with pathological increases in levels of inflammatory cells, including, for example, asthma, eczema, sinusitis, atherosclerosis, rheumatoid arthritis, inflammatory bowel disease, cutaneous and systemic mastocytosis, psoriasis, and multiple sclerosis. Treatment with the provided polypeptide can also reduce itching, for example of healing wounds. Generally, itching results from histamine release by mast cells. The provided polypeptide can reduce mast cell degranulation and histamine release. Thus, the provided polypeptide can be used to treat conditions involving histamine release, including, but not limited to, itching, scratching, sinus irritation, allergic cough, red eyes, asthma, and eczema.

A reduction in inflammation can be measured by a reduction in the density of inflammatory cell types such as, for example, monocytes or astrocytes. A reduction in inflammation can be measured by a reduction in the density of inflammatory cell types such as, for example, neutrophils, mast cells, basophils, and monocytes. A reduction in inflammation can be calculated by an *in vivo* measurement of neutrophil activity (Jones et al., 1994). In addition factors like frequency of mast cell degranulation or measurement of histamine levels or levels of reactive oxygen species can be used as measurements of reduction in inflammation. The level of inflammation can also be indirectly measured by checking for transcription levels of certain genes by qRT-PCR for e.g. genes like, Interferon-alpha, -beta and -gamma, Tumor Necrosis Factor-alpha, Interleukme 1beta, -2, -4, -5, -6, -8, -12, -18, -23, -27, CD4, CD28, CD80, CD86, MHCII, and iNOS. Measurement of pro-inflammatory cytokine levels in the tissues and or bodily fluids of the subject including plasma can measure a reduction in inflammation. It is noteworthy that a mechanism of ACT peptide action may be by inhibition of inflammatory cell migration and/or inhibition of pro-inflammatory chemicals (histamine, reactive oxygen species) and pro-inflammatory cytokines such as Interleukin (IL)-1, IL-6, IL-8 and tumor necrosis factor (TNF).

The provided method can inhibit proliferation of a transformed cell in a subject. By transformed cell is meant a neoplasm, cancer, or tumor cell that divides and reproduces abnormally with uncontrolled growth. Thus, inhibition of proliferation (i.e., hyperplasia) of said transformed cell results in a reduction in the growth and thus malignancy of the cancer. A representative but non-limiting list of cancers that the disclosed compositions and methods can be used to treat is the following: glioma, lymphoma, B cell lymphoma, T cell lymphoma, mycosis fungoides, Hodgkin's Disease, myeloid leukemia, bladder cancer, brain cancer, nervous system cancer, head and neck cancer, squamous cell carcinoma of head and neck, kidney cancer, lung cancers such as small cell lung cancer and non-small cell lung cancer, neuroblastoma, glioblastoma, ovarian cancer, pancreatic cancer, prostate cancer, skin cancer, liver cancer, melanoma, squamous cell carcinomas of the mouth, throat, larynx, and lung, colon cancer, cervical cancer, cervical carcinoma, breast cancer, and epithelial cancer, renal cancer, genitourinary cancer, pulmonary cancer, esophageal carcinoma, head and neck carcinoma, large bowel cancer, hematopoietic cancers, testicular cancer, colon and rectal cancers, prostatic cancer, or pancreatic cancer. Thus, the provided method can be used to treat cancer in a subject. For example, the provided method can be used to treat glioma in a subject.

An inhibition in transformed cell proliferation can be measured by a variety of cell proliferation markers and kits for e.g. Ki67/MIB-1 immunostaining, tritiated thymidine or bromodeoxyuridine labeling indices, DNA S-phase fraction, proliferating cell nuclear antigen expression, potential doubling time and analysis of the nucleolar organizer region associated proteins (AgNORs). Since the proliferative activity of the tumor depends both on the proportion of cells committed to the cycle (growth fraction) and the speed of the cell cycle, the actual proliferative activity of a tumor could well be measured by the equation [PA=Ki67 or MIB-1 scores X AgNORs] (Pich et al., 2004). In another example, histopathologists are skilled in assessing biopsy tissue sections using simple qualitative and quantitative indices of mitosis to determine proliferation in transformed cell populations

Various mouse models have been developed for cancer research. There are specific mouse models for specific types of cancers. For example, Bladder cancer, Cervical cancer, Endometrial cancer, Gastrointestinal cancer, Genitourinary cancer, Head and Neck cancer, Hematopoietic cancer, Kidney cancer, Lung cancer, Mammary Gland cancer, Melanoma, Myeloma, Nervous System cancer, Oral cancer, Ovarian cancer, Pancreatic cancer, Prostate cancer, Sarcoma, Skin cancer. These models are well described and used. The favorable effects of the polypeptides, nucleic acids or vectors provided herein can be studied in any of these models. For example the skin cancer mouse model can be easily used for demonstration. Cancers can be cultivated applying the xenograft model of growing human cancerous tissues using the specific pathogen free, homo inbred mouse (a nude mouse) (Yoo, 2004). The polypeptides, nucleic acids or vectors provided herein can be locally administered for e.g. bioengineered materials such as a hollow fiber membranes (Orlandini and Margaria. 1983; Ming Chu et al., 1998) and microfibers, slow release beads, hypodermic needles, indwelling catheters, which can be inserted locally into the cancerous growth, or systemically administered to reach its target for e.g. intravenous infusions, intramuscularly, intraperitoneal injection. This treatment can be administered by itself or in combination with other therapeutic compounds for e.g. Chemotherapeutic agents.

The provided method can inhibit metastasis of a transformed cell in a subject. By "metastasis" is meant the transmission of cancer cells from an original site to one or more sites elsewhere in the body, usually by way of the blood vessels or lymphatics. Metastasis can be broken down into a series of events. First, cancer cell migration begins the process by which tumor cells leave the primary site of growth, often penetrating the basement membrane and moving towards the local vasculature. Intravasation describes the process of cancer cell entry into the vasculature, and distribution to distant sites. Extravasation refers to the process of cancer cell egression from the vasculature. Finally, proliferation of cancer cells at the distant site is profoundly influenced by localized growth factor availability, influences of stromal cells, and the surrounding extracellular matrix milieu (the so-called "soil") as well as the availability of nutrients and factors provided by the resultant vascularization of the growing tumor. Thus, the provided compositions and methods can inhibit metastasis of a transformed cell in a subject by inhibiting migration (i.e., metastatic migration) of said cell. Tumourigenesis is the result of cell cycle disorganization, leading to an uncontrolled cellular proliferation. Specific cellular processes-mechanisms that control cell cycle progression and checkpoint traversation through the intermitotic phases are deregulated. Normally, these events are highly conserved due to the existence of conservatory mechanisms and molecules such as cell cycle genes and their products. An inhibition in metastatic migration can be measured by the levels of such cell cycle genes and products for e.g. cyclins, cyclin dependent kinases (Cdks), Cdk inhibitors (CKI) and extra cellular factors (i.e. growth factors). Revolutionary techniques using laser cytometry and commercial software are available to quantify and evaluate cell cycle processes and cellular growth. S-phase fraction measurements, including ploidy values, using histograms and estimation of indices such as the mitotic index and tumour-doubling time indices, provide adequate information to the clinician to evaluate tumour aggressiveness.

As used herein, tissue injury can result from, for example, a scrape, cut, laceration wound, crush wound, compression wound, stretch injury, bite wound, graze, bullet wound, explosion injury, body piercing, stab wound, burn wound, wind burn, sun burn, chemical burn, surgical wound, surgical intervention, medical intervention, host rejection following cell, tissue or organ grafting, pharmaceutical effect, pharmaceutical side-effect, bed sore, radiation injury, cosmetic skin wound, internal organ injury, disease process (e.g., asthma, cancer), infection, infectious agent, developmental process, maturational process (e.g., acne), genetic abnormality, developmental abnormality, environmental toxin, allergen, scalp injury, facial injury, jaw injury, foot injury, toe injury, finger injury, bone injury, sex organ injury, joint injury, excretory organ injury, eye injury, corneal injury, muscle injury, adipose tissue injury, lung injury, airway injury, hernia, anus injury, piles, ear injury, retinal injury, skin injury, abdominal injury, arm injury, leg injury, athletic injury, back injury, birth injury, premature birth injury, toxic bite, sting, tendon injury, ligament injury, heart injury, heart valve injury, vascular system injury, cartilage injury, lymphatic system injury, craniocerebral trauma, dislocation, esophageal perforation, fistula, nail injury, foreign body, fracture, frostbite, hand injury, heat stress disorder, laceration, neck injury, self mutilation, shock, traumatic soft tissue injury, spinal cord injury, spinal injury, sprain, strain, tendon injury, ligament injury, cartilage injury, thoracic injury, tooth injury, trauma, nervous system injury, aging, aneurism, stroke, digestive tract injury, infarct, or ischemic injury.

In certain embodiments, the tissue injury results from radiation injury. In certain embodiments, radiation injury includes, without limitation, cutaneous radiation injury (i.e., injury to the skin and underlying tissues from acute exposure to radiation), acute radiation syndrome (i.e., serious illness that occurs following an acute high dose of penetrating whole body irradiation), radiation burns, radiation dermatitis, radiation injury to the nervous system or brain, radiation pneumonitis, and radiation-induced enteritis. In certain aspects, the radiation injury is cutaneous radiation injury. In other aspects, the radiation injury is radiation burns. In yet other aspects, the radiation injury is radiation dermatitis. In some embodiments, the cutaneous radiation injury results from a dirty bomb attack.

In some embodiments, the radiation injury occurs in a tissue including, without limitation, skin, heart, bone, brain, spinal cord, cornea, retina, and peripheral nerve. In certain aspects, the radiation injury occurs on the skin. In certain embodiments, radiation-induced tissue injury causes damage to the basal cell layer of the skin. In some aspects, radiation-induced damage to the basal cell layer of the skin results in symptoms including, but not limited to, inflammation, erythema, desquamation, intense reddening, blistering, and ulceration of the irradiated site.

In certain embodiments, radionuclide-contaminated wounds and burns promote systemic uptake of radionuclides that greatly complicate topical decontamination and triage. In other embodiments, radionuclide contamination also interferes with wound healing. Therefore, in certain aspects, the provided polypeptides prevent or treat radiation injury by preventing systemic uptake of radionuclides from a wound or burn at the site of radiation injury. In other aspects, the provided polypeptides prevent or treat radiation injury by reducing hemorrhage, severity of thermal burn injury, and scar tissue formation at the site of radiation injury. In yet other aspects, the provided polypeptides promote tissue regeneration at the site of radiation injury.

The peptides and/or other formulations embodying the invention will modulate cell migration and proliferation, thereby reducing inflammation, accelerating wound healing, reduce scarring and ultimately promote repair, regeneration and restoration of structure and function in all tissues. Healing of wounds, post-peptide application will involve significantly reduced fibrosis, consequently reduced scarring in skin wounds and fibrous patches in internal tissue injuries, promoting tissue regeneration and restoring tissue and organ structure and function.

Said peptides and/or other formulations can be used to treat internal injury caused by an external wound. In certain embodiments, the external wound is caused by radiation injury. In some embodiments, internal injury may be caused by radionucleotides that cause internal systemic contamination via contaminated burns and wounds. Thus, in some embodiments, the methods and compositions provided herein provide treatment, prevention, and/or inhibition of the progression of internal radiation damage. The internal radiation damage, in some embodiments, results from systemic contamination with radiation. Systemic contamination with radiation may result from direct administration of radionucleotides or entry of radionucleotides from contaminated burns and wounds. In certain aspects, radiation injury includes, without limitation, cutaneous radiation injury (i.e., injury to the skin and underlying tissues from acute exposure to radiation), acute radiation syndrome (i.e., serious illness that occurs following an acute high dose of penetrating whole body irradiation), radiation burns, radiation dermatitis, radiation injury to the nervous system or brain, radiation pneumonitis, and radiation-induced enteritis. In certain aspects, the radiation injury is cutaneous radiation injury. In other aspects, the radiation injury is radiation burns. In yet other aspects, the radiation injury is radiation dermatitis. In some embodiments, the cutaneous radiation injury is caused by a dirty bomb attack.

Injury to internal organs causes a fibrotic response, which leads to loss of structure and function in organ systems. In central nervous system (CNS) this response to injury is mediated by astrocytes (fibroblast-like cells in the CNS) and thus will subsequently be referred to as an astrocytic response. Embodiments of our invention will alleviate this fibrotic/astrocytic response hence helping in repair and regeneration of injured tissues and restoration of tissue and organ structure and function. Further embodiments of the inventions include the use of said peptides and/or other formulations to improve angiogenesis by stimulating angiogenic factors like, but not limited to VEGF, and improve differentiation of vascular tissues thereby improving blood flow to the site of tissue injury. Increased blood supply to the wound site stimulated by our treatments will result in reduced scarring in external and internal wounds and promote improved repair and regeneration of tissues and organs. Additional embodiments of the invention comprises the use of said peptides and/or other formulations for tissue and organ regeneration, when administered in association with stem cells and/or drugs and/or other endogenous and/or clinical regimens promoting stem cell mobilization and/or tissue regeneration. Stem cells will help in tissue regeneration and our treatment will promote differentiation directly and/or indirectly by processes that include, but are not limited to reduced fibrotic/astrocytic scar formation, thereby restoring normal tissue structure and function.

The compositions and methods promote the generation of a permissive environment in vivo for regeneration and restoration of structure and function of tissues and organs. Regenerative processes aided by our peptide include, but are not limited to internal and external injury, regeneration of tissues, organs, or other body parts, healing and restoration of function following vascular occlusion and ischemia, brain stroke, myocardial infarction, spinal cord damage, brain damage, peripheral nerve damage, retinal damage, bone damage, exposure to radiation, and other insults to tissues causing destruction, damage or otherwise resulting from, but not limited to, injury, surgery, cancer, congenital and developmental malformation, and diseases causing progressive loss of tissue structure and function, including but not limited to diabetes, bacterial, viral and prion-associated diseases, Alzheimer's disease, Parkinson's disease, AIDs and other genetically determined, environmentally determined or idiopathic disease processes causing loss of tissue/organ/body part structure and function. In addition, the provided peptides can be administered with drugs or other compounds promoting tissue and cellular regeneration including, but not limited to, trophic factors in processes including, but not limited to, brain, retina, spinal cord and peripheral nervous system regeneration (e.g., NGFs, FGFs, Neurotrophins, Neuregulins, Endothelins, GDNFs, BDNF, BMPs, TGFs, Wnts).

Additional embodiments of the invention comprise the delivery of said peptides and/or other formulations using techniques such as, but not limited to, all Antennapedia sequences, and related cell internalization vectors (e.g., TAT protein transduction domain, all TAT peptides, all TAT fusion proteins), viral gene delivery vectors, DNA expression vectors, and any other delivery method that can help get our peptide to the tissue and/or cellular site of action by itself or in association with other agents including but not limited to co-factors assisting this delivery (e.g., including but limited to TAT-HA2) and/or stem cells, drugs and other formulations which help in repair, regeneration and restoration of organ and tissue structure and function.

The compositions disclosed herein and the compositions necessary to perform the disclosed methods can be made using any method known to those of skill in the art for that particular reagent or compound unless otherwise specifically noted.

For example, the provided nucleic acids can be made using standard chemical synthesis methods or can be produced using enzymatic methods or any other known method. Such methods can range from standard enzymatic digestion followed by nucleotide fragment isolation (see for example, Sambrook et al., Molecular Cloning: A Laboratory Manual, 2nd Edition (Cold Spring Harbor Laboratory Press, Cold Spring Harbor, N.Y., 1989) Chapters 5, 6) to purely synthetic methods, for example, by the cyanoethyl phosphoramidite method using a Milligen or Beckman System 1Plus DNA synthesizer (for example, Model 8700 automated synthesizer of Milligen-Biosearch, Burlington, Mass. or ABI Model 380B). Synthetic methods useful for making oligonucleotides are also described by Ikuta et al., Ann. Rev. Biochem. 53:323-356 (1984), (phosphotriester and phosphite-triester methods), and Narang et al., Methods Enzymol., 65:610-620 (1980), (phosphotriester method). Protein nucleic acid molecules can be made using known methods such as those described by Nielsen et al., Bioconjug. Chem. 5:3-7 (1994).

One method of producing the disclosed polypeptides, such as SEQ ID NO:2, is to link two or more peptides or polypeptides together by protein chemistry techniques. For example, peptides or polypeptides can be chemically synthesized using currently available laboratory equipment using either Fmoc (9-fluorenylmethyloxycarbonyl) or Boc (*tert-*butyloxycarbonoyl) chemistry. (Applied Biosystems, Inc., Foster City, Calif.). One skilled in the art can readily appreciate that a peptide or polypeptide corresponding to the disclosed proteins, for example, can be synthesized by standard chemical reactions. For example, a peptide or polypeptide can be synthesized and not cleaved from its synthesis resin whereas the other fragment of a peptide or protein can be synthesized and subsequently cleaved from the resin, thereby exposing a terminal group which is functionally blocked on the other fragment. By peptide condensation reactions, these two fragments can be covalently joined via a peptide bond at their carboxyl and amino termini, respectively, to form a protein, or fragment thereof. (Grant G A (1992) Synthetic Peptides: A User Guide. W.H. Freeman and Co., N.Y. (1992); Bodansky M and Trost B., Ed. (1993) Principles of Peptide Synthesis. Springer-Verlag Inc., NY (which is herein reffered to at least for material related to peptide synthesis). Alternatively, the peptide or polypeptide is independently synthesized *in vivo* as described herein. Once isolated, these independent peptides or polypeptides may be linked to form a peptide or fragment thereof via similar peptide condensation reactions.

For example, enzymatic ligation of cloned or synthetic peptide segments allow relatively short peptide fragments to be joined to produce larger peptide fragments, polypeptides or whole protein domains (Abrahmsen L et al., Biochemistry, 30:4151 (1991)). Alternatively, native chemical ligation of synthetic peptides can be utilized to synthetically construct large peptides or polypeptides from shorter peptide fragments. This method consists of a two step chemical reaction (Dawson et al. Synthesis of Proteins by Native Chemical Ligation. Science, 266:776-779 (1994)). The first step is the chemoselective reaction of an unprotected synthetic peptide--thioester with another unprotected peptide segment containing an amino-terminal Cys residue to give a thioester-linked intermediate as the initial covalent product. Without a change in the reaction conditions, this intermediate undergoes spontaneous, rapid intramolecular reaction to form a native peptide bond at the ligation site (Baggiolini M et al. (1992) FEBS Lett. 307:97-101; Clark-Lewis I et al., J. Biol. Chem., 269:16075 (1994); Clark-Lewis I et al., Biochemistry, 30:3128 (1991); Rajarathnam K et al., Biochemistry 33:6623-30 (1994)).

Alternatively, unprotected peptide segments are chemically linked where the bond formed between the peptide segments as a result of the chemical ligation is an unnatural (non-peptide) bond (Schnolzer, M et al. Science, 256:221 (1992)). This technique has been used to synthesize analogs of protein domains as well as large amounts of relatively pure proteins with full biological activity (deLisle Milton R C et al., Techniques in Protein Chemistry IV. Academic Press, New York, pp. 257-267 (1992)).

Disclosed are processes for making the compositions as well as the intermediates leading to the compositions. There are a variety of methods that can be used for making these compositions, such as synthetic chemical methods and standard molecular biology methods. It is understood that the methods of making these and the other disclosed compositions are specifically disclosed. Disclosed are nucleic acid molecules produced by the process comprising linking in an operative way a nucleic acid encoding a polypeptide disclosed herein and a sequence controlling the expression of the nucleic acid. Disclosed are cells produced by the process of transforming the cell with any of the herein disclosed nucleic acids. Disclosed are any of the disclosed peptides produced by the process of expressing any of the herein disclosed nucleic acids. Disclosed are animals produced by the process of transfecting a cell within the animal with any of the nucleic acid molecules disclosed herein. Disclosed are animals produced by the process of transfecting a cell within the animal any of the nucleic acid molecules disclosed herein, wherein the animal is a mammal. Also disclosed are animals produced by the process of transfecting a cell within the animal any of the nucleic acid molecules disclosed herein, wherein the mammal is mouse, rat, rabbit, cow, sheep, pig, or primate. Also disclose are animals produced by the process of adding to the animal any of the cells disclosed herein.

The materials described above as well as other materials can be packaged together in any suitable combination as a kit useful for performing, or aiding in the performance of, the disclosed method. It is useful if the kit components in a given kit are designed and adapted for use together in the disclosed method. For example disclosed are kits for promoting wound healing, the kit comprising one or more of the polypeptides, nucleic acids or vectors provided herein in a pharmaceutically acceptable carrier. Such kits can also include gels, bandages, Millipore tapes, Medicated Q-tips, Sprays, props, Syrups, Liquids, Disposable tubes or pouches. The kits also can contain instructions for proper use and safety information of the product or formulation. The kits may contain dosage information based on the application and method of administration as determined by a doctor.

The disclosed methods and compositions are applicable to numerous areas including, but not limited to, laboratory research tools. These formulations play regulatory roles in several cellular processes for e.g. Cell Proliferation, Cell Migration. These formulations can be used in the laboratory in both *in vitro* and *in vivo* model systems for studying various cellular processes, cell cycle regulations, cell behavior, responses of cells, organs or tissues to test compounds etc. The formulations can be supplied by themselves or in combination with other compounds or as part of a kit, such as a kit for cell proliferation assay. The kit may contain the formulations mentioned herein by themselves or in combination with other compounds. Such a kit would include instructions designed to facilitate the experiment. Other uses are disclosed, apparent from the disclosure, and/or will be understood by those in the art.

### Examples

### Example 1: Efficacy of topical administration of Granexin^{®} in the treatment of, and the prevention of a cutaneous radiation injury - delayed application study

Due to similarities of pig and human skin, the pig is considered to be the optimal large animal model to study CRI. Validating studies support Yorkshire pigs as the best animal model for evaluation of efficacy of medical countermeasures to prevent and mitigate the progression of CRI, where 45 Gy cutaneous exposure (delivered in one dose) results in a controlled, reproducible full-thickness cutaneous radiation injury; without causing systemic morbidity or mortality. Using this validated Yorkshire pig model of CRI, an efficacy and dose response study was conducted to evaluate the efficacy of topical administration of Granexin^{®} to prevent and mitigate the progression and severity of CRT. Four animals (2 male and 2 female Yorkshire pigs (S&S Farms)), approximately 3 months of age, 32.4 - 41.1 kg at irradiation were used. On Day 0, each animal was anesthetized and exposed to single fractions of low energy electrons (6 MeV) using a Varian CLINAC 21EX Linear Accelerator to 10 separate 4-cm diameter sites on the paraspinal dorsal skin surface, at an acute target dose of 45 Gy. Irradiation parameters were established to mimic characteristic depth dose drop off patterns associated with beta-type irradiation exposures, similar to beta energy of Sr-90 and other likely isotopes from a RDD. For each site, lead shielding was used to restrict the cone-generated (15 ×15 cm2 cone) electron beam field to the desired exposure area of a 4-cm diameter circle. Tissue equivalent bolus material (8 mm) was placed on the skin surface to ensure that greater than 90% of the prescribed dose was limited to a maximum depth of 2 cm. For irradiation, exposure (Monitor Units, MU) was calculated using the formula: *MU* = *Dose*/*((Total Output Factor) X (Normalization Factor)) where:* MU is the machine monitor unit setting; Dose is the nominal delivered dose, in cGy; Total Output Factor is the output factor which includes the appropriate cone and lead cutout at 100 cm source to surface distance; and Normalization factor is the output measured on the day of irradiation.

The objective of this study was to evaluate the efficacy of topical administration of Granexin^{®} in the treatment of, and the prevention of a cutaneous radiation injury model using the Yorkshire pig. More specifically, the study was conducted to assess whether Granexin^{®} gel could slow the progression and/or mitigate the severity of symptoms associated with CRT.

Granexin^{®} is a topical gel comprising 1.25% hydroxyethyl cellulose gel and the ACT1 peptide. The chemical structure of the ACT1 peptide in Granexin^{®} is: Biotin-Ahx-Arg-Gln-Pro-Lys-Ile-Trp-Phe-Pro-Asn-Arg-Arg-Lys-Pro-Trp-Lys-Lys-Arg-Pro-Arg-Pro-Asp-Asp-Leu-Glu-Ile-OH (SEQ ID NO: 91), wherein Ahx is L-2-aminohexanoic acid (6-aminohexanoic acid). Granexin^{®} used in the present example also included the following inactive ingredients:
▪ Preservatives: methylparaben (0.17% w/w) and propylparaben (0.02% w/w)
▪ Solvents: glycerin (5% w/w), propylene glycol (3% w/w), and purified water (quantity sufficient)
· Buffer agents: sodium phosphate monobasic (0.263% w/w) and sodium phosphate dibasic
   (0.044% w/w)
▪ Chelating agent: edetate disodium (0.05% w/w)
· Stabilizer: D-mannitol (0.05% w/w)

In the study, Granexin^{®} gel was packaged as single-patient-use-containers, containing 5g of gel per tube. Granexin^{®} is a clear, odorless, hypoallergenic, and easy-to-apply gel.

In this study, topical treatment with Granexin^{®} (100 µM or 200 µM) or vehicle treatment was initiated upon observation of erythema (Kumar score > 1.0) at any site (between 11-14 days after exposure to radiation injury) and continued for the course of the study. Beginning on Day 1 and every 3 days thereafter, documentation of erythema, desquamation, and injury (eschar, ulceration, and necrosis) was taken and skin sites were systematically scored (blindly) for erythema and desquamation using the Kumar scale.

Results of treatment are shown in Table 10 below and illustrated in Figure 1A. The Kumar Scale is shown in Table 11.

**Table 10: Average skin score comparisons (± SD)**

| **Day post irradiation** | **Vehicle (n=8)** | **Granexin 100 µM (n=8)** | **Granexin 200 µM (n=8)** |
|---|---|---|---|
| 19 | 1.88 ± 0.25 | 1.63 ± 0.48 | 1.63 ± 0.75 |
| 22 | 2.38 ± 0.48 | 1.75 ± 0.50 | 1.88 ± 0.75 |
| 28 | 3.63 ± 0.25 | 2.88 ± 1.25 | 2.50 ± 1.73 |
| 31 | 3.75 ± 0.29 | 3.00 ± 1.35 | 2.63 ± 1.60 |

**Table 11: Kumar Scale**

| **Score** | **Score Skin Changes** |
|---|---|
| 1.0 | No effect |
| 1.5 | Minimal erythema, mild dry skin |
| 2.0 | Moderate erythema, dry skin |
| 2.5 | Marked erythema, dry desquamation |
| 3.0 | Dry desquamation, minimal dry crusting |
| 3.5 | Dry desquamation, dry crusting, superficial minimal scabbing |
| 4.0 | Patchy moist desquamation, moderate scabbing |
| 4.5 | Confluent moist desquamation, ulcers, large deep scabs |
| 5.0 | Open wound, full thickness skin loss |
| 5.5 | Necrosis |

Subsequent time points demonstrate clear visual treatment differences, as shown in Figure 1B.

The results of the study showed that radiation sites treated with Granexin^{®} (100 µM or 200 µM) showed a decrease in erythema and development of desquamation, as evaluated by the Kumar scale as compared to vehicle treated sites. Thus, the composition was effective in prevention of injury progression and severity, a decrease in erythema and development of desquamation.

Observations of lower Kumar scores with Granexin^{®} 100 µM and 200 µM were consistent with microscopic histopathological findings. Skin tissue was collected from each irradiation site, fixed in 10% neutral buffered formalin, processed to paraffin block, sections and stained with Hematoxylin and Eosin (H&E). Sections were examined (blinded) microscopically. Granexin^{®} gel was more effective at mitigating lesion severity than in sites that treated with vehicle gel, as evaluated by H & E staining of biopsies taken at terminal animal necropsy (Day 43; End of Study) (Figure 1C). The histopathological study showed a dose response effect, where the 200 µM Granexin^{®} concentration was highly effective in mitigating severity (ulceration, neutrophil infiltration).

### Example 2: Treatment with ACT polypeptide composition to prevent and mitigate the progression of CRI following exposure to ionizing radiation - early application study (treatment initiation 24 hours post irradiation)

To evaluate the potential of Granexin^{®} to prevent cutaneous CRI progression, severity, and ulceration, irradiated sites were prophylactically treated (prior to the presentation of CRI symptoms) with vehicle or Granexin^{®} 1,000 µM. Granexin^{®} was applied starting at 24 hours post exposure and continued once daily for 30 days. Briefly, on Day 0, each animal was anesthetized and exposed to single fractions of low energy electrons (6 MeV) using a Varian CLINAC 21EX Linear Accelerator at a target dose of 45 Gy. Granexin^{®} or vehicle gel was applied once a day starting at 24 hours post radiation exposure.

Irradiated sites treated with Granexin^{®} gel showed delayed injury progression and ulceration and reduced injury severity of CR1 as compared to sites treated with vehicle gel. Day 21 photographic comparisons show that prophylactic treatment with Granexin^{®} at 1,000 µM prevents cutaneous radiation injury development as compared to control (Figure 2).

These data showed that Granexin^{®} has a beneficial effect in both preventing and treating cutaneous radiation injury. The data further document a positive dose response curve.

### Example 3. Treatment with ACT polypeptide composition in a GI-targeted murine partial body irradiation model

In this study, C557BL/6 mice (n=40; 20 females and 20 males) were exposed to partial body irradiation by one dose exposure of 15.6 at 50cGy/min using a 60Co gamma source. A Farmer ionization chamber was used to provide real-time quantification of the radiation dose. Animals were irradiated in a custom designed restrainer where their left pelvic limb was extended and maintained in position to permit shielding with a cerrobend structure. This method of shielding 5% of bone marrow serves to rescue mice from hematopoietic syndrome1. Initiating 24 hrs post exposure, animals were treated daily with 0.9% sodium chloride (vehicle) or 10 mg/kg ACT11 (ACT1 polypeptide) delivered intraperitoneally for up to 21 days. Half of the animals (subtracting those that died on days 1-7) were euthanized on day 8 to allow for histological examination of GI organs (histology results pending). Preliminary Kaplan Maier survival analyses indicate that systemic ACT11 treatment has an early effect on animal mortality (Figure 4). Therefore, the results of the study showed that the ACT1 polypeptide reduces internal radiation damage from irradiation.

It is understood that the disclosed method and compositions are not limited to the particular methodology, protocols, and reagents described as these may vary. It is also to be understood that the terminology used herein is for the purpose of describing particular embodiments only, and is not intended to limit the scope of the present invention which will be limited only by the appended claims.

It must be noted that as used herein and in the appended claims, the singular forms "a", "an", and "the" include plural reference unless the context clearly dictates otherwise. Thus, for example, reference to "a polypeptide" includes a plurality of such polypeptides, reference to "the polypeptide" is a reference to one or more polypeptides and equivalents thereof known to those skilled in the art, and so forth.

"Optional" or "optionally" means that the subsequently described event, circumstance, or material may or may not occur or be present, and that the description includes instances where the event, circumstance, or material occurs or is present and instances where it does not occur or is not present.

Ranges may be expressed herein as from "about" one particular value, and/or to "about" another particular value. When such a range is expressed, also specifically contemplated and considered disclosed is the range from the one particular value and/or to the other particular value unless the context specifically indicates otherwise. Similarly, when values are expressed as approximations, by use of the antecedent "about," it will be understood that the particular value forms another, specifically contemplated embodiment that should be considered disclosed unless the context specifically indicates otherwise. It will be further understood that the endpoints of each of the ranges are significant both in relation to the other endpoint, and independently of the other endpoint unless the context specifically indicates otherwise. Finally, it should be understood that all of the individual values and sub-ranges of values contained within an explicitly disclosed range are also specifically contemplated and should be considered disclosed unless the context specifically indicates otherwise. The foregoing applies regardless of whether in particular cases some or all of these embodiments are explicitly disclosed.

Unless defined otherwise, all technical and scientific terms used herein have the same meanings as commonly understood by one of skill in the art to which the disclosed method and compositions belong. Although any methods and materials similar or equivalent to those described herein can be used in the practice or testing of the present method and compositions, the particularly useful methods, devices, and materials are as described. Nothing herein is to be construed as an admission that the present invention is not entitled to antedate such disclosure by virtue of prior invention. No admission is made that any reference constitutes prior art. The discussion of references states what their authors assert, and applicants reserve the right to challenge the accuracy and pertinency of the cited documents. It will be clearly understood that, although a number of publications are referred to herein, such reference does not constitute an admission that any of these documents forms part of the common general knowledge in the art.

Throughout the description and claims of this specification, the word "comprise" and variations of the word, such as "comprising" and "comprises," means "including but not limited to," and is not intended to exclude, for example, other additives, components, integers or steps.

Those skilled in the art will recognize, or be able to ascertain using no more than routine experimentation, many equivalents to the specific embodiments of the method and compositions described herein.

### References

Alonso L, Fuchs E. Stem cells of the skin epithelium. Proc Natl Acad Sci USA. 2003 Sep. 30, 100 Suppl 1:11830-5. 2003
Barker R J, Price R L, Gourdie R G. Increased association of ZO-1 with Connexin43 during remodeling of cardiac gap junctions. Circ Res. February 22; 90(3):3 17-24 (2002).
Bucci, M. et al. In vivo delivery of the caveolin-1 scaffolding domain inhibits nitric oxide synthesis and reduces inflammation. Nat. Med. 6, 1362-1367 (2000).
Chien K R. Stem cells: lost in translation. Nature. April 8; 428(6983):607-608 (2004).
Derossi, D., Joliot, A. H., Chassaing, G. & Prochiantz, A. The third helix of Antennapedia homeodomain translocates through biological membranes. J. Biol. 679-686 (2000).
Elmquist, A., Lindgren, M., Bartfai, T. & Langel, U. VE-cadherin-derived cell-penetrating peptide, pVEC, with carrier functions. Exp. Cell Res. 269, 237-244 (2001).
Elder D., Elenitsas R, Jawaorsky C, & Johnson B. Lever's histopathology of the skin. Lippincott-Raven Publishers, (1997).
Fawcett J W, Asher R A. The glial scar and central nervous system repair. Brain Res. Bull. 49:377-391 (1999).
Fischer, P. M. et al. Structure-activity relationship of truncated and substituted analogues of the intracellular delivery vector Penetratin. J. Pept. Res. 55, 163-172 (2000).
Frankel, A. D. & Pabo, C. O. Cellular uptake of the Tat protein from human immunodeficiency virus. Cell 55, 1189-1193 (1988).
Fu C T, Bechberger J F, Ozog M A, Perbal B, Naus C. CCN3 (NOV) interacts with Connexin43 in C6 glioma cells: possible mechanism of Connexin-mediated growth suppression. J Biol. Chem. August 27; 279(35):36943-50 (2004).
Gao, C. et al. A cell-penetrating peptide from a novel pVII-pIX phage-displayed random peptide library. Bioorg. Med. Chem. 10, 4057-4065 (2002).
Giepmans B N. Gap junctions and Connexin-interacting proteins. Cardiovasc Res. May 1; 62(2):233-45 (2004).
Goodenough D A, Paul D L. Beyond the gap: functions of unpaired connexon channels. Nat Rev Mol Cell Biol. April; 4(4):285-94 (2003).
Green, M. & Loewenstein, P. M. Autonomous functional domains of chemically synthesized human immunodeficiency virus tat trans-activator protein. Cell 55, 1179-1188 (1988).
Hayashi T, Matesic D F, Nomata K, Kang K S, Chang C, Trosko J E. Stimulation of cell proliferation and inhibition of gap junctional intercellular communication by linoleic acid. Cancer Lett. 112:103-111 (1997).
Hayashi T, Nomata K, Chang C, Ruch R J, Trosko J E. Cooperative effects of v-myc and c-Ha-ras oncogenes on gap junctional intercellular communication and tumorigenicity in rat liver epithelial cells. Cancer Lett. 128:145-154 (1998).
Hayashi T, Trosko J E, Hamada K. Inhibition of gap junctional intercellular communication in rat liver epithelial cells with transforming RNA. FEBS Lett. 491:200-206 (2001).
Hong, F. D. & Clayman, G. L. Isolation of a peptide for targeted drug delivery into human head and neck solid tumors. Cancer Res. 60, 6551-6556 (2000).
Kajstura J, Rota M, Whang B, Cascapera S, Hosoda T, Bearzi C, Nurzynska D, Kasahara H, Zias E, Bonafe M, Nadal-Ginard B, Torella D, Nascimbene A, Quaini F, Urbanek K, Leri A, Anversa P. Bone marrow cells differentiate in cardiac cell lineages after infarction independently of cell fusion. Circ Res. January 7; 96(1):127-37 (2005).
Lin, Y. Z., Yao, S. Y., Veach, R. A., Torgerson, T. R. & Hawiger, J. Inhibition of nuclear translocation of transcription factor NF-KB by a synthetic peptide containing a cell membrane-permeable motif and nuclear localization sequence. J. Biol. Chem. 270, 14255-14258 (1995).
Lundberg, P. et al. Cell membrane translocation of the N-terminal (1-28) part of the prion protein. Biochem. Biophys. Res. Commun. 299, 85-90 (2002).
Matsushita M, Noguchi H, Lu Y F, Tomizawa K, Michiue H, Li S T, Hirose K, Bonner-Weir S, Matsui H. Photo-acceleration of protein release from endosome in the protein transduction system. FEBS Lett. 13; 572(1-3):221-6. (2004).
Morris, M. C., Depollier, J., Mery, J., Heitz, F. & Divita, G. A peptide carrier for the delivery of biologically active proteins into mammalian cells. Nature Biotechnol. 19, 1173-1176 (2001).
Norenberg M D. Astrocyte responses to CNS injury. J. Neuropathol. Exp. Neurol. 53:213-220 (1994).
Oehlke, J. et al. Cellular uptake of an alpha.-helical amphipathic model peptide with the potential to deliver polar compounds into the cell interior non-endocytically. Biochim. Biophys. Acta. 1414, 127-139 (1998).
Park, C. B., Yi, K. S., Matsuzaki, K., Kim, M. S. & Kim, S. C. Structure-activity analysis of buforin II, a histone H2A-derived antimicrobial peptide: the proline hinge is responsible for the cell-penetrating ability of buforin 11. Proc. Natl. Acad. Sci. USA 97, 8245-8250 (2000).
Paxinos G, Watson C. The Rat Brain in Stereotaxic Coordinates. 2nd ed. San Diego, Calif.: Academic; 1986.
Pich A, Chiusa L, Navone R. Prognostic relevance of cell proliferation in head and neck tumors Annals of Oncology 2004 15(9):1319-1329.
Pooga, M., Hallbrink, M., Zorko, M. & Langel, U. Cell penetration by transportan. FASEB J. 12, 67-77 (1998).
Poss K D, Wilson L G, Keating M T. Heart regeneration in zebrafish. Science. December 13; 298(5601):2188-90 (2002).
Rousselle, C. et al. New advances in the transport of doxorubicin through the blood-brain barrier by a peptide vector-mediated strategy. Mol. Pharmacol. 57(4):679-86 (2000).
Sawada, M., Hayes, P. & Matsuyama, S. Cytoprotective membrane-permeable peptides designed from the Bax-binding domain of Ku70. Nature Cell Biol. 5, 352-357 (2003).
Silver J, Miller J H. Regeneration beyond the glial scar. Nat Rev Neurosci. February; 5(2):146-56 (2004).
Songyang, Z. et al. Recognition of unique carboxyl-terminal motifs by distinct PDZ domains. Science 275, 73-77 (1997).
Tsao M S, Smith J D, Nelson K G, Grisham J W. A diploid epithelial cell line from normal adult rat liver with phenotypic properties of 'oval' cells. Exp. Cell Res. 154:38-52 (1984).
Vigneron, J. P. et al. Guanidinium-cholesterol cationic lipids: Efficient vectors for the transfection of eukaryotic cells. Proc. Natl. Acad. Sci. USA. 93, 9682-9686 (1998).
Wadia J S, Stan R V, Dowdy S F. 'I'ransducible TAT-HA fusogenic peptide enhances escape of TAT-fusion proteins after lipid raft macropinocytosis. Nat. Med. 10(3):310-5. (2004).
Ming Y. W. Chua, Milton H. Lipskya, Lorrin K. Yeea,c, John Epsteina, Katharine A. Whartenbya, Scott Freemane, Tian M. Chena, Edward Chuc,d, Edwin N. Formanb, Paul Calabresia Predictive Sensitivity of Human Cancer Cells in vivo Using Semipermeable Polysulfone Fibers Pharmacology 1998; 56:318-326
Orlandini G C, Margaria R. Evaluation of the efficiency of a new hollow fiber plasmapheresis filter. Int J Artif Organs. 1983 July; 6 Suppl 1:103-6.
Wilgus T A, Vodovotz Y, Vittadini E, Clubbs E A, Oberysztn T M. Reduction of scar formation in full-thickness wounds with topical celecoxib treatment. Wound Rep Reg 2003; 11:25-34.

### Sequences

SEQ ID NO:1 (ACT2)
   PSSRASSRASSRPRPDDLEI
SEQ ID NO:2 (ACT 1)
   RPRPDDLEI
SEQ ID NO:3 (ACT 3)
   RPRPDDLEV
SEQ ID NO:4 (ACT 4)
   RPRPDDVPV
SEQ ID NO:5 (ACT 5)
   KARSDDLSV
SEQ ID NO:6
   aga cct cgg cct gat gac ctg gag att
SEQ ID NO:7 (Antp)
   RQPKIWFPNRRKPWKK
SEQ ID NO:8 (Antp/ ACT 2)
   RQPKIWFPNRRKPWKKPSSRASSRASSRPRPDDLEI
SEQ ID NO:9 (Antp/ ACT 1)
   RQPKIWFPNRRKPWKKRPRPDDLEI
SEQ ID NO:10 (Antp/ ACT 3)
   RQPKIWFPNRRKPWKKRPRPDDLEV
SEQ ID NO:11 (Antp/ACT4)
   RQPKIWFPNRRKPWKKRPRPDDVPV
SEQ ID NO:12 (Antp/ ACT 5)
   RQPKIWFPNRRKPWKKKARSDDLSV
SEQ ID NO:13 (encodes polypeptide of SEQ ID NO 9)
   egg cag ccc aag atc tgg ttc ccc aac egg aag ccc tgg aag cgg ccc ggc ccg acg acc tgg aga to
SEQ ID NO:14 (HIV-Tat)
   GRKKRRQRPPQ
SEQ ID NO:15 (Penetratin)
   RQIKIWFQNRRMKWKK
SEQ ID NO:16 (Antp-3A)
   RQIAIWFQNRRMKWAA
SEQ ID NO:17 (Tat)
   RKKRRQRRR
SEQ ID NO:18 (Buforin II)
   TRSSRAGLQFPVGRVHRLLRK
SEQ ID NO:19 (Transportan)
   GWTLNSAGYLLGKINKALAALAKKIL
SEQ ID NO:20 (model amphipathic peptide)
   KLALKLALKALKAALKLA
SEQ ID NO:21 (K-FGF)
   AAVALLPAVLLALLAP
SEQ ID NO:22 (Ku70)
   VP1VILK-PMLKE
SEQ ID NO:23 (Prion)
   MANLGYWLLALFVTMWTDVGLCKKRPKP
SEQ ID NO:24 (pVEC)
   LLIILRRRIRKQAHAHSK
SEQ ID NO:25 (Pep-1)
   KETWWETWWTEWSQPKKKRKV
SEQ ID NO:26 (SynB1)
   RGGRLSYSRRRFSTSTGR
SEQ ID NO:27 (Pep-7)
   SDLWEMMMVSLACQY
SEQ ID NO:28 (HN-1)
   TSPLNIHNGQKL
SEQ ID NO:29 (Chick alpha Cx43 ACT)
   PSRASSRASSRPRPDDLEI
SEQ ID NO:30 (Human alpha Cx45)
   GSNKSTASSKSPDPKNSVWI
SEQ ID NO:3 1 (Chick alpha Cx45)
   GSNKSSASSKSGDGKNSVWI
SEQ ID: 32 (Human alpha Cx46)
   GRASKASRASSGRARPEDLAI
SEQ ID: 33 (Human alpha Cx46.6)
   GSASSRDGKTVW1
SEQ ID NO:34 (Chimp alpha Cx36)
   PRVSVPNFGRTQSSDSAYV
SEQ ID NO:35 (Chick alpha Cx36)
   PRMSMPNFGRTQSSDSAYV
SEQ ID NO:36 (Human alpha Cx47)
   PRAGSEKGSASSRDGKTTVWI
SEQ ID NO:37 (Human alpha Cx40)
   GYHSDKRRLSKASSKARSDDLSV
SEQ ID NO:38 (Human alpha Cx50)
   PLSRLSKASSRARSDDLTV
SEQ ID NO:39 (Human alpha Cx59)
   PNHVVSLTNNLIGRRVPTDLQI
SEQ ID NO:40 (Rat alpha Cx33)
   PSCVSSSAVLTTICSSDQVVPVGLSSFYM
SEQ ID NO:41 (Sheep alpha Cx44)
   GRSSKASKSSGGRARAADLA1
SEQ ID NO:42 (Human beta Cx26)
   LCYLLIRYCSGKSKKPV
SEQ ID: 43 (Human alpha Cx37)
   G QK PP SRP SSSAS K KQ*YV
SEQ ID 44: (conservative Cx43 variant)
   SSRASSRASSRPRPDDLEV
SEQ ID 45: (conservative Cx43 variant)
   RPKPDDLEI,
SEQ ID 46: (conservative Cx43 variant)
   SSRASSRASSRPKPDDLEI,
SEQ ID 47: (conservative Cx43 variant)
   RPKPDDLDI
SEQ ID 48: (conservative Cx43 variant)
   SSRASSRASSRPRPDDLDI
SEQ ID 49: (conservative Cx43 variant)
   SSRASTRΛSSRPRPDDLEI
SEQ ID 50: (conservative Cx43 variant)
   RPRPEDLE1
SEQ ID 51: (conservative Cx43 variant)
   SSRASSRASSRPRPEDLEI,
SEQ ID 52: (conservative Cx45 variant)
   GDGKNSVWV
SEQ ID 53: (conservative Cx45 variant)
   SKAGSNKSTASSKSGDGKNSVWV
SEQ ID 54: (conservative Cx37 variant)
   GQKPPSRPSSSASKKLYV
SEQ ID NO: 55 (non-active control peptide)
   RQPKIWFPNRRKPWKIELDDPRPR
SEQ ID NO:56 (HIV-Tat/ ACT 1)
   GRKKRRQRPPQ RPRPDDLEI
SEQ ID NO:57 (Penetratin/ ACT 1)
   RQIKIWFQNRRMKWKK RPRPDDLEI
SEQ ID NO:58 (Antp-3A/ ACT 1)
   RQIAIWFQNRRMKWAA RPRPDDLEI
SEQ ID NO:59 (Tat/ ACT 1)
   RKKRRQRRR RPRPDDLEI
SEQ ID NO:60 (Buforin II/ ACT 1)
   TRSSRAGLQFPVGRVHRLLRK RPRPDDLEI
SEQ ID NO:61 (Transportan/ ACT 1)
   GWTLNSAGYLLGKINKALAALAKKIL RPRPDDLEI
SEQ ID NO:62 (MAP/ ACT 1)
   KLALKLALKALKAALKLA RPRPDDLEI
SEQ ID NO:63 (K-FGF/ ACT 1)
   AAVALLPAVLLALLAP RPRPDDLEI
SEQ ID NO:64 (Ku70/ ACT 1)
   VPMLKPMLKE RPRPDDLEI
SEQ ID NO:65 (Prion/ ACT 1)
   MANLGYWLLALFVTMWTDVGLCKKRPKP RPRPDDLEI
SEQ ID NO:66 (pVEC/ ACT 1)
   LLIILRRRIRKQAHAHSK RPRPDDLEI
SEQ ID NO:67 (Pep-1/ ACT 1)
   KETWWETWWTEWSQPKKKRKV RPRPDDLEI
SEQ ID NO:68 (SynB 1 / ACT 1)
   RGGRLSYSRRRFSTSTGR RPRPDDLEI
SEQ ID NO:69 (Pep-7/ ACT 1)
   SDLWEMMMVSLACQY RPRPDDLEI
SEQ ID NO:70 (HN-1/ ACT 1)
   TSPLNIHNGQKL RPRPDDLEI
SEQ ID NO:72 (20 to 120 residues flanking amino acid 363 of human Cx43 
KGKSDPYHATSGALSPAKDCGSQKYAYFNGCSSPTAPLSPMSPPGYKLVT
GDRNNSSCRNYNKQASEQNWANYSAEQNRMGQAGSTISNSHAQPFDFPDD
NQNSKKLAAGHELQPLAIVD
SEQ ID NO:73 (20 to 120 residues flanking amino acid 362 of chick Cx43) 
KTDPYSHSGTMSPSKDCGSPKYAYYNGCSSPTAPLSPMSPPGYKLVTGDR
NNSSCRNYNKQASEQNWANYSAEQNRMGQAGSTISNSHAQPFDFADEHQN
TKKLASGHELQPLTIVDQRP
SEQ ID NO:74 (20 to 120 residues flanking amino acid 377 of human Cx45) 
LGFGTIRDSLNSKRRELEDPGAYNYPFTWNTPSAPPGYNIAVKPDQIQYT
ELSNAKIAYKQNKANTAQEQQYGSHEENLPADLEALQREIRMAQERLDLA
VQAYSHQNNPHGPREKKAKV
SEQ ID NO:75 (20 to 120 residues flanking amino acid 375 of chick Cx45) 
GFGTIRDTLNNKRKELEDSGTYNYPFTWNTPSAPPGYNIAVKPDQMQYTE
LSNAKMAYKQNKANIAQEQQYGSNEENIPADLENLQREIKVAQERLDMAI
QAYNNQNNPGSSSREKKSKA.
SEQ ID NO:76 (20 to 120 residues flanking amino acid 313 of human Cx37 
PYLVDCFVSRPTEKTIFIIFMLVVGLISLVLNLLELVHLLCRCLSRGMRA
RQGQDAPPTQGTSSDPYTDQVFFYLPVGQGPSSPPCPTYNGLSSSEQNWA
NLTTEERLASSRPPLFLDPP
SEQ ID NO:77 (20 to 120 residues flanking amino acid 258 of rat Cx33) 
CGSKEHGNRKMRGRLLLTYMASIFFKSVFEVAFLLIQWYLYGFTLSAVYI
CEQSPCPHRVDCFLSRPTEKTIFILFMLVVSMVSFVLNVIELFYVLFKAI
KNHLGNEKEEVYCNPVELQK.
SEQ ID NO:78 (enhanced green fluorescent protein) 
MVSKGEELFTGVVPILVELDGDVNGHKFSVSGEGEGDATYGKLTLKFICT
TGKLPVPWPTLVTTLTYGVQCFSRYPDHMKQHDFFKSAMPEGYVQERTIF
FKDDGNYKTRAEVKFEGDTLVNRIELKGIDFKEDGNILGHKLEYNYNSHN
VYIMADKQKNGIKVNFKIRHNIEDGSVQLADHYQQNTPIGDGPVLLPDNH
YLSTQSALSKDPNEKRDHMVLLEFVTAAGITLGMDELYK
SEQ ID NO:78 ACT 2) 
CCCTCCTCCCGGGCCTCCTCCCGGGCCTCCTCCCGGCCCCGGCCCGAC G
ACCTGGAGATC
SEQ ID NO:79 (ACT 1)
   CGGCCCCGGCCCGACGACCTGGAGATC
SEQ ID NO:80 (ACT 3)
   CGGCCCCGGCCCGACGACCTGGAGGTG
SEQ ID NO:81 (ACT 4)
   CGGCCCCGGCCCGACGACGTGCCCGTG
SEQ ID NO:82 (ACT 5)
   AAGGCCCGGTCCGACGACCTGTCCGTG
SEQ ID NO:83 (Antp)
   CGGCAGCCCAAGATCTGGTTCCCCAACCGGCGGAAGCCCTGGAAG AAG
SEQ ID NO:84 (Antp/ ACT 2) 
CGGCAGCCCAAGATCTGGTTCCCCAACCGGCGGAAGCCCTGGAAGAAGCC
CTCCTCCCGGGCCTCCTCCCGGGCCTCCTCCCGGCCCCGGCCCGACGACC
TGGAGATC
SEQ ID NO:85 (Antp/ ACT 1) 
CGGCAGCCCAAGATCTGGTTCCCCAACCGGCGGAAGCCCTGGAAGAAGCG
GCCCCGGCCCGACGACCTGGAGATC
SEQ ID NO:86 (Antp/ ACT 3) 
CGGCAGCCCAAGATCTGGTTCCCCAACCGGCGGAAGCCCTGGAAGAAGCG
GCCCCGGCCCGACGACCTGGAGGTG
SEQ ID NO:87 (Antp/ ACT 4) 
CGGCAGCCCAAGATCTGGTTCCCCAACCGGCGGAAGCCCTGGAAGAAGCG
GCCCCGGCCCGACGACGTGCCCGTG
SEQ ID NO:88 (Antp/ ACT 5) 
CGGCAGCCCAAGATCTGGTTCCCCAACCGGCGGAAGCCCTGGAAGAAGAA
GGCCCGGTCCGACGACCTGTCCGTG
SEQ ID NO:89 (Zebrafish alpha Cx43)
   PCSRASSRMSSRARPDDLDV
SEQ ID NO:90 (Chick alpha Cx36)
   PRVSVPNFGRTQSSDSAYV
SEQ ID NO:91 (Peptide 328967 (ACT 1))

Biotin-Ahx-Arg-Gln-Pro-Lys-Ile-Trp-Phe-Pro-Asn-Arg-Arg-Lys-Pro-Trp-Lys-Lys-Arg-Pro-
Arg-Pro-Asp-Asp-Leu-Glu-Ile-OH

## Claims

1. A composition comprising an isolated polypeptide comprising SEQ ID NO: 2, for the use in a method of preventing radiation injury or mitigating the progression of radiation injury in a subject.

2. The composition for the use according to claim 1, wherein the composition is administered to the subject
(i) prior to exposure of the subject to radiation, and/or
(ii) following exposure to radiation, wherein the method inhibits the progression of the radiation injury.

3. The composition for the use according to claim 1, wherein the composition is administered topically to the subject.

4. The composition for the use according to claim 1, wherein the composition further comprises hydroxyethylcellulose gel, optionally wherein the hydroxyethylcellulose gel is present at a concentration of about 1.25% (w/w).

5. The composition for the use according to claim 1, wherein the composition is administered to the subject by aerosolized delivery or wherein the composition is administered to the subject parenterally, optionally wherein the composition is administered to the subject by an intravenous, subcutaneous, intraperitoneal, intramuscular route.

6. The composition for the use according to claim 1, wherein the concentration of the polypeptide in the composition is from 10 µM to 2000 µM; or wherein the composition is administered to the subject at a dose of from 1 mg/kg to 50 mg/kg.

7. The composition for the use according to claim 1, wherein the radiation injury is selected from the group consisting of cutaneous radiation injury (CRI), acute radiation syndrome (ARS), combined radiation injury, radiation burns, radiation dermatitis, radiation injury to the nervous system or brain, radiation pneumonitis, radiation-induced enteritis, or a combination thereof, preferably wherein the radiation injury comprises CRI and ARS.

8. The composition for the use according to claim 7, wherein the radiation dermatitis is a result of a medical intervention, optionally wherein the medical intervention is a cancer therapy, optionally wherein the cancer therapy is radiotherapy.

9. The composition for the use according to claim 1, wherein the radiation injury results from exposure to a source of radiation selected from the group consisting of weapons of mass destruction (WMD), nuclear explosions, dirty bombs, a diagnostic machine, the sun, and a tanning bed.

10. The composition for the use according to claim 1, wherein the radiation injury occurs in a tissue selected from the group consisting of skin, heart, bone, brain, spinal cord, cornea, retina, and peripheral nerve.

11. The composition for the use according to claim 1, wherein the polypeptide prevents uptake of radionuclides.

12. The composition for the use according to claim 1, wherein the polypeptide comprises the amino acid sequence of SEQ ID NO:9; or the composition comprises SEQ ID NO: 91.

13. The composition for the use according to claim 1, wherein the polypeptide further comprises a cellular internalization sequence, optionally wherein the cellular internalization sequence comprises an amino acid sequence of a protein selected from a group consisting of Antennapedia, TAT, HIV-Tat, Penetratin, Antp-3A (Antp mutant), Buforin II, Transportan, MAP (model amphipathic peptide), K-FGF, Ku70, Prion, pVEC, Pep-1, SynB 1, Pep-7, HN-1, BGSC (Bis-Guanidinium-Spermidine-Cholesterol) and BGTC (Bis-Guanidinium-Tren-Cholesterol).

14. The composition for the use according to any one of claims 1 - 13 wherein said use is specific for the use in a method of preventing radiation injury in a subject.

## Patentansprüche

1. Zusammensetzung umfassend ein isoliertes Polypeptid, das SEQ ID NO: 2 umfasst, zur Verwendung in einem Verfahren zur Verhinderung von Strahlenschäden oder zur Milderung des Fortschreitens von Strahlenschäden in einem Subjekt.

2. Zusammensetzung zur Verwendung gemäß Anspruch 1, wobei die Zusammensetzung dem Subjekt
(i) vor der Einwirkung von Strahlung auf das Subjekt, und/oder
(ii) nach der Einwirkung von Strahlung auf das Subjekt, verabreicht wird, wobei das Verfahren das Fortschreiten der Strahlenschäden hemmt.

3. Zusammensetzung zur Verwendung gemäß Anspruch 1, wobei die Zusammensetzung topisch an das Subjekt verabreicht wird.

4. Zusammensetzung zur Verwendung gemäß Anspruch 1, wobei die Zusammensetzung ferner Hydroxyethylcellulosegel umfasst, wobei optional das Hydroxyethylcellulosegel in einer Konzentration von etwa 1,25 % (w/w) vorliegt.

5. Zusammensetzung zur Verwendung gemäß Anspruch 1, wobei die Zusammensetzung dem Subjekt durch aerosolisierte Verabreichung verabreicht wird oder wobei die Zusammensetzung dem Subjekt parenteral verabreicht wird, wobei optional die Zusammensetzung dem Subjekt auf intravenösem, subkutanem, intraperitonealem oder intramuskulärem Weg verabreicht wird.

6. Zusammensetzung zur Verwendung gemäß Anspruch 1, wobei die Konzentration des Polypeptids in der Zusammensetzung 10 µM bis 2000 µM ist; oder wobei die Zusammensetzung dem Subjekt in einer Dosis von 1 mg/kg bis 50 mg/kg verabreicht wird.

7. Zusammensetzung zur Verwendung gemäß Anspruch 1, wobei der Strahlenschaden ausgewählt ist aus der Gruppe bestehend aus kutanem Strahlenschaden (CRI), akutem Strahlensyndrom (ARS), kombiniertem Strahlenschaden, Strahlenverbrennungen, Strahlendermatitis, Strahlenschaden des Nervensystems oder des Gehirns, Strahlenpneumonitis, strahleninduzierter Enteritis oder eine Kombination davon, wobei vorzugsweise der Strahlenschaden CRI und ARS umfasst.

8. Zusammensetzung zur Verwendung gemäß Anspruch 7, wobei die Strahlendermatitis ein Resultat eines medizinischen Eingriffs ist, optional wobei der medizinische Eingriff eine Krebstherapie ist, optional wobei die Krebstherapie eine Strahlentherapie ist.

9. Zusammensetzung zur Verwendung gemäß Anspruch 1, wobei der Strahlenschaden aus Exposition gegenüber einer Strahlungsquelle resultiert, die aus der Gruppe bestehend aus Massenvernichtungswaffen (WMD), Atomexplosionen, schmutzigen Bomben, einem Diagnosegerät, der Sonne und einer Sonnenbank ausgewählt ist.

10. Zusammensetzung zur Verwendung gemäß Anspruch 1, wobei der Strahlenschaden in einem Gewebe auftritt, das ausgewählt ist aus der Gruppe bestehend aus Haut, Herz, Knochen, Gehirn, Rückenmark, Hornhaut, Netzhaut und peripherem Nerv.

11. Zusammensetzung zur Verwendung gemäß Anspruch 1, wobei das Polypeptid die Aufnahme von Radionukliden verhindert.

12. Zusammensetzung zur Verwendung gemäß Anspruch 1, wobei das Polypeptid die Aminosäuresequenz von SEQ ID NO: 9 umfasst; oder die Zusammensetzung SEQ ID NO: 91 umfasst.

13. Zusammensetzung zur Verwendung gemäß Anspruch 1, wobei das Polypeptid ferner eine zelluläre Internalisierungssequenz umfasst, optional wobei die zelluläre Internalisierungssequenz eine Aminosäuresequenz eines Proteins umfasst, das ausgewählt ist aus einer Gruppe bestehend aus Antennapedia, TAT, HIV-Tat, Penetratin, Antp-3A (Antp-Mutante), Buforin II, Transportan, MAP (amphipathisches Modellpeptid), K-FGF, Ku70, Prion, pVEC, Pep-1, SynB 1, Pep-7, HN-1, BGSC (Bis-Guanidinium-Spermidin-Cholesterin) und BGTC (Bis-Guanidinium-Tren- Cholesterin).

14. Zusammensetzung zur Verwendung gemäß irgendeinem der Ansprüche 1 bis 13, wobei die Verwendung spezifisch für die Verwendung in einem Verfahren zur Verhinderung von Strahlenschäden bei einem Subjekt ist.

## Revendications

1. Composition comprenant un polypeptide isolé comprenant le SEQ ID N° : 2, destinée à être utilisée dans un procédé de prévention d'une radiolésion ou d'atténuation de la progression d'une radiolésion chez un sujet.

2. Composition destinée à être utilisée selon la revendication 1, dans laquelle la composition est administrée au sujet
(i) avant l'exposition du sujet à un rayonnement, et/ou
(ii) après l'exposition à un rayonnement, dans laquelle le procédé inhibe la progression de la radiolésion.

3. Composition destinée à être utilisée selon la revendication 1, dans laquelle la composition est administrée localement au sujet.

4. Composition destinée à être utilisée selon la revendication 1, dans laquelle la composition comprend en outre un gel d'hydroxyéthylcellulose, facultativement dans laquelle le gel d'hydroxyéthylcellulose est présent à une concentration d'environ 1,25 % (p/p).

5. Composition destinée à être utilisée selon la revendication 1, dans laquelle la composition est administrée au sujet par voie aérosol ou dans laquelle la composition est administrée au sujet par voie parentérale, facultativement dans laquelle la composition est administrée au sujet par une voie intraveineuse, sous-cutanée, intrapéritonéale, intramusculaire.

6. Composition destinée à être utilisée selon la revendication 1, dans laquelle la concentration du polypeptide dans la composition est comprise entre 10 µM et 2 000 µM ; ou dans laquelle la composition est administrée au sujet à une dose de 1 mg/kg à 50 mg/kg.

7. Composition destinée à être utilisée selon la revendication 1, dans laquelle la radiolésion est sélectionnée dans le groupe constitué d'une lésion par irradiation cutanée (CRI), d'un syndrome d'irradiation aigu (ARS), d'une lésion par irradiation combinée, de brûlures par irradiation, d'une dermatite radique, d'une lésion par irradiation du système nerveux ou du cerveau, d'une pneumopathie résultant d'une irradiation, d'une entérite radio-induite, ou d'une combinaison de ceux-ci, de préférence dans laquelle la radiolésion comprend la CRI et l'ARS.

8. Composition destinée à être utilisée selon la revendication 7, dans laquelle la dermatite radique est le résultat d'une intervention médicale, facultativement dans laquelle l'intervention médicale est une thérapie anticancéreuse, facultativement dans laquelle la thérapie anticancéreuse est une radiothérapie.

9. Composition destinée à être utilisée selon la revendication 1, dans laquelle la radiolésion résulte d'une exposition à une source de rayonnement sélectionnée dans le groupe constitué des armes de destruction massive (ADM), des explosions nucléaires, des bombes sales, d'un appareil de diagnostic, du soleil et d'un lit de bronzage.

10. Composition destinée à être utilisée selon la revendication 1, dans laquelle la radiolésion survient dans un tissu sélectionné dans le groupe constitué de la peau, du coeur, des os, du cerveau, de la moelle épinière, de la cornée, de la rétine et du nerf périphérique.

11. Composition destinée à être utilisée selon la revendication 1, dans laquelle le polypeptide empêche l'absorption des radionucléides.

12. Composition destinée à être utilisée selon la revendication 1, dans laquelle le polypeptide comprend la séquence d'acides aminés du SEQ ID N°: 9 ; ou la composition comprend le SEQ ID N°: 91.

13. Composition destinée à être utilisée selon la revendication 1, dans laquelle le polypeptide comprend en outre une séquence d'internalisation cellulaire, facultativement dans laquelle la séquence d'internalisation cellulaire comprend une séquence d'acides aminés d'une protéine sélectionnée dans un groupe constitué d'Antennapedia, TAT, HIV-Tat, pénétratine, Antp-3A (Antp mutante), buforine II, transportane, MAP (peptide amphipathique modèle), K-FGF, Ku70, Prion, pVEC, Pep-1, SynB 1, Pep-7, HN-1, BGSC (Bis-Guanidinium-Spermidine-Cholestérol) et BGTC (Bis-Guanidinium-Tren-cholestérol).

14. Composition destinée à être utilisée selon l'une quelconque des revendications 1 à 13, dans laquelle ladite utilisation est spécifique à l'utilisation dans un procédé de prévention d'une radiolésion chez un sujet.
